# EUROPEAN PATENT SPECIFICATION

(11) **EP 1 074 549 B1**
(45) Date of publication and mention of the grant of the patent: **19.11.2003**
(21) Application number: 00116091.0
(22) Date of filing: 27.07.2000
(51) Int. Cl.: C07D 403/04, A61K 31/55, A61P 43/00, C07D 403/14, C07D 401/04, C07D 223/04, C07D 405/14, C07D 487/04, C07D 401/14, C07F 7/18, C07D 521/00, G01N 33/50, C07D 471/04

(54) **Tetrahydro-benzo(d)azepines and their use as antagonists at metabotropic glutamate receptors**
Tetrahydro-benzo(d)azepine und deren Verwendung als metabotrope Glutamatrezeptor-Antagonisten
Tétrahydro-benzo(d)azépines et leurs utilisations en tant qu'antagonistes du récepteur de glutamate métabotropique

(30) Priority: 06.08.1999 EP 99115557
(43) Date of publication of application: 07.02.2001
(73) Proprietor: F. HOFFMANN-LA ROCHE AG, 4070 Basel (CH)
(72) Inventor: Adam, Geo, 79650 Schopfheim (DE); Binggeli, Alfred, 4102 Binningen (CH); Maerki, Hans-Peter, 4059 Basle (CH); Mutel, Vincent, 68100 Mulhouse (FR); Wilhelm, Maurice, 68790 Morschwiller le Bas (FR); Wostl, Wolfgang, 79639 Grenzach-Wyhlen (DE)
(74) Representative: Poppe, Regina

(56) References cited:
- EP-A- 0 891 978
- WO-A-02/06288

## Description

European patent application 0891978 A2 discloses 5h-thiazolo (3,2-a) pyrimidine derivatives which are metabotrophic glutamate receptor agonists and/or antagonists.

International patent application 02/06288 A1 (publication date 24.02.2002) discloses tetrahydro-hetrocyloazepinyl pyrimidine derivatives, which are metabotrophic glutamate receptor antagonists.

The present invention is concerned with 1,2,4,5-tetrahydro-benzo[d]azepin derivatives of the general formula 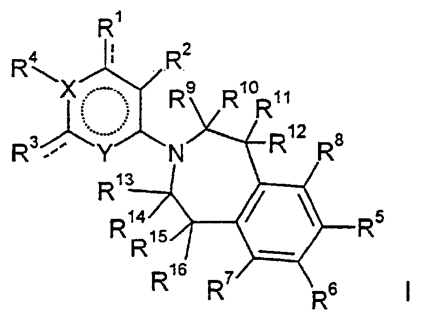 wherein
- R¹: signifies hydrogen, lower alkyl, oxygen, halogen, or
-OR, -O(C₃-C₆)cycloalkyl, -O(CHR)ₙ-(C₃-C₆)cycloalkyl, -O(CHR)ₙCN, -O(CHR)ₙCF₃, -O(CHR)(CHR)ₙNR₂, -O(CHR)(CHR)ₙOR, -O(CHR)ₙ-lower alkenyl, -OCF₃, -OCF₂-R, -OCF₂-lower alkenyl, -OCHRF, -OCHF-lower alkenyl, -OCF₂CRF₂, -OCF₂Br, -O(CHR)ₙCF₂Br, -O(CHR)ₙ-phenyl, wherein the phenyl group maybe optionally substituted independently from each other by one to three lower alkyl, lower alkoxy, halogen, nitro or cyano groups,
-O(CHR)(CHR)ₙ-morpholino, -O(CHR)(CHR)ₙ-pyrrolidino, -O(CHR)(CHR)ₙ-piperidino, -O(CHR)(CHR)ₙ-imidazolo, -O(CHR)(CHR)ₙ-triazolo, -O(CHR)ₙ-pyridino, -O(CHR)(CHR)ₙ-OSi-lower alkyl, -O(CHR)(CHR)ₙOS(O)₂-lower alkyl, -O(CH₂)ₙCH=CF₂, -O(CHR)ₙ-2,2-dimethyl-[1.3]dioxolane, -O(CHR)ₙ-CHOR-CH₂OR, -O(CHR)ₙ-CHOR-(CHR)ₙ-CH₂OR or
-SR or -S(CHR)ₙCOOR, or -NR², -N(R)(CHR)(CHR)ₙOR, -N(R)(CHR)ₙCF₃, -N(R)(CHR)(CHR)ₙ-morpholino, -N(R)(CHR)(CHR)ₙ-imidazolo, -N(R)(CHR)(CHR)ₙ-pyrrolidino, -N(R)(CHR)(CHR)ₙ-pyrrolidin-2-one, -N(R)(CHR)(CHR)ₙ-piperidino, -N(R)(CHR)(CHR)ₙ-triazolo, -N(R)(CHR)ₙ-pyridino, or
- n: is 1 - 6;
- R: signifies hydrogen, lower alkyl or lower alkenyl, independently from each other, if more than one R is present;
- R²: signifies nitro or cyano;
- R³: signifies hydrogen, lower alkyl, =O, =S, -SR, -S(O)₂-lower alkyl, -(C₃-C₆)cycloalky or piperazino, optionally substituted by lower alkyl, or
-CONR₂, -(CHR)ₙCONR₂, -(CHR)ₙOR, -(CH₂)ₙ-CF₃, -CF₃, -(CHR)ₙOC(O)CF₃, -(CHR)ₙCOOR, -(CHR)ₙSC₆H₅, wherein the phenyl group may be optionally substituted independently from each other by one to three lower alkyl, lower alkoxy, halogen, nitro or cyano groups, -(CHR)ₙ-1,3-dioxo-1,3-dihydro-isoindol, -(CHR)ₙ-tetrahydro-pyran-2-yloxy or -(CHR)ₙ-S-lower alkyl, or
-NR₂, -NRCO-lower alkyl, -NRCHO, -N(R)(CHR)ₙCN, -N(R)(CHR)ₙCF₃, -N(R)(CHR)(CHR)ₙ-OR, -N(R)C(O)(CHR)ₙO-lower alkyl, -NR(CHR)ₙ-lower alkyl, -NR(CHR)(CHR)ₙ-OR, -N(R)(CHR)(CHR)ₙ-O-phenyl, wherein the phenyl group may be optionally substituted independently from each other by one to three lower alkyl, lower alkoxy, halogen, nitro or cyano groups, -N(R)(CHR)ₙ-lower alkenyl, -N(R)(CHR)(CHR)ₙ-O-(CHR)ₙOR, -N(R)(CHR)ₙC(O)O-lower alkyl, -N(R)(CHR)ₙC(O)NR-lower alkyl, -N(R)(CH₂)ₙ-2,2-dimethyl-[1.3]dioxolane, -N(R)(CHR)(CHR)ₙmorpholino, -N(R)(CHR)ₙ-pyridino, -N(R)(CHR)(CHR)ₙ-piperidino, -N(R)(CHR)(CHR)ₙ-pyrrolidino, -N(R)(CHR)(CHR)ₙ-O-pyridino, -N(R)(CHR)(CHR)ₙimidazolo, -N(R)(CHR)ₙCR₂-(CHR)ₙ-OR, -N(R)(CHR)ₙ-CR₂-OR, -N(R)(CHR)ₙ-CHOR-CH₂OR, -N(R)(CHR)ₙ-CHOR-(CHR)ₙ-CH₂OR, or -OR, -O(CHR)ₙCF₃, -OCF₃, -O(CHR)(CHR)ₙ-O-phenyl, wherein the phenyl group may be optionally substituted independently from each other by one to three lower alkyl, lower alkoxy, halogen, nitro or cyano groups, -O(CHR)(CHR)ₙ-O-lower alkyl, -O(CHR)ₙ-pyridino or -O(CHR)(CHR)ₙ-morpholino; and
- R⁴: signifies hydrogen, lower alkyl, lower alkenyl or nitro, or
-OR, -OCF₃, -OCF₂-R, -OCF₂-lower alkenyl, -OCHRF, -OCHF-lower alkenyl, -O(CHR)ₙCF₃, or
-(CHR)ₙCHRF, -(CHR)ₙCF₂R, -(CHR)ₙCF₃, -(C₃-C₆)cycloalkyl, -(CHR)ₙ(C₃-C₆)cycloalkyl, -(CHR)ₙCN, -(CHR)ₙ-phenyl, wherein the phenyl group may be optionally substituted independently from each other by one to three lower alkyl, lower alkoxy, halogen, nitro or cyano groups, -(CHR)(CHR)ₙOR, -(CHR)ₙCHORCH₂OR, -(CHR)(CHR)ₙNR₂, -(CHR)ₙCOOR, -(CHR)(CHR)ₙOSi-lower alkyl, -(CHR)(CHR)ₙ-OS(O)₂-lower alkyl, -(CH₂)ₙ-CH=CF₂, -CF₃, -CF₂-R, -CF₂-lower alkenyl, -CHRF, -CHF-lower alkenyl, -(CHR)ₙ-2,2-dimethyl-[1.3]dioxolane, -(CH₂)ₙ-2-oxo-azepan-1-yl, -(CHR)(CHR)ₙ-morpholino, -(CHR)ₙ-pyridino, -(CHR)(CHR)ₙ-imidazolo, -(CHR)(CHR)ₙ-triazolo, -(CHR)(CHR)ₙ-pyrrolidino, optionally substituted by -(CH₂)ₙOH, -(CHR)(CHR)ₙ-3-hydroxy-pyrrolidino or -(CHR)(CHR)ₙ-piperidino, or
-NR₂, -N(R)(CHR)ₙ-pyridino, -N(R)C(O)O-lower alkyl, -N(CH₂CF₃)C(O)O-lower alkyl, -N[C(O)O-lower alkyl]₂, -NR-NR-C(O)O-lower alkyl or -N(R)(CHR)ₙCF₃, -NRCF₃, -NRCF₂-R, -NRCF₂-lower alkenyl, -NRCHRF, -NRCHF-lower alkenyl;
or is absent, if X is -N= or =N-;
or R⁴ and R¹ or R³ and R⁴ are interconnected to the groups -(CH₂)₃₋₅-, -(CH₂)₂-N=, -CH=N-N=-, -CH=CH-N=, -NH-CH=CH- or -NR-CH₂-CH₂- and form together with the N and C atoms to which they are attached an additional ring;
- R⁵, R⁶: signify hydrogen, lower alkyl, lower alkoxy, amino, nitro, -SO₂NH₂ or halogen; or
- R⁵ and R⁶: are interconnected to the group -O-CH₂-O- and form together with the C atoms to which they are attached an additional 5-membered ring;
- R⁷, R⁸: signify hydrogen, lower alkyl, lower alkoxy, amino, nitro or halogen;
- R⁹, R¹⁰: signify hydrogen or lower alkyl;
- R¹¹, R¹²: signifies hydrogen, lower alkyl, hydroxy, lower alkoxy, lower alkoxycarbonyloxy or lower alkanoyloxy;
- R¹³, R¹⁴: signify hydrogen, tritium or lower alkyl;
- R¹⁵, R¹⁶: signifies hydrogen, tritium, lower alkyl, hydroxy, lower alkoxy, lower alkoxycarbonyloxy or lower alkanoyloxy or are together an oxo group; or
- X: signifies -N=, =N-, -N<, >C= or =C<;
- Y: signifies -N=, =N-, -NH-, -CH= or =CH-; and
the dotted line may be a bond,
as well as with their pharmaceutically acceptable salts in their racemic and optically active form.

It has surprisingly been found that the compounds of general formula I are antagonists at metabotropic glutamate receptors.

In the central nervous system (CNS) the transmission of stimuli takes place by the interaction of a neurotransmitter sent out by a neuron, with another neuroreceptor.

L-glutamic acid, the most commonly occurring neurotransmitter in the CNS, plays a critical role in a large number of physiological processes. The glutamate-dependent stimulus receptors are divided into two main groups. The first main group forms ligand-controlled ion channels. The metabotropic glutamate receptors (mGluR) belong to the second main group and, furthermore, belong to the family of G-protein-coupled receptors.

At present, eight different members of these mGluRs are known and some of these even have sub-types. On the basis of structural parameters, the different second messenger signaling pathways and their different affinity to low-molecular weight chemical compounds, these eight receptors can be sub-divided into three sub-groups:
mGluR1 and mGluR5 belong to group I, mGluR2 and mGluR3 belong to group II and mGluR4, mGluR6, mGluR7 and mGluR8 belong to group III.

Ligands of metabotropic glutamate receptors belonging to the first group can be used for the treatment or prevention of acute and/or chronic neurological disorders such as epilepsy, stroke, chronic and acute pain, psychosis, schizophrenia, Alzheimer's disease, cognitive disorders and memory deficits.

Other treatable indications in this connection are restricted brain function caused by bypass operations or transplants, poor blood supply to the brain, spinal cord injuries, head injuries, hypoxia caused by pregnancy, cardiac arrest and hypoglycaemia. Further treatable indications are Huntington's chorea, amyotrophic lateral sclerosis (ALS), dementia caused by AIDS, eye injuries, retinopathy, idiopathic parkinsonism or parkinsonism caused by medicaments as well as conditions which lead to glutamate-deficiency functions, such as e.g. muscle spasms, convulsions, migraine, urinary incontinence, nicotine addiction, opiate addiction, anxiety, vomiting, dyskinesia and depression.

Objects of the present invention are compounds of formula I and their pharmaceutically acceptable salts per se and as pharmaceutically active substances, their manufacture, medicaments based on a compound in accordance with the invention and their production as well as the use of the compounds in accordance with the invention in the control or prevention of illnesses of the aforementioned kind, and, respectively, for the production of corresponding medicaments. Furthermore, the use of radiolabeled mGluR1 receptor antagonists of formula I in a binding assay is also an object of the present invention.

Preferred compounds of formula I in the scope of the present invention are those, in which
R¹ is =O or hydroxy and R² is NO₂.

The following are examples of such compounds:
3-Ethyl-2-methyl-5-nitro-6-(1,2,4,5-tetrahydro-benzo[d]azepin-3-yl)-3H-pyrimidin-4-one,
3-(2-fluoro-ethyl)-2-methyl-5-nitro-6-(1,2,4,5-tetrahydro-benzo[d]azepin-3-yl)-3H-pyrimidin-4-one,
2-methyl-5-nitro-6-(1,2,4,5-tetrahydro-benzo[d] azepin-3-yl)-3-(2,2,2-trifluoro-ethyl)-3H-pyrimidin-4-one or
2-methyl-5-nitro-6-(1,2,4,5-tetrahydro-benzo[d]azepin-3-yl)-pyrimidin-4-ol.

Compounds of formula I, in which
R¹ is =O and R² is -CN are also preferred.
The following are examples of such compounds:
2-Amino-6-oxo-4-(1,2,4,5-tetrahydro-benzo[d]azepin-3-yl)-1,6-dihydro-pyrimidine-5-carbonitrile,
6-oxo-4-(1,2,4,5-tetrahydro-benzo[d]azepin-3-yl)-1,6-dihydro-pyrimidine-5-carbonitrile,
2-ethylamino-6-oxo-4-(1,2,4,5-tetrahydro-benzo[d]azepin-3-yl)-1,6-dihydro-pyrimidine-5-carbonitrile,
1,2-dimethyl-6-oxo-4-(1,2,4,5-tetrahydro-benzo[d]azepin-3-yl)-1,6-dihydro-pyrimidine-5-carbonitrile,
1-ethyl-2-methyl-6-oxo-4-(1,2,4,5-tetrahydro-benzo[d]azepin-3-yl)-1,6-dihydro-pyrimidine-5-carbonitrile,
2-amino-1-ethyl-6-oxo-4-(1,2,4,5-tetrahydro-benzo[d]azepin-3-yl)-1,6-dihydro-pyrimidine-5-carbonitrile,
1-cyclopropylmethyl-2-methyl-6-oxo-4-(1,2,4,5-tetrahydro-benzo[d]azepin-3-yl)-1,6-dihydro-pyrimidine-5-carbonitrile,
1-allyl-2-methyl-6-oxo-4-(1,2,4,5-tetrahydro-benzo[d]azepin-3-yl)-1,6-dihydro-pyrimidine-5-carbonitrile,
1-cyanomethyl-2-methyl-6-oxo-4-(1,2,4,5-tetrahydro-benzo[d]azepin-3-yl)-1,6-dihydro-pyrimidine-5-carbonitrile,
1-(2-dimethylamino-ethyl)-2-methyl-6-oxo-4-(1,2,4,5-tetrahydro-benzo[d]azepin-3-yl)-1,6-dihydro-pyrimidine-5-carbonitrile,
1-isopropyl-6-oxo-4-(1,2,4,5-tetrahydro-benzo[d]azepin-3-yl)-1,6-dihydro-pyrimidine-5-carbonitrile,
1-(2-hydroxy-ethyl)-6-oxo-4-(1,2,4,5-tetrahydro-benzo[d]azepin-3-yl)-1,6 dihydro-pyrimidine-5-carbonitrile,
2-(2-hydroxy-ethyl)-6-oxo-4-(1,2,4,5-tetrahydro-benzo[d]azepin-3-yl)-1,6 dihydro-pyrimidine-5-carbonitrile,
2-methyl-6-oxo-4-(1,2,4,5-tetrahydro-benzo[d]azepin-3-yl)-1-(2,2,2-trifluoro-ethoxy)-1,6-dihydro-pyrimidine-5-carbonitrile,
2-methyl-1-methylamino-6-oxo-4-(1,2,4,5-tetrahydro-benzo[d]azepin-3-yl)-1,6-dihydro-pyrimidine-5-carbonitrile or
1-amino-2-methyl-6-oxo-4-(1,2,4,5-tetrahydro-benzo[d]azepin-3-yl)-1,6-dihydro-pyrimidine-5-carbonitrile.

Preferred compounds of formula I in the scope of the present invention are those, in which
R¹ is 2,2,2-trifluoroethoxy and R² is -CN.

The following are examples of such compounds:
2-(2-Morpholin-4-yl-ethylamino)-4-(1,2,4,5-tetrahydro-benzo[d]azepin-3-yl)-6-(2,2,2-trifluoro-ethoxy)-pyrimidine-5-carbonitrile,
2-(3-morpholin-4-yl-propylamino)-4-(1,2,4,5-tetrahydro-benzo[d]azepin-3-yl)-6-(2,2,2-trifluoro-ethoxy)-pyrimidine-5-carbonitrile,
2-(2-hydroxy-ethylamino)-4-(1,2,4,5-tetrahydro-benzo[d]azepin-3-yl)-6-(2,2,2-trifluoro-ethoxy)-pyrimidine-5-carbonitrile or
(3-imidazol-1-yl-propylamino)-4-(1,2,4,5-tetrahydro-benzo[d]azepin-3-yl)-6-(2,2,2-trifluoro-ethoxy)-pyrimidine-5-carbonitrile.

Preferred compounds of formula I in the scope of the present invention are those, in which
R¹ is 3-[1,2,4]triazol-1-yl-propoxy and R² is -NO₂ or -CN.

The following example represents such a compound:
3-[2-Methyl-5-nitro-6-(3-[1,2,4]triazol-1-yl-propoxy)-pyrimidin-4-yl]-2,3,4,5-tetrahydro-1H-benzo [d]azepine.
Preferred compounds of formula I in the scope of the present invention are those, in which R³ and R⁴ are interconnected to the group -(CH₂)₅- and form together with the N and C atoms to which they are attached an additional 7 membered ring and R² is -NO₂ or -CN.

The following example represents such a compound:
4-Oxo-2-(1,2,4,5-tetrahydro-benzo[d]azepin-3-yl)-4,6,7,8,9,10-hexahydro-pyrimido[1,2-a]azepine-3-carbonitrile.

The term "lower alkyl" used in the present description denotes straight-chain or branched saturated hydrocarbon residues with 1-7 carbon atoms, preferably with 1-4 carbon atoms, such as methyl, ethyl, n-propyl, i-propyl and the like.
The term "lower alkylen" used in the present description denotes straight-chain or branched unsaturated hydrocarbon residues with 2-7 carbon atoms, preferably with 2-4 carbon atoms.

The term "lower alkoxy" denotes a lower alkyl residue in the sense of the foregoing definition bonded via an oxygen atom.

The term "halogen" embraces fluorine, chlorine, bromine and iodine.

The compounds of general formula I and their pharmaceutically acceptable salts can be manufactured by
a) reacting a compound of the formula 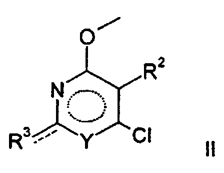 with a compound of formula 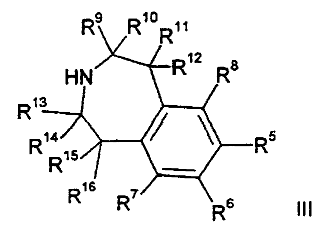 to a compound of formula 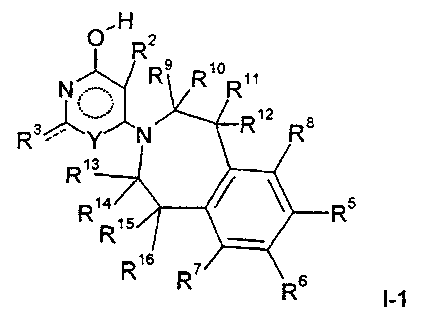 wherein the substituents are described above, or
b) reacting a compound of the formula 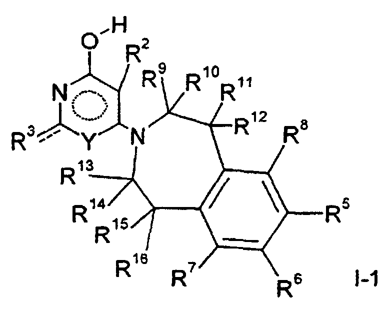 to a compound of formula 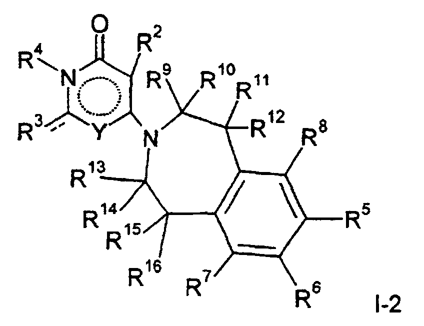 or to a compound of formula 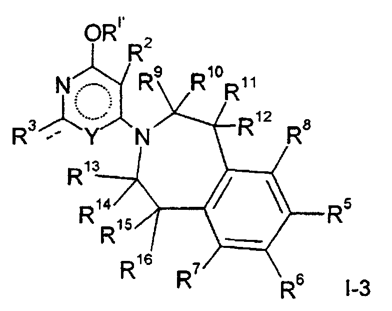 wherein R² to R¹⁶ have the significances given above and R^{I'} is lower alkyl, -(C₃-C₆)cycloalkyl, -(CHR)ₙ-(C₃-C₆)cycloalkyl, -(CHR)ₙCN, -(CHR)ₙCF₃, -(CHR)(CHR)ₙNR₂, -(CHR)(CHR)ₙOR, -(CHR)ₙ-lower alkenyl, -CF₃, -CF₂-R, -CF₂-lower alkenyl, -CHRF, -CHF-lower alkenyl, -CF₂CRF₂, -CF₂Br, -(CHR)ₙCF₂Br, -(CHR)ₙ-phenyl, wherein the phenyl group may be optionally substituted independently from each other by one to three lower alkyl, lower alkoxy, halogen, nitro or cyano groups, -(CHR)(CHR)ₙ-morpholino, -(CHR)(CHR)ₙ-pyrrolidino, -(CHR)(CHR)ₙ-piperidino, -(CHR)(CHR)ₙ-imidazolo, -(CHR)(CHR)ₙ-triazolo, -(CHR)ₙ-pyridino, -(CHR)(CHR)ₙ-OSi-lower alkyl, -(CHR)(CHR)ₙOS(O)₂-lower alkyl, -(CH₂)ₙCH=CF₂, -(CHR)ₙ-2,2-dimethyl-[1.3]dioxolane, -(CHR)ₙ-CHOR-CH₂OR or -(CHR)ₙ-CHOR-(CHR)ₙ-CH₂OR.
c) reacting a compound of formula 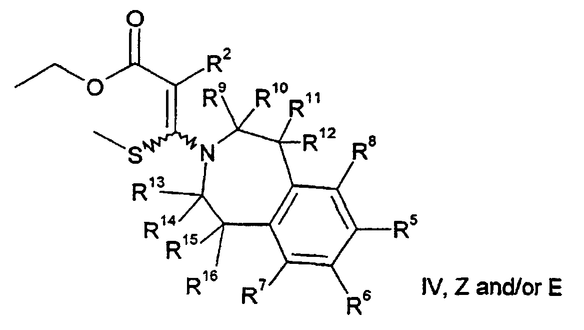 with a compound of formula 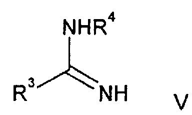 to a compound of formula 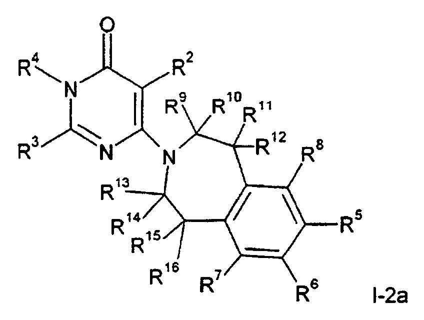 wherein the substituents are described above,
   or
d) reacting a compound of formula 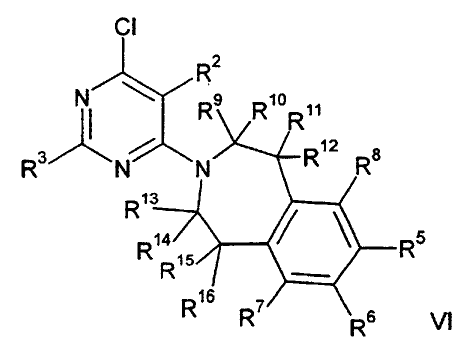 with a an alcohol, thiol, a primary or secondary amine to a compound of formula 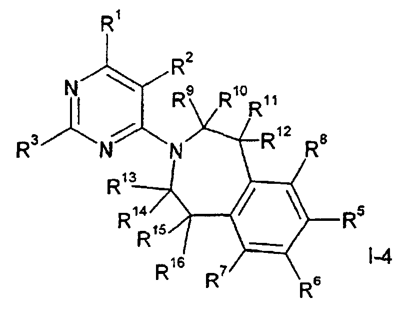 wherein the substituents are given above, or
e) reacting a compound of formula 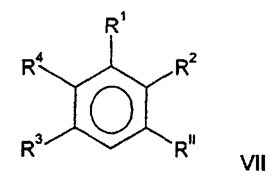 wherein R^{II} in formula VII is fluoro, chloro, bromo or a trifluoro-methansulfonyloxy group,
   with a compound of formula 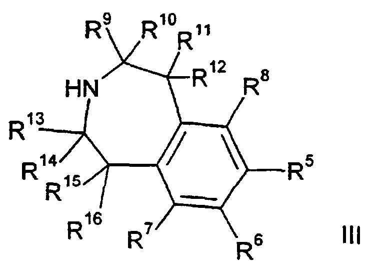 to a compound of formula 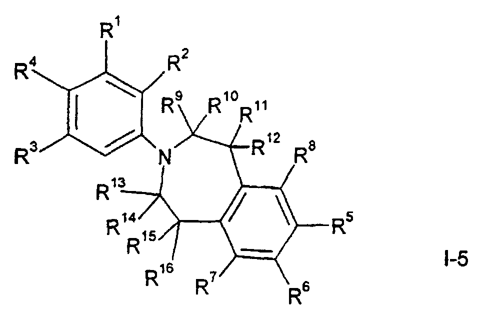 wherein the substituents are described above,
f) reacting a compound of formula 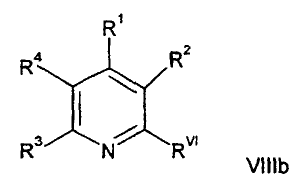 wherein, R^{VI} is a fluoro, chloro, bromo or a trifluoro-methansulfonyloxy group,
   with a compound of formula 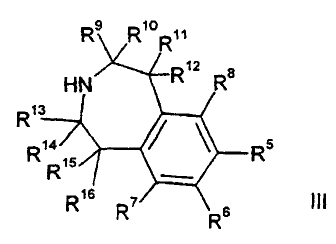 to a compound of formula I-5, 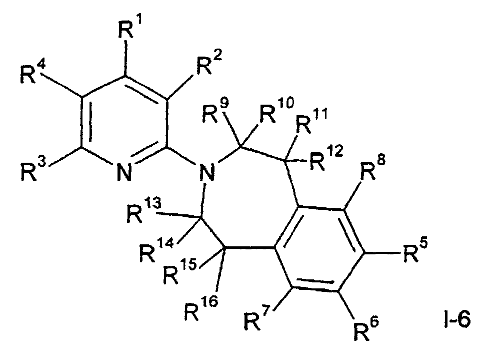 wherein the substituents are described above,
   and, if desired,
   introducing and removing protective groups in compounds of formula I, alkylating of OH or NH functions in compounds of formula I, cleaving ether functions, converting a functional group in a compound of formula I into another functional group directly or via a suitable activating group and, if desired,
   converting a compound of formula I into a pharmaceutically acceptable salt or into its optically active form.

In the following schemes I - VIII and in Examples 1 - 273 the reaction steps and reaction variants a) - f) are described in more detail.

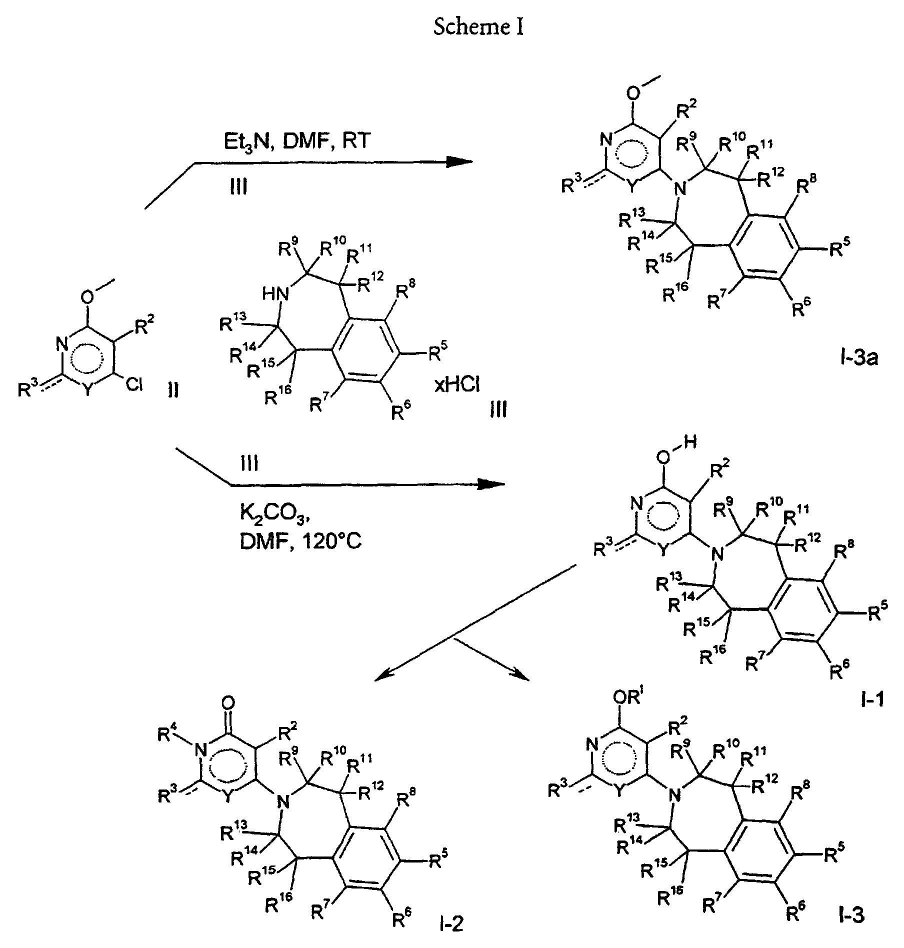

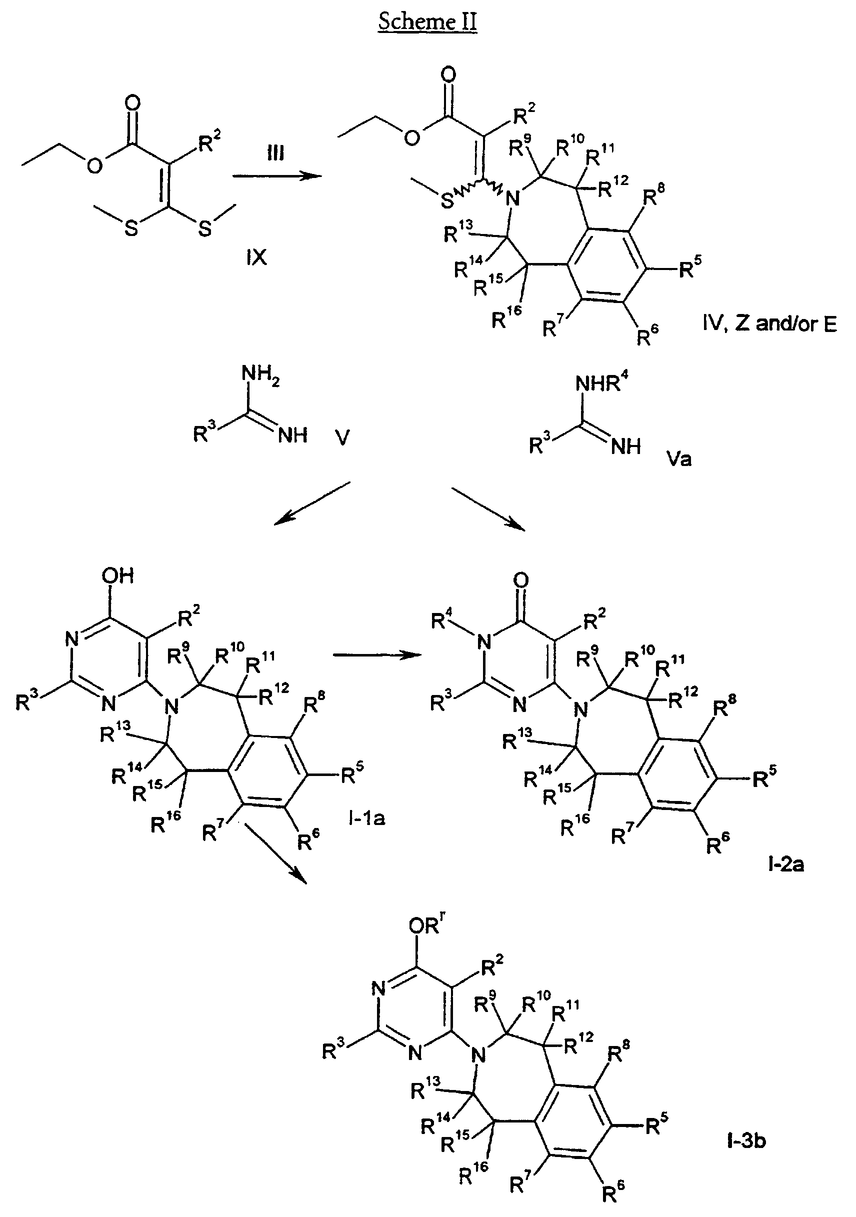

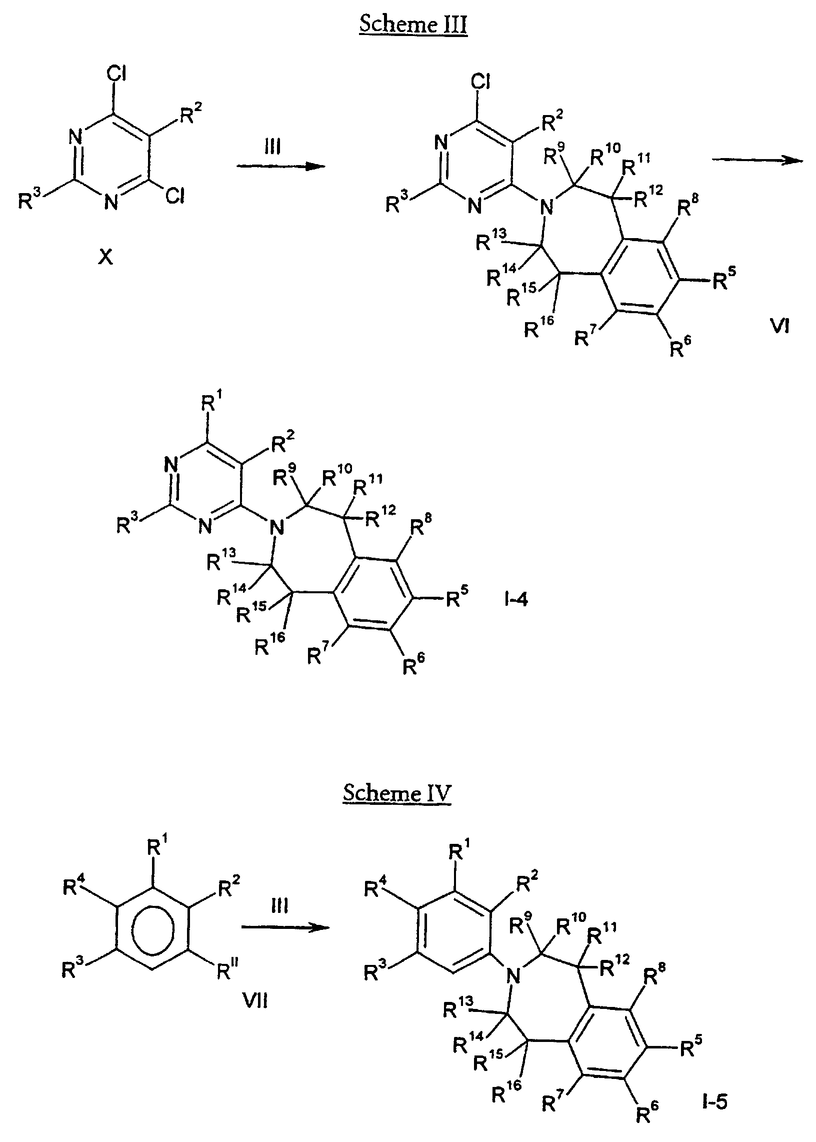

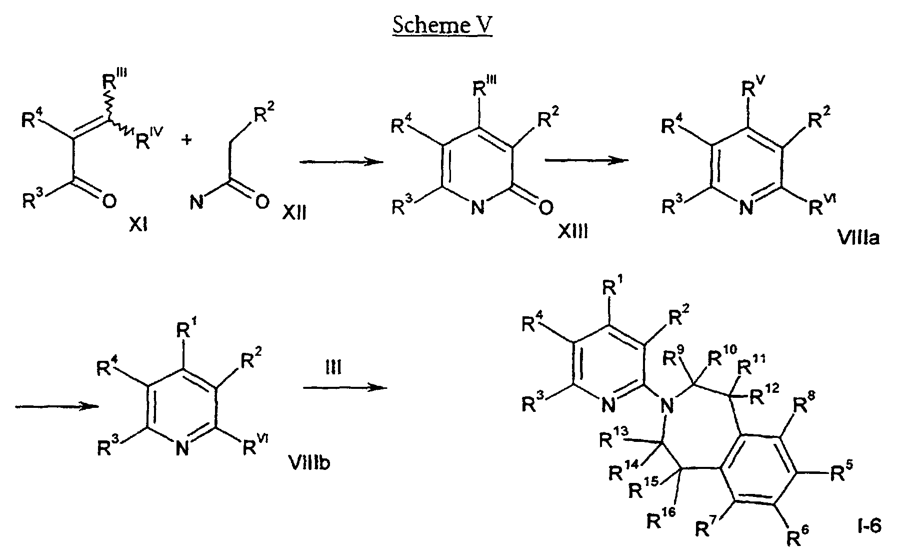

Chloro-methoxy-nitro or cyano pyrimidines II (Scheme I) are known [e.g. 6-chloro-4-methoxy-2-methyl-5-nitro-pyrimidine: Helv. (1958), 41, 1806] or can be prepared in analogy to procedures described for known compounds, e.g. from 4,6-dichloro-5-cyano-pyrimidine [Monatshefte Chemie (1965), 96, 1573-1578] and sodium methoxide in methanol at low temperature, preferably between -20 °C and + 20 °C. They react with optionally substituted secondary amines III in the presence of a base like triethylamine in solvents like N,N-dimethylformamide, dimethylsulfoxide, acetone, methyl-ethylketone or tetrahydrofuran at temperatures between 0°C and 100°C to the tertiary amines I-3a; at elevated temperatures however, preferentially 100 °C to 150 °C in the presence of potassium carbonate in solvents like N,N-dimethylformamide or N-methylpyrrolidone the tertiary amines I-1 are formed where simultaneously the methoxy group is transformed into a hydroxy group. Known analogues of II bearing a carbonyl function instead of the methoxy moiety, e.g. 2-amino-6-chloro-5-nitro-4(2H)-pyrimidinone [J. Chem. Soc. 1964, 4769-4774] react with optionally substituted secondary amines III preferentially at elevated temperatures, preferentially 100 °C to 150 °C in the presence of potassium carbonate, triethylamine or ethyl-diisopropylamine in solvents like N,N-dimethylformamide or N-methylpyrrolidone to adducts I-1. Alkylation of adducts I-1 using optionally substituted alkyl halides, tosylates, mesylates or trifluoro-methansulfonates in solvents like ethanol, methanol, dichloromethane, chloroform, N,N-dimethylformamide, dimethylsulfoxide, acetone, methyl-ethylketone or tetrahydrofuran in the presence of base like alkali carbonates, e.g. sodium, potassium or cesium carbonate, tertiary amines like triethylamine or ethyl-diisopropylamine, alkali methyl hydrides, like sodium or potassium hydride, or phase transfer catalysts like benzyl-trimethylammonium chloride in the presence of solid or concentrated aqueous sodium hydroxide gives variable mixtures of N- and/ or O-alkylated products I-2 and I-3. The products I-2 and I-3 may contain in the N- or O-alkyl function functional groups in protected form which allow further structural modifications after removal of the protective functions.

The introduction of an R⁴ substituent equal OH or NH₂ into pyrimidinoles I-1 (Scheme I) can be achieved using suitable oxygen or nitrogen transfer reagents. Chloramine or preferentially the more stable mesitylenesulfonylhydroxylamine [Synthesis 1972, 140] are suitable agents for the introduction of an NH₂ group. Both are used in solvents as ethers like tetrahydrofuran or dimethoxyethane or in N,N-dimethylformamide or dimethylsulfoxide in the presence of a base like sodium hydride or potassium carbonate at temperatures between room temperature and 60 °C. Conversion of pyrimidinoles I-1 into their O-silylated analogues by treatment with suitable silylating agents like hexamethyldisilazane and trimethylchlorosilane followed by treatment with the oxodiperoxymolybdenum (pyridine) (HMPA) complex [J. Org. Chem. 43 (1978), 188-196] in solvents like dichloromethane or chloroform at temperatures between room temperature and 60 °C gives pyrimidinones I-2 with R⁴ equal OH. Compounds I-2 with R⁴ equal OH can be further derivatized by known methods as alkylation with a suitable alkyl halide, tosylate or triflate in the presence of a base like potassium carbonate or sodium hydride in solvents like tetrahydrofuran, acetonitril or N,N-dimethylformamide at temperatures between roomt temperature and 100 °C. Compounds I-2 with R⁴ equal NH₂ are preferentially converted into a mono Boc derivative (preparation of the di-Boc derivative with di-tert. butyl dicarbonate, 4-dimethylamino pyridine in dichloromethane at room temperature followed by removal of one Boc group by stirring in dichloromethane in the presence of silica gel) and then alkylated under similar conditions as described for compounds I-2 with R⁴ equal OH. The Boc group can then easily be removed by known methods.

Bis(methylthio)-acrylates IX react with optionally substituted secondary amines III in the presence of bases like potassium carbonate and/or triethylamine in solvents like ethanol, methanol, acetone or methyl-ethylketone at temperatures between room temperature and 100 °C to adducts IV, which can be formed as Z-isomer, as mixture of E and Z isomers or as E isomer (Scheme II). Adducts IV can be reacted with amidines, urea or thiourea derivatives V either in the presence of 1,8-diazabicyclo[5.4.0]undec-7-ene in N,N-dimethylformamide or dimethylsulfoxide at temperatures between 70 °C and 140 °C or in the presence of sodium ethylate in ethanol preferentially at reflux thus yielding pyrimidineoles I-1a or pyrimidinones I-2a. Pyrimidinoles I-1a can then be alkylated as described for the sequence I-1 => I-2 and I-3 in Scheme 1. If an allyl moiety is introduced as R⁴, then, it can also serve as protective function. Thus, it allows modification at other parts of the molecules, e.g. in R³ and a later removal of the N-allyl function by lithium borohydride in the presence of palladium(II)acetate and triphenylphosphin in an inert solvent like tetrahydrofuran or 1,2-dimethoxyethane at temperatures between room temperature and 60 °C. Alternativeley, adducts IV can be reacted with substituted amidine derivatives Va in which R³ and R⁴ are optionally connected to form a 5, 6 or 7 membered ring either in the presence of 1,8-diazabicyclo[5.4.0]undec-7-ene in N,N-dimethylformamide or dimethylsulfoxide at temperatures between 70 °C and 140 °C or in the presence of sodium ethylate in ethanol preferentially at reflux yielding pyrimidinones I-2a.

Selective monosubstituion of di-chloro pyrimidines X (Scheme III) with optionally substituted secondary amines III can be performed in solvents like N,N-dimethylformamide or dimethylsulfoxide in presence of a base like triethylamine at temperatures between -10 °C and room temperature producing mono-chloro pyrimidines VI. The remaining chloro atom in compounds VI can then be replaced by i) alkoxy functions by treatment with an alcoholate in the corresponding alcohol as solvent or in an inert solvent like tetrahydrofuran, N,N-dimethylformamide or dimethylsulfoxide at temperatures between room temperature and 100 °C; by ii) amino functions by treatment with an amine in an inert solvent like tetrahydrofuran, N,N-dimethylformamide or dimethylsulfoxide at temperatures between room temperature and 100 °C; by iii) thio functions by treatment with a thiol in the presence of a base like triethylamine or sodium hydride in an alcohol, N,N-dimethylformamide or dimethylsulfoxide at temperatures between room temperature and 100 °C. The replacement of the chloro-group by a hydroxy function is preferentially performed in a two step prodecure: a 4-methoxy-benzyloxy function is introduced first by reacting VI with the corresponding alcoholate as described above followed by treatment with methanolic hydrogen chloride at temperatures between 0 °C and 50 °C.

Compounds of general formula X (Scheme III) where R³ is methylthio and R² is cyano [J.Heterocycl.Chem. (1971), 8(3), 445] or R² is nitro [Aust.J.Chem. (1990), 43(1), 55] are known. Selective monosubstitution with optionally substituted secondary amines of general formula III giving compounds VI and, thereupon, substitution of the remaining chloro atom can be performed as described above to yield compounds of general formula I-4. After conversion of the 2-methylthio derivatives, already appropriately substituted in the 6-position of the pyrimidines, into the 2-methylsulphonyl derivatives according to known oxidative methods, the corresponding O-, N- or S-substituted pyrimidine derivatives can be obtained by treatment with alcoholates, amines and thiolates in tetrahydrofuran, 1,2-dimethoxyethane, dimethylformamide or dimethyl sulphoxide at temperatures between room temperature and about 150 °C.

Compounds of general formula I-4, where R² is cyano, R¹ is methylthio and R³ is amino, also can be synthesized by reacting 2-amino-4-bromo-6-methylsulfanyl-pyrimidine-5-carbonitrile, prepared in an analogous manner to the 4-chloro derivative described in J.Chem.Soc. Chem.Commun. 1974, 9, 350, with optionally substituted secondary amines of general formula III. Furthermore, compounds of general formula I-4, where R² is cyano, R¹ is alkylthio can be synthesized starting from compounds of general formula VI, where R³ is methylthio, by its transformation into the 2-methylsulphonyl derivatives according to known oxidative methods, followed by the treatment with alcoholates, amines and thiolates in tetrahydrofuran, 1,2-dimethoxyethane, dimethylformamide or dimethylsulfoxide at temperatures between room temperature and about 150 °C. Thereupon, substitution of the remaining chloro atom by alkylthiolates in tetrahydrofurane, 1,2-dimethoxyethane, dimethylformamide or dimethylsulfoxide at temperatures between room temperature and about 150 °C yields the O-, N- or S-substituted 6-alkylthio pyrimidin derivatives.

Alpha amino substituted nitro or cyano benzene compounds I-5 (Scheme IV) are prepared from the corresponding known benzene derivatives VII with R^{II} being a fluoro, chloro, bromo or a trifluoro-methansulfonyloxy function by treatment with secondary amines III at temperatures preferentially between room temperature and 100 °C in the presence of a base like potassium carbonate or triethylamine in solvents like methanol, ethanol, acetonitrile, tetrahydrofuran, acetone, methyl-ethylketone, N,N-dimethylformamide or dimethylsulfoxide. Cyano or nitro pyridones XIII are known or can be prepared from unsaturated ketones XI (Scheme V) bearing a leaving group R^{III} or R^{IV} being an ONa or an S-alkyl function together with a second functionality R^{III} or R^{IV} being a hydrogen or an alkoxy function. Such unsaturated ketones XI can be condensed with cyano or nitro acetamide either with mixed acid base catalysis using a mixture of a base like piperidine or pyrrolidine with acetic or formic acid in solvents water, ethanol and tetraydrofuran or in the presence of a base like sodium hydride or a sodium or potassium alkoholate in solvents like ethanol, methanol, tert.-butanol, N,N-dimethylformamide or dimethylsulfoxide at temperatures between room temperature and 120 °C leading to pyridones XIII with R^{III} being a hydrogen or an alkoxy function. R^{III} equal alkoxy in compounds XIII can optionally be transformed into R^{III} equal OH by known methods as boron tribromide in dichloromethane. The transformation of pyridones XIII into pyridines VIIIa bearing a chloro group R^{VI} and optionally a second chloro group R^{V} can be performed by known methods as by phosphorous pentachloride neat, by mixtures of phosphorous pentachloride and phosphorous oxychloride with and without additional bases and solvents like ethyl-diisopropyl amine and acetonitrile at temperatures between 80 °C and 140 °C. Alternatively pyridines VIIIa bearing two trifluoro-methanesulfonyloxy groups R^{V} and R^{VI} [known for R² = NO₂: US 5352784 A (1994)], can be prepared form pyridones XIII (R^{III} equal OH) and trifluoro-methanesulfonic acid anhydride and a base like triethylamine in an inert solvent like dichloromethane at temperatures between -40 °C and 60 °C. Compounds VIIIa with two identical leaving groups R^{V} and R^{VI} equal to chloro or trifluoro-methanesulfonyloxy groups react with nucleophiles such as primary and secondary alcoholates in solvents like tetrahydrofuran or N,N-dimethylformamide, water (pH 8 to 14) in the presence of a water miscible solvent like tetrahydrofuran or with primary or secondary amines in solvents like dichloromethane, tetrahydrofuran or N,N-dimethylformamide preferentially at room temperature by first substituting R^{V} by an alkoxy, hydroxy or an amino substituent R^{I} thus producing compounds VIIIb. Compounds VIIIb with only the R^{VI} leaving group left can then be reacted with secondary amines III at temperatures preferentially between room temperature and 100 °C in the presence of a base like potassium carbonate or triethylamine in solvents like methanol, ethanol, acetonitrile, tetrahydrofuran, acetone, methyl-ethylketone, N,N-dimethylformamide or dimethylsulfoxide leading to derivatives I-6.

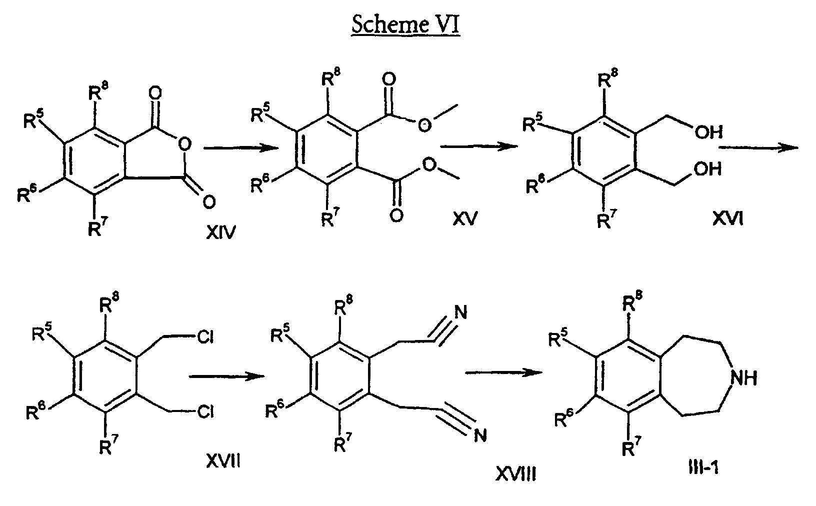

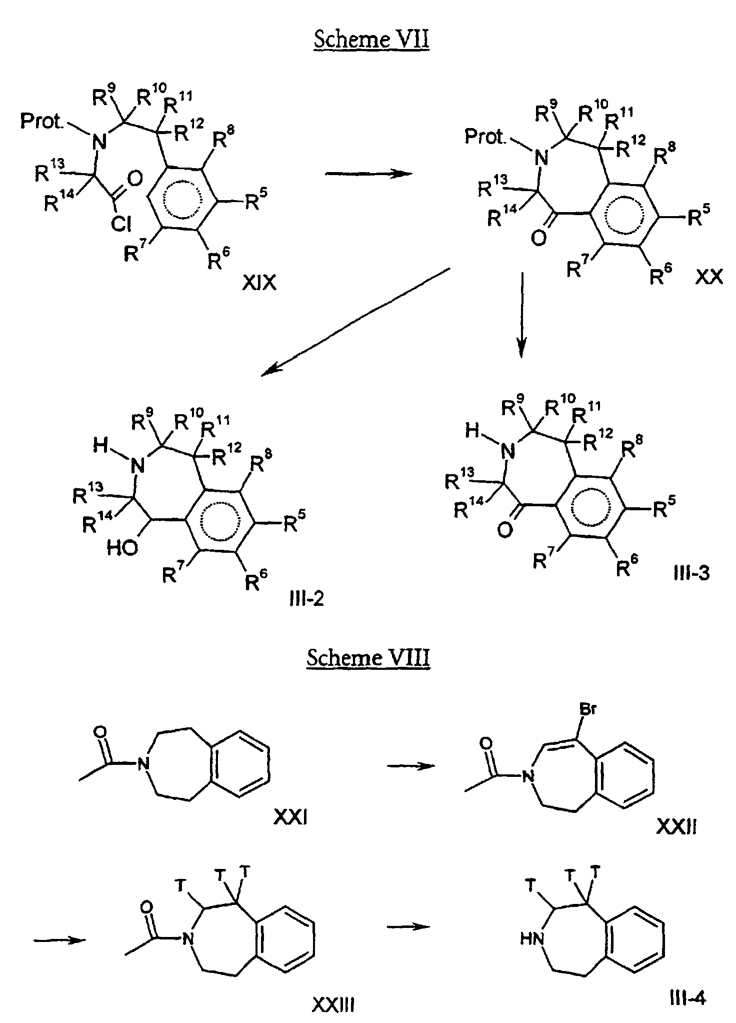

Benzazepines III-1 with various substitution patterns in the benzene part optionally bearing additional alkyl substituents in the azepine ring (e.g. 1,1,5,5-tetramethyl-2,3,4,5-tetrahydro-1H-3-benzazepine [Ger. Offen., DE 1921861 691120; CAS 72:31646]) are known [see e.g.: J. Heterocycl. Chem. (1971), 8(5), 779-83]. Alternatively they can be prepared as outlined in Scheme VI [compare J. Med. Chem. (1984) 27, 918-921 describing a similar reaction sequence]: Transformation of optionally substituted phthalic anhydrides XIV into the corresponding dimethylesters using sulfuric acid in methanol at reflux, followed by reduction of the diester with lithiumaluminium hydride in ether or tetrahydrofuran between room temperature and 60 °C and transformation of the so formed diols XVI using thionylchloride in a solvent like toluene or dichloromethane in the presence of a base like pyridine between room temperature and 60 °C yields dichlorides XVII. The further transformation of dichlorides XVII into dinitriles XVIII can be performed using sodium or potassium cyanide in solvents like dimethylsulfoxide or N,N-dimethylformamide between room temperature and 80 °C. Reductive cyclisation of dinitriles XVIII into benzazepines III-1 can then be performed with raney nickel in a mixture of conc. aq. ammonia and ethanol at temperatures around 100 °C as described in J. Heterocycl. Chem. (1971), 8(5), 779-83.

Benzazepines III-2 and III-3 bearing a keto respectively hydroxy function at the benzylic position of the azepine ring can be prepared in close analogy to the procedure described for thieno[2,3-d]azepines [J. Heterocyclic Chemistry 22, 1011 (1985)] (Scheme VII): precursor acid chlorides XIX bearing preferentially a tosyloxy protective function at the secondary nitrogen function are cyclized in an inert solvent like 1,2-dichloroethane, dichloromethane or nitrobenzene in the presence of a Lewis acid catalyst like aluminium trichloride, tin tetrachloride or phosphorous pentachloride at temperatures between -40 °C and 80 °C to yield the protected ketones XX. Keto benzazepines III-3 are then prepared by cleavage of N-tosyl function with hydrobromic acid in the presence of a scavenger reagent like phenol in a solvent like ethyl acetate at room temperature, whereas hydroxy benzazepines III-2 can be obtained by simultaneous reduction of the keton function and removal of the N-tosyl protective function by treatment with sodium bis(methoxyethoxy)aluminium-hydride in toluene at reflux.

A labeled amine as the 1,1,2-tritritio-2,3,4,5-tetrahydro-1H-benzo[d]azepine III-4 usable as precursor for the preparation of a labeled compound I according to synthesis schemes I - V can be prepared as outlined in Scheme VIII. The 1-(5-bromo-1,2-dihydro-benzo[d]azepin-3-yl)-ethanone XXII can be prepared by reaction of the 1-(1,2,4,5-tetrahydro-benzo[d]azepin-3-yl)-ethanone XXI [J. Heterocycl. Chem. (1971), 8(5), 779-83] with N-bromosuccinimide in carbon tetrachloride in the presence of a radical initiator like dibenzoylperoxide or 1,1'-azobis-(cyclohexanecarbonitrile) preferentially at reflux. Hydrogenation of the 1-(5-bromo-1,2-dihydro-benzo[d]azepin-3-yl)-ethanone XXII with tritium gas using a palladium or platinum catalyst in solvents methanol, ethanol or an ether like tetrahydrofuran preferentially in the presence of a base like triethylamine gives the 1-(1,1,2-tritritio-1,2,4,5-tetrahydro-benzo[d]azepin-3-yl)-ethanone XXIII which can be converted into the 1-(1,1,2-tritritio-1,2,4,5-tetrahydro-benzo[d]azepin-3-yl)-ethanone III-4 with conc. aq. hydrochloric acid in methanol.

The pharmaceutically acceptable salts can be manufactured readily according to methods known per se and taking into consideration the nature of the compound to be converted into a salt. Inorganic or organic acids such as, for example, hydrochloric acid, hydrobromic acid, sulphuric acid, nitric acid, phosphoric acid or citric acid, formic acid, fumaric acid, maleic acid, acetic acid, succinic acid, tartaric acid, methanesulphonic acid, p-toluenesulphonic acid and the like are suitable for the formation of pharmaceutically acceptable salts of basic compounds of formula I. Compounds which contain the alkali metals or alkaline earth metals, for example sodium, potassium, calcium, magnesium or the like, basic amines or basic amino acids are suitable for the formation or pharmaceutically acceptable salts of acidic compounds of formula I.

The compounds of formula I and their pharmaceutically acceptable salts are, as already mentioned above, metabotropic glutamate receptor antagonists and can be used for the treatment or prevention of acute and/or chronic neurological disorders, such as epilepsy, stroke, chronic and acute pain, psychosis, schizophrenia, Alzheimer's disease, cognitive disorders, memory deficits and psychosis. Other treatable indications are restricted brain function caused by bypass operations or transplants, poor blood supply to the brain, spinal cord injuries, head injuries, hypoxia caused by pregnancy, cardiac arrest and hypoglycaemia. Further treatable indications are Huntington's chorea, ALS, dementia caused by AIDS, eye injuries, retinopathy, idiopathic parkinsonism or parkinsonism caused by medicaments as well as conditions which lead to glutamate-deficient functions, such as e.g. muscle spasms, convulsions, migraine, urinary incontinence, nicotine addiction, psychoses, opiate addiction, anxiety, vomiting, dyskinesia and depression.

The compounds of the present invention are group I mGluR antagonists:

### a) functional assay for the characterization of mGluR 1 antagonistic properties

cDNA encoding rat mGluR1a receptor obtained from Prof. S. Nakanishi (Kyoto, Japan) was transiently transfected into HEK-EBNA cells using a procedure described by Schlaeger et. al., New Dev. New Appl. Anim. Cell Techn., Proc. ESACT Meet., 15^{th} ( 1998), 105-112 and 117-120. [Ca²⁺]i measurements were performed on mGluR1a transfected HEK-EBNA cells after incubation of the cells with Fluo-3 AM (0.5 µM final concentration) for 1 hour at 37 °C followed by 4 washes with assay buffer (DMEM supplemented with Hank's salt and 20 mM HEPES. [Ca²⁺]i measurements were done using a fluorometric imaging plate reader (FLIPR, Molecular Devices Corporation, La Jolla, CA, USA). 10 µM glutamate as agonist was used to evaluate the potency of the antagonists.

Increasing concentrations of antagonists were applied to the cells 5 minutes prior to the application of the agonist. The inhibition (antagonists) curves were fitted with a four parameter logistic equation giving IC₅₀, and Hill coefficient using the iterative nonlinear curve fitting software Origin (Microcal Software Inc., Northampton, MA, USA).

The preferred compounds have an IC₅₀ range of 0.001-1.00 µM (F-IC₅₀).

### b) binding assay for the characterization of mGluR 1 antagonistic properties

Binding assay with tritiated 1-ethyl-2-methyl-6-oxo-4-(1,1,2-tritritio-1,2,4,5-tetrahydro-benzo[d]azepin-3-yl)-1,6-dihydro-pyrimidine-5-carbonitrile: HEK 293 cells were transiently transfected with the rat mGluR1a receptor. The cells were collected and washed 3 times with PBS. The cell pellets were frozen at -80 °C. Membranes were prepared from HEK 293 cells transfected with the rat mGluR1a receptor and used in the binding experiments at 10 µg proteins per assay after resuspension in a HEPES NaOH 20mM, pH=7.4 binding buffer. 1-Ethyl-2-methyl-6-oxo-4-(1,1,2-tritritio-1,2,4,5-tetrahydro-benzo[d]azepin-3-yl)-1,6-dihydro-pyrimidine-5-carbonitrile (S.A 33.4Ci/mmol) was used at 3 nM final concentration. The incubation with variable concentrations of potential inhibitors was performed for 1 hour at room temperature, the incubate was then filtered onto GF/B glass fiber filter preincubated 1 hour in PEI 0,1% and washed 3 times with 1ml of cold binding buffer. The radioactivity retained on the unifilter 96 was counted using a Topcount β counter. After correction for non specific binding the data were normalized and the IC₅₀ value calculated using a 4 parameters logistic equation which was fitted to the inhibition curve.

The preferred compounds have an IC₅₀ range of 0.001-1.00 µM (B-IC₅₀).

In the table below are shown some specific activity data of preferred compounds:

| **Example** | **F-IC** _{**50**} **(µM)** | **B-IC** _{**50**} **(µM )** |
|---|---|---|
| 220 | 0.038 | 0.002 |
| 30 | 0.009 | 0.003 |
| 190 | 0.20 | 0.007 |
| 154 | 0.21 | 0.01 |
| 78 | 0.026 | 0.011 |
| 249 | 0.023 | 0.011 |
| 25 | 0.005 | 0.015 |
| 11 | 0.008 | 0.018 |
| 214 | 0.12 | 0.020 |
| 132 | 0.014 | 0.080 |
| 174 | 0.97 | 0.088 |
| 17 | 0.088 | 0.33 |
| 126 | 0.10 | 0.72 |

The compounds of formula I and pharmaceutically acceptable salts thereof can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. However, the administration can also be effected rectally, e.g. in the form of suppositories, or parenterally, e.g. in the form of injection solutions.

The compounds of formula I and pharmaceutically acceptable salts thereof can be processed with pharmaceutically inert, inorganic or organic carriers for the production of pharmaceutical preparations. Lactose, corn starch or derivatives thereof, talc, stearic acid or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like; depending on the nature of the active substance no carriers are, however, usually required in the case of soft gelatine capsules. Suitable carriers for the production of solutions and syrups are, for example, water, polyols, sucrose, invert sugar, glucose and the like. Adjuvants, such as alcohols, polyols, glycerol, vegetable oils and the like, can be used for aqueous injection solutions of water-soluble salts of compounds of formula I, but as a rule are not necessary. Suitable carriers for suppositories are, for example, natural or hardened oils, waxes, fats, semi-liquid or liquid polyols and the like.

In addition, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

As mentioned earlier, medicaments containing a compound of formula I or a pharmaceutically acceptable salt thereof and a therapeutically inert excipient are also an object of the present invention, as is a process for the production of such medicaments which comprises bringing one or more compounds of formula I or pharmaceutically acceptable salts thereof and, if desired, one or more other therapeutically valuable substances into a galenical dosage form together with one or more therapeutically inert carriers.

The dosage can vary within wide limits and will, of course, be fitted to the individual requirements in each particular case. In general, the effective dosage for oral or parenteral administration is between 0.01-20 mg/kg/day, with a dosage of 0.1-10 mg/ kg/day being preferred for all of the indications described. The daily dosage for an adult human being weighing 70 kg accordingly lies between 0.7-1400 mg per day, preferably between 7 and 700 mg per day.

Finally, as mentioned earlier, the use of compounds of formula I and of pharmaceutically acceptable salts thereof for the production of medicaments, especially for the control or prevention of acute and/or chronic neurological disorders of the aforementioned kind, is also an object of the invention.

### Example 1

### 2-Methyl-5-nitro-6-(1,2,4,5-tetrahydro-benzo[d]azepin-3-yl)-pyrimidin-4-ol

A suspension of 2.0 g (9.8 mmol) of 2-methyl-4-methoxy-5-nitro-6-chloro-pyrimidine [Helv. (1958), 41, 1806], 1.98 g (10.8 mmol) 2,3,4,5-tetrahydro-1H-benzo[d]azepine hydrochloride [J. Heterocycl. Chem. (1971), 8(5), 779-83] and 4.08 g (29.5 mmol) potassium carbonate in 40 ml N,N-dimethylformamide was stirred at 120 °C for 2 hours. The reaction mixture was then allowed to cool to room temperature, poured into 150 ml of an ice/water mixture and extracted three times with 200 ml of dichloromethane. The combined organic phases were washed twice with 100 ml of water, dried over magnesium sulphate, evaporated under reduced pressure and dried in a high vacuum. Crystallization from ethyl acetate/methanol gave 1.95 g (6.5 mmol), 66.1%, 2-methyl-5-nitro-6-(1,2,4,5-tetrahydro-benzo[d]azepin-3-yl)-pyrimidin-4-ol as a yellow solid; m.p. >200 °C; MS: [M+H]⁺ = 301.

### Example 2

### 3-Ethyl-2-methyl-5-nitro-6-(1,2,4,5-tetrahydro-benzo[d]azepin-3-yl)-3H-pyrimidin-4-one

From 2-methyl-5-nitro-6-(1,2,4,5-tetrahydro-benzo[d]azepin-3-yl)-pyrimidin-4-ol and iodoethane, potassium carbonate in N,N-dimethylformamide (r.t.), yellow solid; m.p. 145-147°C; MS: [M+H]⁺ = 329; see example 3.

### Example 3

### 3-(6-Ethoxy-2-methyl-5-nitro-pyrimidin-4-yl)-2,3,4,5-tetrahydro-1H-benzo[d]azepine

A suspension of 0.30 g (1.0 mmol) of 2-methyl-5-nitro-6-(1,2,4,5-tetrahydro-benzo[d]azepin-3-yl)-pyrimidin-4-ol (example 1), 0.24 g (1.5 mmol) ethyl iodide and 0.28 g (2.0 mmol) potassium carbonate in 10.0 ml N,N-dimethylformamide was stirred at room temperature for 24 h. The reaction mixture was then poured into 50 ml of an ice/water mixture and extracted three times with 100 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 9:1 to 1:1 v/v mixture of hexane and ethyl acetate as eluent to yield 0.245 g (0.746 mmol), 74.6 %, of 3-ethyl-2-methyl-5-nitro-6-(1,2,4,5-tetrahydro-benzo[d]azepin-3-yl)-3H-pyrimidin-4-one as yellow solid; m.p. 145-147 °C; MS: [M+H]⁺ = 329; and 0.070 g (0.213 mmol), 21.3 %, of 3-(6-ethoxy-2-methyl-5-nitro-pyrimidin-4-yl)-2,3,4,5-tetrahydro-1H-benzo[d]azepine as yellow oil; MS: [M+H]⁺ = 329.

### Example 4

### 3-(6-Methoxy-2-methyl-5-nitro-pyrimidin-4-yl)-2,3,4,5-tetrahydro-1H-benzo[d]azepine

A solution of 0.204 g (1.0 mmol) of 2-methyl-4-methoxy-5-nitro-6-chloro-pyrimidine [Helv. (1958), 41, 1806], 0.20 g (1.1 mmol) 2,3,4,5-tetrahydro-1H-benzo[d]azepine hydrochloride [J. Heterocycl. Chem. (1971), 8(5), 779-83] and 0.30 g (3.0 mmol) triethylamine in 10.0 ml N,N-dimethylformamide was stirred at room temperature for 60 h. The reaction mixture was then poured into 50 ml of an ice/water mixture and extracted three times with 60 ml of dichloromethane. The combined organic phases were washed twice with 50 ml of water, dried over magnesium sulphate, evaporated under reduced pressure and dried in a high vacuum. The residue obtained was then crystallized from dichloromethane/hexane to yield 0.28 g (0.9 mmol), 90%, 3-(6-methoxy-2-methyl-5-nitro-pyrimidin-4-yl)-2,3,4,5-tetrahydro-1H-benzo[d]azepine as a yellow solid; m.p. 123-128°C.

### Example 5

### 2,3-Dimethyl-5-nitro-6-(1,2,4,5-tetrahydro-benzo[d]azepin-3-yl)-3H-pyrimidin-4-one

In analogy to the procedure described in example 3, the 2-methyl-5-nitro-6-(1,2,4,5-tetrahydro-benzo[d]azepin-3-yl)-pyrimidin-4-ol was treated with methyl iodide in N,N-dimethylformamide in the presence of potassium carbonate to yield the 2,3-dimethyl-5-nitro-6-(1,2,4,5-tetrahydro-benzo[d]azepin-3-yl)-3H-pyrimidin-4-one as yellow foam; MS: [M+H]⁺ = 315.

### Example 6

### 3-Butyl-2-methyl-5-nitro-6-(1,2,4,5-tetrahydro-benzo[d]azepin-3-yl)-3H-pyrimidin-4-one

From 2-methyl-5-nitro-6-(1,2,4,5-tetrahydro-benzo[d]azepin-3-yl)-pyrimidin-4-ol and 1-iodobutane, potassium carbonate in DMF (r.t.) yellow solid; m.p. 158-161 °C; MS: [M+H]⁺ = 357; see example 7.

### Example 7

### 3-(6-Butoxy-2-methyl-5-nitro-pyrimidin-4-yl)-2,3,4,5-tetrahydro-1H-benzo[d]azepine

In analogy to the procedure described in example 3 the 2-methyl-5-nitro-6-(1,2,4,5-tetrahydro-benzo[d]azepin-3-yl)-pyrimidin-4-ol was treated with 1-iodobutane in N,N-dimethylformamide in the presence of potassium carbonate to yield the 3-butyl-2-methyl-5-nitro-6-(1,2,4,5-tetrahydro-benzo[d]azepin-3-yl)-3H-pyrimidin-4-one as yellow solid; m.p. 158-161 °C; MS: [M+H]⁺ = 357; and the 3-(6-butoxy-2-methyl-5-nitro-pyrimidin-4-yl)-2,3,4,5-tetrahydro-1H-benzo[d]azepine as yellow oil; MS: [M+H]⁺ = 357.

### Example 8

### 3-Isobutyl-2-methyl-5-nitro-6-(1,2,4,5-tetrahydro-benzo[d]azepin-3-yl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol was treated with 1-iodo-2-methyl-propane in N,N-dimethylformamide at 80 °C in the presence of potassium carbonate to yield the 3-isobutyl-2-methyl-5-nitro-6-(1,2,4,5-tetrahydro-benzo[d]azepin-3-yl)-3H-pyrimidin-4-one as light brown oil; MS: [M+H]⁺ = 357.

### Example 9

### 3-Isopropyl-2-methyl-5-nitro-6-(1,2,4,5-tetrahydro-benzo[d]azepin-3-yl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol was treated with 2-iodopropane in N,N-dimethylformamide in the presence of potassium carbonate to yield the 3-isopropyl-2-methyl-5-nitro-6-(1,2,4,5-tetrahydro-benzo[d]azepin-3-yl)-3H-pyrimidin-4-one as yellow oil; MS: [M+H]⁺ = 343.

### Example 10

### 3-(2-Fluoro-ethyl)-2-methyl-5-nitro-6-(1,2,4,5-tetrahydro-benzo[d]azepin-3-yl)-3H-pyrimidin-4-one

In analogy to the procedure described in example 3 the 2-methyl-5-nitro-6-( 1,2,4,5-tetrahydro-benzo[d]azepin-3-yl)-pyrimidin-4-ol was treated with 1-bromo-2-fluoroethane in N,N-dimethylformamide in the presence of potassium carbonate at 50 °C to yield the 3-(2-fluoro-ethyl)-2-methyl-5-nitro-6-(1,2,4,5-tetrahydro-benzo[d]azepin-3-yl)-3H-pyrimidin-4-one as yellow oil; MS: [M+H]⁺ = 347.

### Example 11

### 2-Methyl-5-nitro-6-(1,2,4,5-tetrahydro-benzo[d]azepin-3-yl)-3-(2,2,2-trifluoro-ethyl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol was treated with 2,2,2-trifluoroethyl iodide in N,N-dimethylformamide in the presence of potassium carbonate at 80 °C to yield the 2-methyl-5-nitro-6-(1,2,4,5-tetrahydro-benzo[d]azepin-3-yl)-3-(2,2,2-trifluoro-ethyl)-3H-pyrimidin-4-one as light brown oil; MS: [M+H]⁺ = 383.

### Example 12

### 2-Methyl-5-nitro-3-propyl-6-(1,2,4,5-tetrahydro-benzo[d]azepin-3-yl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol was treated with 1-chloropropane in N,N-dimethylformamide in the presence of potassium carbonate at 50 °C to yield 2-methyl-5-nitro-3-propyl-6-(1,2,4,5-tetrahydro-benzo[d]azepin-3-yl)-3H-pyrimidin-4-one as yellow solid; m.p. 164-170°C; MS: [M+H]⁺ = 343.

### Example 13

### 2-Amino-6-oxo-4-(1,2,4,5-tetrahydro-benzo[d]azepin-3-yl)-1,6-dihydro-pyrimidine-5-carbonitrile

a) A solution of 2.13 g (9.80 mmol) of ethyl 2-cyano-3,3-bis(methylthio)acrylate and 1.80 g (9.80 mmol) 2,3,4,5-tetrahydro-1H-benzo[d]azepine hydrochloride [J. Heterocycl. Chem. (1971), 8(5), 779-83], 1.19 g (11.8 mmol) triethylamine and 0.5 g (3.6 mmol) potassium carbonate in 15 ml of ethanol was heated at reflux for 8 h. The reaction mixture was then evaporated and the residue chormatographed on silica gel using a 97:3 v/v mixture of dichloromethane and ether as eluent. Thus, 2.0 g (6.3 mmol), 64 %, of E and/or Z 2-cyano-3-methylsulfanyl-3-(1,2,4,5-tetrahydro-benzo[d]azepin-3-yl)-acrylic acid ethyl ester were obtained as a yellowish solid; m.p. 88-93 °C; MS: [M]⁺ = 316.
b) A solution of 0.253 g (0.80 mmol) of E and/or Z 2-cyano-3-methylsulfanyl-3-(1,2,4,5-tetrahydro-benzo[d]azepin-3-yl)-acrylic acid ethyl ester, 0.199 g (1.60 mmol) guanidine nitrate and 0.376 g (2.40 mmol) 1,8-diazabicyclo[5.4.0]undec-7-ene in 1.0 ml of N,N-dimethylformamide was heated at 100 °C for 2 h. Then, the reaction mixture was poured into 10 ml of ice-water, acidified with 1 N hydrogen chloride and the precipitate formed filtered off and washed with water followed by ether. There were thus obtained 0.180 g (0.64 mmol), 80 %, of 2-amino-6-oxo-4-(1,2,4,5-tetrahydro-benzo[d]azepin-3-yl)-1,6-dihydro-pyrimidine-5-carbonitrile as a colorless solid; m.p. >200 °C; MS: [M]⁺ = 281.

### Example 14

### 2,4-Dioxo-6-(1,2,4,5-tetrahydro-benzo[d]azepin-3-yl)-1,2,3,4-tetrahydro-pyrimidine-5-carbonitrile

A solution of 0.158 g (0.50 mmol) of Z and/or E 2-cyano-3-methylsulfanyl-3-(1,2,4,5-tetrahydro-benzo[d]azepin-3-yl)-acrylic acid ethyl ester [example 13 a)], 0.142 g (0.50 mmol) of S-methylthiourea sulfate and 0.228 g (1.50 mmol) 1,8-diazabicyclo[5.4.0]undec-7-en in 1.0 ml N,N-dimethylformamide was stirred at 100 °C for 3 hours. The reaction mixture was then poured into 50 ml of ice-water and filtered off, the filtrate was acidified with 1 N hydrogen chloride and filtered again. The combined residues were chromatographed on silica gel using a 2:1 v/v mixture of dichloromethane and ethyl acetate as eluent. There were thus obtained 0.063 g (0.192 mmol), 38 %, of Z and/or E 2-cyano-3-(1,2,4,5-tetrahydro-benzo[d]azepin-3-yl)-3-ureido-acrylic acid ethyl ester as colorless solid; m.p. 183-186 °C; MS: [M]⁺ = 328; and 0.0087 g (0.031 mmol), 6.1%, of 2,4-dioxo-6-(1,2,4,5-tetrahydro-benzo[d]azepin-3-yl)-1,2,3,4-tetrahydro-pyrimidine-5-carbonitrile as colorless solid; m.p. >200 °C; MS: [M+H]⁺ = 283.

### Example 15

### 6-Oxo-4-(1,2,4,5-tetrahydro-benzo[d]azepin-3-yl)-1,6-dihydro-pyrimidine-5-carbonitrile

0.070 g (3.0 mmol) sodium were dissolved in 8.0 ml of ethanol, then 0.316 g (1.0 mmol) of Z and/or E 2-cyano-3-methylsulfanyl-3-(1,2,4,5-tetrahydro-benzo[d]azepin-3-yl)-acrylic acid ethyl ester [example 13 a)] and 0.164 g (2.0 mmol) of formamidine hydrochloride were added and the reaction mixture heated under reflux for 4 h. After evaporation of the solvent, the residue was treated with 30 ml of ice-water followed by 1 N hydrogen chloride and extracted three times with 50 ml of a 95:5 v/v mixture of dichloromethane and methanol. The combined organic extracts were dried over magnesium sulfate and evaporated again. The residue formed was then chromatographed on silica gel using a 98:2 v/v mixture of dichloromethane and methanol as eluent. Thus yielding 0.144 g (0.054 mmol), 54%, of 6-oxo-4-(1,2,4,5-tetrahydro-benzo[d]azepin-3-yl)-1,6-dihydro-pyrimidine-5-carbonitrile as colorless solid; m.p. >200 °C; MS: [M]⁺ = 266.

### Example 16

### 4-Oxo-6-(1,2,4,5-tetrahydro-benzo[d]azepin-3-yl)-2-thioxo-1,2,3,4-tetrahydro-pyrimidine-5-carbonitrile

In analogy to the procedure described in example 15 the Z and/or E 2-cyano-3-methylsulfanyl-3-(1,2,4,5-tetrahydro-benzo[d]azepin-3-yl)-acrylic acid ethyl ester [example 13 a)] and thiourea dissolved in ethanol where heated at reflux in the presence of sodium ethylate to yield the 4-oxo-6-(1,2,4,5-tetrahydro-benzo[d]azepin-3-yl)-2-thioxo-1,2,3,4-tetrahydro-pyrimidine-5-carbonitrile as colorless solid; m.p. >200 °C; MS: [M]⁺ = 299.

### Example 17

### 2-Methyl-6-oxo-4-(1,2,4,5-tetrahydro-benzo[d]azepin-3-yl)-1,6-dihydro-pyrimidine-5-carbonitrile

A solution of 0.158 g (0.50 mmol) of Z and/or E 2-cyano-3-methylsulfanyl-3-(1,2,4,5-tetrahydro-benzo[d]azepin-3-yl)-acrylic acid ethyl ester [example 13 a)], 0.0975 g (1.0 mmol) acetamidine hydrochloride and 0.235 g (1.5 mmol) 1,8-diazabicyclo[5.4.0]undec-7-en in 1.0 ml N,N-dimethylformamide was stirred at 100 °C for 2 hours. The reaction mixture was then poured into 30 ml of ice-water and acidified with 1 N hydrogen chloride. The residue formed was filtered off and then chromatographed on silica gel using a 95:5 v/v mixture of dichloromethane and methanol as eluent. There was thus obtained 0.0965 g (0.34 mmol), 69 %, of 2-methyl-6-oxo-4-(1,2,4,5-tetrahydro-benzo[d]azepin-3-yl)-1,6-dihydro-pyrimidine-5-carbonitrile as colorless solid; m.p. >200 °C; MS: [M]⁺ = 281.

### Example 18

### 2-Cyclopropyl-6-oxo-4-(1,2,4,5-tetrahydro-benzo[d]azepin-3-yl)-1,6-dihydro-pyrimidine-5-carbonitrile

In analogy to the procedure described in example 15 the Z and/or E 2-cyano-3-methylsulfanyl-3-(1,2,4,5-tetrahydro-benzo[d]azepin-3-yl)-acrylic acid ethyl ester [example 13 a)] was treated with cyclopropylcarbamidine hydrochloride in ethanol in the presence of sodium ethylate at reflux to yield the 2-cyclopropyl-6-oxo-4-(1,2,4,5-tetrahydro-benzo[d]azepin-3-yl)-1,6-dihydro-pyrimidine-5-carbonitrile as colorless solid; m.p. >200 °C; MS: [M]⁺ = 307.

### Example 19

### 2-[5-Cyano-6-oxo-4-(1,2,4,5-tetrahydro-benzo[d]azepin-3-yl)-1,6-dihydro-pyrimidin-2-yl]-acetamide

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with malonamamidine hydrochloride and 1,8-diazabicyclo[5.4.0]undec-7-en in N,N-dimethylformamide at 100 °C to yield the 2-[5-cyano-6-oxo-4-(1,2,4,5-tetrahydro-benzo[d]azepin-3-yl)-1,6-dihydro-pyrimidin-2-yl]-acetamide as light yellow solid; m.p. >200 °C; MS: [M]⁺ = 324.

### Example 20

### 6-Oxo-2-phenylsulfanylmethyl-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-(phenylthio)acetamidine hydrochloride and 1,8-diazabicyclo[5.4.0]undec-7-en in N,N-dimethylformamide at 100 °C to yield the 6-oxo-2-phenylsulfanylmethyl-4-(1,2,4,5-tetrahydro-benzo[d]azepin-3-yl)-1,6-dihydro-pyrimidine-5-carbonitrile as colorless solid; m.p. 189-194 °C; MS: [M+ H]⁺ = 389.

### Example 21

### 2-Dimethylamino-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 1,1-dimethyl-guanidine-sulfate and 1,8-diazabicyclo[5.4.0]undec-7-en in N,N-dimethylformamide at 100 °C to yield the 2-dimethylamino-6-oxo-4-(1,2,4,5-tetrahydro-benzo[d] azepin-3-yl)-1,6-dihydro-pyrimidine-5-carbonitrile as colorless solid; m.p. >200 °C; MS: [M+ H]⁺ = 310.

### Example 22

### 2-[2-(1,3-Dioxo-1,3-dihydro-isoindol-2-yl)-ethyl]-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 3-(1,3-dioxo-1,3-dihydro-isoindol-2-yl)-propionamidine hydrochloride [PCT Int. Appl. WO 9503305 A1 950202; CA 123:256545] and 1,8-diazabicyclo[5.4.0]undec-7-en in N,N-dimethylformamide at 100 °C to yield the 2-[2-(1,3-dioxo-1,3-dihydro-isoindol-2-yl)-ethyl]-6-oxo-4-(1,2,4,5-tetrahydro-benzo[d]azepin-3-yl)-1,5-dihydro-pyrimidine-5-carbonitrile as colorless solid; m.p. >200 °C; MS: [M+ H]⁺ = 440.

### Example 23

### 6-Oxo-2-propyl-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with butyramidine hydrochloride and 1,8-diazabicyclo[5.4.0]undec-7-en in N,N-dimethylformamide at 100 °C to yield 6-oxo-2-propyl-4-(1,2,4,5-tetrahydro-benzo[d]azepin-3-yl)-1,6-dihydro-pyrimidine-5-carbonitrile as colorless solid; m.p. 191-193 °C; MS: [M+ H]⁺ = 309.

### Example 24

### 2-(2-Hydroxy-ethyl)-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 3-hydroxy-propionamidine hydrochloride (1:1) [Tetrahedron Lett. (1990), 31(14), 1969-72] and 1,8-diazabicyclo[5.4.0]undec-7-en in N,N-dimethylformamide at 100 °C to yield the 2-(2-hydroxy-ethyl)-6-oxo-4-(1,2,4,5-tetrahydro-benzo[d]azepin-3-yl)-1,6-dihydro-pyrimidine-5-carbonitrile as light yellow solid; m.p. 183.5-188 °C; MS: [M+ H]⁺ = 311.

### Example 25

### 2-Ethylamino-6-oxo-4-(1,2,4,5-tetrahydro-benzo[d]azepin-3-yl)-1,6-dihydro-pyrimidine-5-carbonitrile

In analogy to the procedure described in example 15 the Z and/or E 2-cyano-3-methylsulfanyl-3-(1,2,4,5-tetrahydro-benzo[d]azepin-3-yl)-acrylic acid ethyl ester [example 13 a)] and 1-ethylguanidine sulfate dissolved in ethanol where heated at reflux in the presence of sodium ethylate to yield the 2-ethylamino-6-oxo-4-(1,2,4,5-tetrahydro-benzo[d]azepin-3-yl)-1,6-dihydro-pyrimidine-5-carbonitrile as colorless amorphous solid; MS: [M]⁺ = 310.

### Example 26

### 1.2-Dimethyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with methyl iodide in N,N-dimethylformamide in the presence of potassium carbonate to yield the 1,2-dimethyl-6-oxo-4-(1,2,4,5-tetrahydro-benzo[d]azepin-3-yl)-1,6-dihydro-pyrimidine-5-carbonitrile as pink solid; m.p. 155-158 °C; MS: [M]⁺ = 295.

### Example 27

### 1-Ethyl-2-methyl-6-oxo-4-(1,2,4,5-tetrahydro-benzo[d]azepin-3-yl)-1,6-dihydro-pyrimidine-5-carbonitrile

From 2-methyl-6-oxo-4-(1,2,4,5-tetrahydro-benzo[d]azepin-3-yl)-1,6-dihydro-pyrimidine-5-carbonitrile, iodoethane, N,N-dimethylformamide, potassium carbonate as colorless solid; m.p. 154.5-158 °C; see example 28.

### Example 28

### 4-Ethoxy-2-methyl-6-(1,2,4,5-tetrahydro-benzo[d]azepin-3-yl)-pyrimidine-5-carbonitrile

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with iodoethane in N,N-dimethylformamide in the presence of potassium carbonate to yield the 4-ethoxy-2-methyl-6-(1,2,4,5-tetrahydro-benzo[d]azepin-3-yl)-pyrimidine-5-carbonitrile as colorless solid; m.p. 106-109 °C; MS: [M+H]⁺ = 309; and the 1-ethyl-2-methyl-6-oxo-4-(1,2,4,5-tetrahydro-benzo[d]azepin-3-yl)-1,6-dihydro-pyrimidine-5-carbonitrile as colorless solid; m.p. 154.5-158 °C.

### Example 29

### 2-Amino-4-ethoxy-6-(1,2,4,5-tetrahydro-benzo[d]azepin-3-yl)-pyrimidine-5-carbonitrile

From 2-amino-6-oxo-4-(1,2,4,5-tetrahydro-benzo[d]azepin-3-yl)-1,6-dihydro-pyrimidine-5-carbonitrile, potassium carbonate, iodoethane, N,N-dimethylformamide, colorless amorphous solid; MS: [M+H]⁺ = 310; see example 31.

### Example 30

### 2-Amino-1-ethyl-6-oxo-4-(1,2,4,5-tetrahydro-benzo[d]azepin-3-yl)-1,6-dihydro-pyrimidine-5-carbonitrile

From 2-amino -6-oxo-4-(1,2,4,5-tetrahydro-benzo[d]azepin-3-yl)-1,6-dihydro-pyrimidine-5-carbonitrile, potassium carbonate, iodoethane, N,N-dimethylformamide, colorless solid; m.p. 195-203 °C; MS: [M+H]⁺ = 310; see example 31.

### Example 31

### 1-Ethyl-2-ethylamino-6-oxo-4-(1,2,4,5-tetrahydro-benzo[d]azepin-3-yl)-1,6-dihydro-pyrimidine-5-carbonitrile

In analogy to the procedure described in example 3 the 2-amino-6-oxo-4-(1,2,4,5-tetrahydro-benzo[d]azepin-3-yl)-1,6-dihydro-pyrimidine-5-carbonitrile [example 13 b)] was treated with iodoethane in N,N-dimethylformamide in the presence of potassium carbonate to yield the 2-amino-4-ethoxy-6-(1,2,4,5-tetrahydro-benzo[d]azepin-3-yl)-pyrimidine-5-carbonitrile as colorless amorphous solid; MS: [M+H]⁺ = 310; the 2-amino-1-ethyl-6-oxo-4-(1,2,4,5-tetrahydro-benzo[d]azepin-3-yl)-1,6-dihydro-pyrimidine-5-carbonitrile as colorless solid; m.p. 195-203 °C; MS: [M+H]⁺ = 310; and the 1-ethyl-2-ethylamino-6-oxo-4-(1,2,4,5-tetrahydro-benzo[d]azepin-3-yl)-1,6-dihydro-pyrimidine-5-carbonitrile as colorless amorphous solid; MS: [M+H]⁺ = 338.

### Example 32

### 1-Cyclopropylmethyl-2-methyl-6-oxo-4-(1,2,4,5-tetrahydro-benzo[d]azepin-3-yl)-1,6-dihydro-pyrimidine-5-carbonitrile

From 2-methyl-6-oxo-4-(1,2,4,5-tetrahydro-benzo[d]azepin-3-yl)-1,6-dihydro-pyrimidine-5-carbonitrile, bromomethylcyclopropane, potassium carbonate, N,N-dimethylformamide as colorless solid; m. p. 157-161 °C ;MS: [M+H]⁺ = 335; see example 33.

### Example 33

### 4-Cyclopropylmethoxy-2-methyl-6-(1,2,4,5-tetrahydro-benzo[d]azepin-3-yl)-pyrimidine-5-carbonitrile

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with bromomethylcyclopropane in N,N-dimethylformamide in the presence of potassium carbonate to yield the 1-cyclopropylmethyl-2-methyl-6-oxo-4-(1,2,4,5-tetrahydro-benzo[d]azepin-3-yl)-1,6-dihydro-pyrimidine-5-carbonitrile as colorless solid; m. p. 157-161 °C ;MS: [M+H]⁺ = 335; and the 4-cyclopropylmethoxy-2-methyl-6-(1,2,4,5-tetrahydro-benzo[d]azepin-3-yl)-pyrimidine-5-carbonitrile as colorless solid; m.p. 119-122 °C; MS: [M+H]⁺ = 335.

### Example 34

### 1-Allyl-2-methyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with allylbromide in N,N-dimethylformamide in the presence of potassium carbonate to yield the 1-allyl-2-methyl-6-oxo-4-(1,2,4,5-tetrahydro-benzo[d]azepin-3-yl)-1,6-dihydro-pyrimidine-5-carbonitrile as colorless solid; m. p. 141-144 °C; MS: [M+H]⁺ = 321.

### Example 35

### 1-Cyanomethyl-2-methyl-6-oxo-4-(1,2,4,5-tetrahydro-benzo[d]azepin-3-yl)-1,6-dihydro-pyrimidine-5-carbonitrile

From 2-methyl-6-oxo-4-(1,2,4,5-tetrahydro-benzo[d]azepin-3-yl)-1,6-dihydro-pyrimidine-5-carbonitrile, bromoacetonitrile, potassium carbonate, N,N-dimethylformamide, as colorless solid; m. p. >200 °C; MS: [M+H]⁺ = 320; see example 36.

### Example 36

### 4-Cyanomethoxy-2-methyl-6-(1,2,4,5-tetrahydro-benzo[d]azepin-3-yl)-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with bromoacetonitrile in N,N-dimethylformamide in the presence of potassium carbonate to yield the 1-cyanomethyl-2-methyl-6-oxo-4-(1,2,4,5-tetrahydro-benzo[d]azepin-3-yl)-1,6-dihydro-pyrimidine-5-carbonitrile as colorless solid; m. p. >200 °C; MS: [M+H]⁺ = 320; and the 4-cyanomethoxy-2-methyl-6-(1,2,4,5-tetrahydro-benzo[d]azepin-3-yl)-pyrimidine-5-carbonitrile as colorless amorphous solid; MS: [M+H]⁺ = 320.

### Example 37

### 1-(2-Dimethylamino-ethyl)-2-methyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with 1-chloro-2-dimethylaminoethane hydrochloride in N,N-dimethylformamide in the presence of potassium carbonate to yield the 1-(2-dimethylamino-ethyl)-2-methyl-6-oxo-4-(1,2,4,5-tetrahydro-benzo[d]azepin-3-yl)-1,6-dihydro-pyrimidine-5-carbonitrile as colorless solid; m.p. 134-139 °C; MS: [M+H]⁺ = 352.

### Example 38

### 1-Ethyl-6-oxo-4-(1,2,4,5-tetrahydro-benzo[d]azepin-3-yl)-1,6-dihydro-pyrimidine-5-carbonitrile

From 6-oxo-4-(1,2,4,5-tetrahydro-benzo[d]azepin-3-yl)-1,6-dihydro-pyrimidine-5-carbonitrile, iodoethane, potassium carbonate, N,N-dimethylformamide, as yellowish solid; m. p. 138-140 °C; MS: [M+H]⁺ = 295; see example 39.

### Example 39

### 4-Ethoxy-6-(1,2,4,5-tetrahydro-benzo[d]azepin-3-yl)-pyrimidine-5-carbonitrile

In analogy to the procedure described in example 3 the 6-oxo-4-(1,2,4,5-tetrahydro-benzo[d]azepin-3-yl)-1,6-dihydro-pyrimidine-5-carbonitrile (example 15) was treated with iodoethane in N,N-dimethylformamide in the presence of potassium carbonate to yield the 1-ethyl-6-oxo-4-(1,2,4,5-tetrahydro-benzo[d]azepin-3-yl)-1,6-dihydro-pyrimidine-5-carbonitrile as yellowish solid; m. p. 138-140°C; MS: [M+H]⁺ = 295; and the 4-ethoxy-6-(1,2,4,5-tetrahydro-benzo[d]azepin-3-yl)-pyrimidine-5-carbonitrile as colorless amorphous solid; MS: [M+H]⁺ = 295.

### Example 40

### 1-Isopropyl-6-oxo-4-(1,2,4,5-tetrahydro-benzo[d]azepin-3-yl)-1,6-dihydro-pyrimidine-5-carbonitrile

In analogy to the procedure described in example 3 the 6-oxo-4-(1,2,4,5-tetrahydro-benzo[d]azepin-3-yl)-1,6-dihydro-pyrimidine-5-carbonitrile (example 15) was treated with 2-iodopropane in N,N-dimethylformamide in the presence of potassium carbonate to yield the 1-isopropyl-6-oxo-4-(1,2,4,5-tetrahydro-benzo[d]azepin-3-yl)-1,6-dihydro-pyrimidine-5-carbonitrile as colorless solid; m. p. 152-154 °C; MS: [M+H]⁺ = 309.

### Example 41

### 1-(2-Hydroxy-ethyl)-6-oxo-4-(1,2,4,5-tetrahydro-benzo[d]azepin-3-yl)-1,6 dihydro-pyrimidine-5-carbonitrile

In analogy to the procedure described in example 3 the 6-oxo-4-(1,2,4,5-tetrahydro-benzo[d]azepin-3-yl)-1,6-dihydro-pyrimidine-5-carbonitrile (example 15) was treated with 2-bromoethanol in N,N-dimethylformamide in the presence of potassium carbonate to yield the 1-(2-hydroxy-ethyl)-6-oxo-4-(1,2,4,5-tetrahydro-benzo[d]azepin-3-yl)-1,6dihydro-pyrimidine-5-carbonitrile as light rose solid; m. p. 167-171 °C; MS: [M+H]⁺ = 311.

### Example 42

### [5-Cyano-6-oxo-4-(1,2,4,5-tetrahydro-benzo[d]azepin-3-yl)-6H-pyrimidin-1-yl]-acetic acid methyl ester

In analogy to the procedure described in example 3 the 6-oxo-4-(1,2,4,5-tetrahydro-benzo[d]azepin-3-yl)-1,6-dihydro-pyrimidine-5-carbonitrile (example 15) was treated with methylbromoacetate in N,N-dimethylformamide in the presence of potassium carbonate to yield the [5-cyano-6-oxo-4-(1,2,4,5-tetrahydro-benzo[d]azepin-3-yl)-6H-pyrimidin-1-yl]-acetic acid methyl ester as light yellow solid; m. p. 156-160 °C; MS: [M+H]⁺ = 339.

### Example 43

### 1-Methyl-6-oxo-4-(1,2,4,5-tetrahydro-benzo[d]azepin-3-yl)-1,6-dihydro-pyrimidine-5-carbonitrile

In analogy to the procedure described in example 3 the 6-oxo-4-(1,2,4,5-tetrahydro-benzo[d]azepin-3-yl)-1,6-dihydro-pyrimidine-5-carbonitrile (example 15) was treated with methyliodide in N,N-dimethylformamide in the presence of potassium carbonate to yield the 1-methyl-6-oxo-4-(1,2,4,5-tetrahydro-benzo[d]azepin-3-yl)-1,6-dihydro-pyrimidine-5-carbonitrile as colorless solid; m. p. 197-203 °C; MS: [M+H]⁺ = 281.

### Example 44

### 2-Amino-5-nitro-6-(1,2,4,5-tetrahydro-benzo[d]azepin-3-yl)-3H-pyrimidin-4-one

In analogy to the procedure described in example 1 the 2-amino-6-chloro-5-nitro-pyrimidin-4-ol [J. Chem. Soc. 1964, 4769-4774] was treated with the 2,3,4,5-tetrahydro-1H-benzo[d]azepine hydrochloride [J. Heterocycl. Chem. (1971), 8(5), 779-83] in N,N-dimethylformamide/potassiumcarbonate at 140 °C to yield the 2-amino-5-nitro-6-(1,2,4,5-tetrahydro-benzo[d]azepin-3-yl)-3H-pyrimidin-4-one as a yellow solid; m.p. 275 °C (decomposition).

### Example 45

### N-[5-Nitro-6-oxo-4-(1,2,4,5-tetrahydro-benzo[d]azepin-3-yl)-1,6-dihydro-pyrimidin-2-yl]-acetamide

A solution of 0.30 g (1.0 mmol) of 2-amino-5-nitro-6-(1,2,4,5-tetrahydro-benzo[d]azepin-3-yl)-3H-pyrimidin-4-one (example 44) in 1.0 ml acetic acid anhydride and 1.0 ml pyridine was stirred for 2 h at 60 °C. The reaction mixture was the poured into 25 ml of ice-water and acidified with 1 N hydrogen chloride solution. The residue formed was filtered off and washed with water and ether. There was thus obtained 0.302 g (0.88 mmol), 88 %, of N-[5-nitro-6-oxo-4-(1,2,4,5-tetrahydro-benzo[d]azepin-3-yl)-1,6-dihydro-pyrimidin-2-yl]-acetamide as yellow solid; m.p. >200 °C; MS: [M]⁺ = 344.

### Example 46

### 3-(6-Methoxy-5-nitro-pyrimidin-4-yl)-2,3,4,5-tetrahydro-1H-benzo[d]azepine

a) A solution of 0.396 g (2.0 mmol) of 4,6-dichloro-5-nitro-pyrimidine and of 0.367 g (2.0 mmol) 2,3,4,5-tetrahydro-1H-benzo[d]azepine hydrochloride [J. Heterocycl. Chem. (1971), 8(5), 779-83] in 30 ml N,N-dimethylformamide was treated slowly at 0°C to 5°C with 0.70 ml (5.0 mmol) of triethylamine and the reaction mixture stirred at the same temperature for 2 hours. It was then poured into 50 ml of an ice/water mixture and extracted three times with 100 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 99:1 v/v mixture of dichloromethane and methanol as eluent to yield 0.54 g (1.78 mmol), 88.9 %, of 3-(6-chloro-5-nitro-pyrimidin-4-yl)-2,3,4,5-tetrahydro-1H-benzo[d]azepine as yellow solid; m.p. 165-171 °C; MS: [M+H]⁺ = 305.
(b) 0.305 g (1.0 mmol) of 3-(6-chloro-5-nitro-pyrimidin-4-yl)-2,3,4,5-tetrahydro-1H-benzo[d]azepine were suspended in 15 ml of methanol and treated with 0.93 ml (5 mmol) of a solution of sodium methylate in methanol (30%). The reaction mixture was then heated to 60 °C for 20 minutes and stirred at room temperature for 4 hours, then filtered. The crystals obtained were washed with hexane and dried in a high vacuum. There were thus obtained 0.260 g (0.866 mmol), 86.6%, of 3-(6-methoxy-5-nitro-pyrimidin-4-yl)-2,3,4,5-tetrahydro-1H-benzo[d]azepine as yellow solid; m.p. 147-150 °C; MS: [M+H]⁺ = 301.

### Example 47

### 5-Nitro-6-(1,2,4,5-tetrahydro-benzo[d]azepin-3-yl)-pyrimidin-4-ol

a) 0.183 g (1.3 mmol) 4-methoxy-benzylalcohol were dissolved in 10 ml of tetrahydrofuran, then 0.048 g (1.1 mmol) sodium hydride dispersion (55% in mineral oil) added followed by 0.305 g (1.0 mmol) of 3-(6-chloro-5-nitro-pyrimidin-4-yl)-2,3,4,5-tetrahydro-1H-benzo[d]azepine [example 46 (a)] dissolved in 5 ml of tetrahydrofuran. The reaction mixture was then stirred at room temperature for 2 hours, poured into 50 ml of an ice/water mixture and extracted three times with 50 ml of ethyl acetate. The combined organic phases were washed twice with 50 ml of water, dried over magnesium sulphate, evaporated under reduced pressure and dried in a high vacuum. The thus obtained crude product was purified by chromatography on silica gel using dichloromethane as eluent to yield 0.41 g (1.3 mmol), 100 %, of 3-[6-(4-methoxy-benzyloxy)-5-nitro-pyrimidin-4-yl]-2,3,4,5-tetrahydro-1H-benzo[d]azepine as yellow solid; m.p. 105-109 °C; MS: [M+H]⁺ = 407.
b) 0.10 g (0.25 mmol) of 3-[6-(4-methoxy-benzyloxy)-5-nitro-pyrimidin-4-yl]-2,3,4,5-tetrahydro-1H-benzo[d]azepine were dissolved in 5.0 ml of methanol and treated with 0.63 ml 1.5M hydrogen chloride solution in methanol and the reaction mixture was stirred at room temperature for 2 hours. The crystals formed were filtered off, washed with hexane and dried in a high vaccum to yield 0.069 g (0.241 mmol), 98 %, of 5-nitro-6-(1,2,4,5-tetrahydro-benzo[d]azepin-3-yl)-pyrimidin-4-ol as yellow solid; m.p. >200 °C; MS: [M-H]⁻ = 285.

### Example 48

### 5-Nitro-6-(1,2,4,5-tetrahydro-benzo[d]azepin-3-yl)-pyrimidin-4-ylamine

0.305 g (1.0 mmol) of 3-(6-chloro-5-nitro-pyrimidin-4-yl)-2,3,4,5-tetrahydro-1H-benzo[d]azepine [example 46 (a)] were dissolved in 15 ml of tetrahydrofuran and treated with 0.22 ml of a 25% aqueous ammonia solution and stirred at room temperature for 60 hours. The reaction mixture was then evaporated to dryness and the thus obtained crude product was purified by chromatography on silica gel using a 98:2 v/v mixture of dichloromethane and methanol as eluent to yield 0.219 g (0.768 mmol), 76.8 %, of 5-nitro-6-(1,2,4,5-tetrahydro-benzo[d]azepin-3-yl)-pyrimidin-4-ylamine as yellow solid; m.p. >200 °C; MS: [M+H]⁺ = 286.

### Example 49

### Methyl-[5-nitro-6-(1,2,4,5-tetrahydro-benzo[d]azepin-3-yl)-pyrimidin-4-yl]-amine

In analogy to example 48 the 3-(6-chloro-5-nitro-pyrimidin-4-yl)-2,3,4,5-tetrahydro-1H-benzo[d]azepine [example 46 (a)] was treated with methylamine solution (40% in H₂O) in tetrahydrofuran at room temperature to yield the methyl-[5-nitro-6-(1,2,4,5-tetrahydro-benzo[d]azepin-3-yl)-pyrimidin-4-yl]-amine as yellow solid; m.p. 144-146 °C; MS: [M+H]⁺ = 300.

### Example 50

### Cyclopropyl-[5-nitro-6-(1,2,4,5-tetrahydro-benzo[d]azepin-3-yl)-pyrimidin-4-yl]-amine

In analogy to example 48 the 3-(6-chloro-5-nitro-pyrimidin-4-yl)-2,3,4,5-tetrahydro-1H-benzo[d]azepine [example 46 (a)] was treated with cyclopropylamine in tetrahydrofuran at room temperature to yield the cyclopropyl-[5-nitro-6-(1,2,4,5-tetrahydro-benzo[d]azepin-3-yl)-pyrimidin-4-yl]-amine as light yellow solid; m.p. 135-138 °C; MS: [M+H]⁺ = 326.

### Example 51

### [5-Nitro-6-(1,2,4,5-tetrahydro-benzo[d]azepin-3-yl)-pyrimidin-4-ylsulfanyl]-acetic acid methyl ester

In analogy to example 48 the 3-(6-chloro-5-nitro-pyrimidin-4-yl)-2,3,4,5-tetrahydro-1H-benzo[d]azepine [example 46 (a)] was treated with methyl thioglycolate and triethylamine in methanol at reflux to yield the [5-nitro-6-(1,2,4,5-tetrahydro-benzo[d]azepin-3-yl)-pyrimidin-4-ylsulfanyl]-acetic acid methyl ester as yellow solid; m.p. 113-115 °C; MS: [M+H]⁺ = 375.

### Example 52

### 6-Methyl-2-(1,2,4,5-tetrahydro-benzo[d]azepin-3-yl)-nicotinonitrile

0.509 g (3.3 mmol) of 2-chloro-3-cyano-6-methylpyridin, 0.551 g (3.0 mmol) of 2,3,4,5-tetrahydro-1H-benzo[d]azepine hydrochloride [J. Heterocycl. Chem. (1971), 8(5), 779-83] and 0.92 g (6.6 mmol) of potassium carbonate were dissolved in 3.0 ml of acetonitrile and heated at reflux for 16 hours. The reaction mixture was then allowed to cool to room temperature, poured into 30 ml of an ice/water mixture and extracted three times with 50 ml of dichloromethane. The combined organic phases were washed twice with 50 ml of water, dried over magnesium sulphate evaporated under reduced pressure and dried in a high vacuum. The thus obtained crude product was purified by chromatography on silica gel using a 4:1 v/v mixture of hexane and ethyl acetate as eluent to yield 0.724 g (2.75 mmol), 91.6 %, of 6-methyl-2-(1,2,4,5-tetrahydro-benzo[d]azepin-3-yl)-nicotinonitrile as colorless solid; m.p. 78.5-80.7 °C; MS: [M]⁺ = 263.

### Example 53

### 2-(1,2,4,5-Tetrahydro-benzo[d]azepin-3-yl)-benzonitrile

In analogy to example 52 the trifluoro-methanesulfonic acid 2-cyano-phenyl ester [J. Org. Chem. (1992), 57(5), 1481-6] and the 2,3,4,5-tetrahydro-1H-benzo[d]azepine hydrochloride [J. Heterocycl. Chem. (1971), 8(5), 779-83] were treated with potassium carbonate in acetonitrile at reflux to yield the 2-(1,2,4,5-tetrahydro-benzo[d]azepin-3-yl)-benzonitrile as yellowish solid; m.p. 68-74 °C; MS: [M]⁺ = 248.

### Example 54

### 3-(3-Nitro-pyridin-2-yl)-2,3,4,5-tetrahydro-1H-benzo[d]azepine

In analogy to example 52 the 2-chlor-3-nitropyridine and the 2,3,4,5-tetrahydro-1H-benzo[d]azepine hydrochloride [J. Heterocycl. Chem. (1971), 8(5), 779-83] were treated with potassium carbonate in acetonitrile at reflux to yield the 3-(3-nitro-pyridin-2-yl)-2,3,4,5-tetrahydro-1H-benzo[d]azepine as yellow solid; m.p. 129-136 °C.

### Example 55

### 2-(1,2,4,5-Tetrahydro-benzo[d]azepin-3-yl)-nicotinonitrile

In analogy to example 52 the chloronicotinonitrile and the 2,3,4,5-tetrahydro-1H-benzo[d]azepine hydrochloride [J. Heterocycl. Chem. (1971), 8(5), 779-83] were treated with potassium carbonate in acetonitrile at reflux to yield the 2-(1,2,4,5-tetrahydro-benzo[d]azepin-3-yl)-nicotinonitrile as colorless solid; m.p. 110-112.5 °C; MS: [M]⁺ = 249.

### Example 56

### 3-(2-Nitro-phenyl)-2,3,4,5-tetrahydro-1H-benzo[d]azepine

In analogy to example 52 the trifluoro-methanesulfonic acid 2-nitro-phenyl ester [J. Org. Chem. (1992), 57(5), 1481-6] and the 2,3,4,5-tetrahydro-1H-benzo[d]azepine hydrochloride [J. Heterocycl. Chem. (1971), 8(5), 779-83] were treated with potassium carbonate in acetonitrile at reflux to yield the 3-(2-nitro-phenyl)-2,3,4,5-tetrahydro-1H-benzo[d]azepine as orange oil; MS: [M+H]⁺ = 269.

### Example 57

### 3-Methyl-2,4-dioxo-6-(1,2,4,5-tetrahydro-benzo[d]azepin-3-yl)-1,2,3,4-tetrahydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with O-methylisourea hemisulfate and 1,8-diazabicyclo[5.4.0]undec-7-en in N,N-dimethylformamide at 100 °C to yield the 3-methyl-2,4-dioxo-6-(1,2,4,5-tetrahydro-benzo[d]azepin-3-yl)-1,2,3,4-tetrahydro-pyrimidine-5-carbonitrile as light yellow solid; m.p. >200 °C; MS: [M+ H]⁺ = 297.

### Example 58

### 3-(3,5-Dinitro-pyridin-2-yl)-2,3,4,5-tetrahydro-1H-benzo[d]azepine

In analogy to the procedure described in example 4 the 2-chloro-3,5-dinitro-pyridine and the 2,3,4,5-tetrahydro-1H-benzo[d]azepine hydrochloride [J. Heterocycl. Chem. (1971), 8(5), 779-83] were treated in N,N-dimethylformamide in the presence of triethylamine at room temperature to yield the 3-(3,5-dinitro-pyridin-2-yl)-2,3,4,5-tetrahydro-1H-benzo[d]azepine light yellow solid; m.p. 137-140 °C; MS: [M+H]⁺ = 315.

### Example 59

### 1-Methoxymethyl-6-oxo-4-(1,2,4,5-tetrahydro-benzo[d]azepin-3-yl)-1,6-dihydro-pyrimidine-5-carbonitrile

From 6-oxo-4-(1,2,4,5-tetrahydro-benzo[d]azepin-3-yl)-1,6-dihydro-pyrimidine-5-carbonitrile, chloromethyl-methylether, sodium hydride, N,N-dimethylformamide, as colorless solid; m.p. 160-162.5 °C; MS: [M]⁺ = 310; see example 60.

### Example 60

### 4-Methoxy-6-(1,2,4,5-tetrahydro-benzo[d]azepin-3-yl)-pyrimidine-5-carbonitrile

0.085 g (0.320 mmol) of 6-oxo-4-(1,2,4,5-tetrahydro-benzo[d]azepin-3-yl)-1,6-dihydro-pyrimidine-5-carbonitrile (example 15) were dissolved in 0.8 ml ofN,N-dimethylformamide and treated with 0.023 g (0.5 mmol) of sodium hydride dispersion (55% in mineral oil). Then, 0.041 g (0.5 mmol) chloromethyl-methyl-ether were added at once and the reaction mixture stirred at room temperature for 16 hours, poured into 30 ml of an ice/water mixture, acidified with 1 N hydrogen chloride solution and extracted three times with 30 ml of ethyl acetate. The combined organic phases were washed twice with 30 ml of water, dried over magnesium sulphate, evaporated under reduced pressure and dried in a high vacuum. The thus obtained crude product was purified by chromatography on silica gel using a 9:1 v/v mixture of dichloromethane and ether as eluent to yield 0.043 g (0.138 mmol), 43 %, 1-methoxymethyl-6-oxo-4-(1,2,4,5-tetrahydro-benzo[d]azepin-3-yl)-1,6-dihydro-pyrimidine-5-carbonitrile as colorless solid; m.p. 160-162.5 °C; MS: [M]⁺ = 310; and 0.021 g (0.076 mmol), 24 %, 4-methoxy-6-(1,2,4,5-tetrahydro-benzo[d]azepin-3-yl)-pyrimidine-5-carbonitrile colorless solid; m.p. 119-124.5 °C; MS: [M]⁺ = 280.

### Example 61

### 6-Oxo-1-[3-(2-oxo-azepan-1-yl)-propyl]-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with formamidine hydrochloride and 1,8-diazabicyclo[5.4.0]undec-7-en in N,N-dimethylformamide at 100 °C to yield the 6-oxo-4-(1,2,4,5-tetrahydro-benzo[d]azepin-3-yl)-1,6-dihydro-pyrimidine-5-carbonitrile (example 15) and the 6-oxo-1-[3-(2-oxo-azepan-1-yl)-propyl]-4-(1,2,4,5-tetrahydro-benzo[d]azepin-3-yl)-1,6-dihydro-pyrimidine-5-carbonitrile as colorless solid; m.p. 139-140 °C; MS: [M+H]⁺ = 420.

### Example 62

### 6-(7,8-Dimethoxy-1,2,4,5-tetrahydro-benzo[d]azepin-3-yl)-2-methyl-5-nitro-3H-pyrimidin-4-one

In analogy to the procedure described in example 1 the 2-methyl-4-methoxy-5-nitro-6-chloro-pyrimidine [Helv. (1958), 41, 1806] was treated with the 7,8-dimethoxy-2,3,4,5-tetrahydro-1H-benzo[d]azepine hydrochloride [Ann. Chim. (Paris) (1966), 1(5/6), 221-54] in N,N-dimethylformamide/potassium carbonate at 120°C to yield the 6-(7,8-dimethoxy-1,2,4,5-tetrahydro-benzo[d]azepin-3-yl)-2-methyl-5-nitro-3H-pyrimidin-4-one as a yellow solid; m.p. 268-270 °C (decomposition); MS: [M+H]⁺ = 361.

### Example 63

### 2-Methyl-5-nitro-6-(5,6,8,9-tetrahydro-1,3-dioxa-7-aza-cyclohepta[f]inden-7-yl)-3H-pyrimidin-4-one

In analogy to the procedure described in example 1 the 2-methyl-4-methoxy-5-nitro-6-chloro-pyrimidine [Helv. (1958), 41, 1806] was treated with the 6,7,8,9-tetrahydro-5H-1,3-dioxa-7-aza-cyclohepta[f]indene hydrochloride [J. Heterocycl. Chem. (1972), 9(3), 617-21] in N,N-dimethylformamide/potassium carbonate at 120 °C to yield the 2-methyl-5-nitro-6-(5,6,8,9-tetrahydro-1,3-dioxa-7-aza-cyclohepta[f]inden-7-yl)-3H-pyrimidin-4-one as a light yellow solid; m.p. 258-262 °C (decomposition); MS: [M+H]⁺ = 345.

### Example 64

### 2-Methyl-5-nitro-6-(7-nitro-1,2,4,5-tetrahydro-benzo[d]azepin-3-yl)-3H-pyrimidin-4-one

In analogy to the procedure described in example 4 the 6-bromo-2-methyl-5-nitro-3H-pyrimidin-4-one (prepared from 2-methyl-4-methoxy-5-nitro-6-chloro-pyrimidine [Helv. (1958), 41, 1806] and hydrobromic acid (48% in water) in acetic acid at room temperature) was treated with the 7-nitro-2,3,4,5-tetrahydro-1H-benzo[d]azepine [J. Heterocycl. Chem. (1971), 8(5), 779-83] in N,N-dimethylformamide in the presence of N-ethyl-N,N-diisopropylamine at room temperature to yield the 2-methyl-5-nitro-6-(7-nitro-1,2,4,5-tetrahydro-benzo[d]azepin-3-yl)-3H-pyrimidin-4-one as yellow solid; m.p. 237-238 °C (decomposition); MS: [M+H]⁺ = 346.

### Example 65

### 3-(2-Methyl-5-nitro-6-oxo-1,6-dihydro-pyrimidin-4-yl)-2,3,4,5-tetrahydro-1H-benzo[d]azepine-7-sulfonic acid amide

In analogy to the procedure described in example 4 the 6-bromo-2-methyl-5-nitro-3H-pyrimidin-4-one (prepared from 2-methyl-4-methoxy-5-nitro-6-chloro-pyrimidine [Helv. (1958), 41, 1806] and hydrobromic acid (48% in water) in acetic acid at room temperature) was treated with the 2,3,4,5-tetrahydro-1H-benzo[d]azepine-7-sulfonic acid amide [Ger. Offen. DE 1921737] in N,N-dimethylformamide in the presence of N-ethyl-N,N-diisopropylamine at room temperature to yield the 3-(2-methyl-5-nitro-6-oxo-1,6-dihydro-pyrimidin-4-yl)-2,3,4,5-tetrahydro-1H-benzo[d]azepine-7-sulfonic acid amide as light yellow solid; m.p. 268-270 °C (decomposition); MS: [M+H]⁺ = 380.

### Example 66

### 6-(7-Amino-1,2,4,5-tetrahydro-benzo[d]azepin-3-yl)-2-methyl-5-nitro-3H-pyrimidin-4-one

In analogy to the procedure described in example 4 the 6-bromo-2-methyl-5-nitro-3H-pyrimidin-4-one (prepared from 2-methyl-4-methoxy-5-nitro-6-chloro-pyrimidine [Helv. (1958), 41, 1806] and hydrobromic acid (48% in water) in acetic acid at room temperature) was treated with the 2,3,4,5-tetrahydro-1H-benzo[d]azepin-7-ylamine [J. Heterocycl. Chem. (1971), 8(5), 779-83] in N,N-dimethylformamide in the presence of N-ethyl-N,N-diisopropylamine at room temperature to yield the 6-(7-amino-1,2,4,5-tetrahydro-benzo[d]azepin-3-yl)-2-methyl-5-nitro-3H-pyrimidin-4-one as yellow solid; m.p. 218-220 °C (decomposition); MS: [M+H]⁺ = 316.

### Example 67

### 6-Oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile

0.160 g (0.516 mmol) of 2-(2-hydroxy-ethyl)-6-oxo-4-(1,2,4,5-tetrahydro-benzo[d]azepin-3-yl)-1,6-dihydro-pyrimidine-5-carbonitrile (example 24), 0.084 g (1.0 mmol) 3,4-dihydro-2H-pyran and 0.130 g (0.516 mmol) of pyridinium-(toluene-4-sulfonate) were dissolved in 5.0 ml of dichloromethane and stirred for 18 hours at room temperature. Then, the reaction mixture was poured into 30 ml of an ice/dil. aq. sodium bicarbonate mixture and extracted three times with 20 ml of dichloromethane. The combined organic phases were washed twice with 1 N HCl solution, twice with dil. aq. sodium bicarbonate solution, dried over magnesium sulphate and evaporated under reduced pressure. There were thus obtained 0.176 g (0.446 mmol), 87 %, 6-oxo-4-(1,2,4,5-tetrahydro-benzo [d]azepin-3-yl)-2-[2-(tetrahydro-pyran-2-yloxy)-ethyl]-1,6-dihydro-pyrimidine-5-carbonitrile as colorless solid; m.p. 185-187°C.

### Example 68

### 4-Ethoxy-6-(1,2,4,5-tetrahydro-benzo[d]azepin-3-yl)-2-[2-(tetrahydro-pyran-2-yloxy)-ethyl]-pyrimidine-5-carbonitrile

From 6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile, ethyliodide, potassium carbonate, N,N-dimethylformamide as colorless amorphous solid; MS: [M+H]⁺ = 423; see example 69.

### Example 69

### 1-Ethyl-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile

In analogy to the procedure described in example 3 the 6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile (example 67) was treated with ethyliodide in N,N-dimethylformamide in the presence of potassium carbonate to yield the 1-ethyl-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile as colorless amorphous solid; MS: [M+H]⁺ = 423; and the 4-ethoxy-6-(1,2,4,5-tetrahydro-benzo[d]azepin-3-yl)-2-[2-(tetrahydro-pyran-2-yloxy)-ethyl]-pyrimidine-5-carbonitrile as colorless amorphous solid; MS: [M+H]⁺ = 423.

### Example 70

### 1-Ethyl-2-(2-hydroxy-ethyl)-6-oxo-4-(1,2,4,5-tetrahydro-benzo[d]azepin-3-yl)-1,6-dihydro-pyrimidine-5-carbonitrile

0.144g (0.341 mmol) of 1-ethyl-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile (example 69) were dissolved in 2.5 ml of methanol and treated with 0.45 ml of an 1.5 M solution of HCl in methanol. After stirring at room temperature for 30 minutes, 250 mg solid powdered sodium bicarbonate was added and the reaction mixture evaporated to dryness. The thus obtained crude product was purified by chromatography on silica gel using a 95:5 v/v mixture of dichloromethane and methanol as eluent to yield 0.115 g (0.341 mmol), 100 %, of 1-ethyl-2-(2-hydroxy-ethyl)-6-oxo-4-(1,2,4,5-tetrahydro-benzo[d]azepin-3-yl)-1,6-dihydro-pyrimidine-5-carbonitrile as colorless solid; MS: [M+H]⁺ = 339; m.p. 114-115 °C.

### Example 71

### 4-Ethoxy-2-(2-hydroxy-ethyl)-6-(1,2,4,5-tetrahydro-benzo[d]azepin-3-yl)-pyrimidine-5-carbonitrile

In analogy to the procedure described in example 70 the 4-ethoxy-6-(1,2,4,5-tetrahydro-benzo[d]azepin-3-yl)-2-[2-(tetrahydro-pyran-2-yloxy)-ethyl]-pyrimidine-5-carbonitrile (example 68) was treated with 1.5 N HCl in methanol to yield the 4-ethoxy-2-(2-hydroxy-ethyl)-6-( 1,2,4,5-tetrahydro-benzo[d] azepin-3-yl)-pyrimidine-5-carbonitrile as colorless solid; MS: [M+H]⁺= 339; m.p. 108-111 °C.

### Example 72

### 4-(2-Hydroxy-ethoxy)-6-(1,2,4,5-tetrahydro-benzo[d]azepin-3-yl)-2-[2-(tetrahydro-pyran-2-yloxy)-ethyl]-pyrimidine-5-carbonitrile

From 6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile, 2-bromoethanol, potassium carbonate, N,N-dimethylformamide, as colorless foam; MS: [M+H]⁺ = 439; see example 73.

### Example 73

### 1-(2-Hydroxy-ethyl)-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile

In analogy to the procedure described in example 3 the 6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile (example 67) was treated with 2-bromo-ethanol in N,N-dimethylformamide in the presence of potassium carbonate to yield the 4-(2-hydroxy-ethoxy)-6-(1,2,4,5-tetrahydro-benzo [d]azepin-3-yl)-2-[2-(tetrahydro-pyran-2-yloxy)-ethyl]-pyrimidine-5-carbonitrile as colorless foam; MS: [M+H]⁺ = 439; and the 1-(2-hydroxy-ethyl)-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile as yellow foam; MS: [M+H]⁺ = 439.

### Example 74

### 1,2-Bis-(2-hydroxy-ethyl)-6-oxo-4-(1,2,4,5-tetrahydro-benzo[d]azepin-3-yl)-1,6-dihydro-pyrimidine-5-carbonitrile

In analogy to the procedure described in example 70 the 1-(2-hydroxy-ethyl)-6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile (example 73) was treated with HCl in methanol to yield the 1,2-bis-(2-hydroxy-ethyl)-6-oxo-4-(1,2,4,5-tetrahydro-benzo[d]azepin-3-yl)-1,6-dihydro-pyrimidine-5-carbonitrile as colorless solid; MS: [M+H]⁺ = 355; m.p. 164 °C.

### Example 75

### 4-(2-Hydroxy-ethoxy)-2-(2-hydroxy-ethyl)-6-(1,2,4,5-tetrahydro-benzo[d]azepin-3-yl)-pyrimidine-5-carbonitrile

In analogy to the procedure described in example 70 the 4-(2-hydroxy-ethoxy)-6-(1,2,4,5-tetrahydro-benzo[d]azepin-3-yl)-2-[2-(tetrahydro-pyran-2-yloxy)-ethyl]-pyrimidine-5-carbonitrile (example 72) was treated with HCl in methanol to yield the 4-(2-hydroxy-ethoxy)-2-(2-hydroxy-ethyl)-6-(1,2,4,5-tetrahydro-benzo[d]azepin-3-yl)-pyrimidine-5-carbonitrile as colorless solid; MS: [M+H]⁺ = 355; m.p. 118-120 °C.

### Example 76

### 4-(1,2,4,5-Tetrahydro-benzo[d]azepin-3-yl)-pyrimidine-5-carbonitrile

In analogy to the procedure described in example 4 the 4-chloro-pyrimidine-5-carbonitrile (prepared from 4-hydroxy-5-pyrimidine-carbonitrile and phosphorus oxychloride, phosphorus pentachloride and N-ethyl-N,N-diisopropylamine in acetonitril at reflux) was treated with 2,3,4,5-tetrahydro-1H-benzo[d]azepine hydrochloride [J. Heterocycl. Chem. ( 1971), 8(5), 779-83] in N,N-dimethylformamide in the presence of N-ethyl-N,N-diisopropylamine at room temperature to yield the 4-(1,2,4,5-tetrahydro-benzo[d]azepin-3-yl)-pyrimidine-5-carbonitrile as off white solid; MS: [M+H]⁺ = 251; m.p. 148-150 °C.

### Example 77

### 6-(7-Chloro-1,2,4,5-tetrahydro-benzo[d]azepin-3-yl)-2-methyl-5-nitro-3H-pyrimidin-4-one

In analogy to the procedure described in example 4 the 6-bromo-2-methyl-5-nitro-3H-pyrimidin-4-one (prepared from 2-methyl-4-methoxy-5-nitro-6-chloro-pyrimidine [Helv. (1958), 41, 1806] and hydrobromic acid (48% in water) in acetic acid at room temperature) was treated with the 7-chloro-2,3,4,5-tetrahydro-1H-benzo[d]azepine [J. Heterocycl. Chem. (1971), 8(5), 779-83] in N,N-dimethylformamide in the presence of N-ethyl-N,N-diisopropylamine at room temperature to yield the 6-(7-chloro-1,2,4,5-tetrahydro-benzo[d]azepin-3-yl)-2-methyl-5-nitro-3H-pyrimidin-4-one as yellow solid; m.p. 218 °C (decomposition); MS: [M+H]⁺ = 335.

### Example 78

### 2-Methyl-6-oxo-4-(1,2,4,5-tetrahydro-benzo[d]azepin-3-yl)-1-(2,2,2-trifluoro-ethyl)-1,6-dihydro-pyrimidine-5-carbonitrile

From 2-methyl-6-oxo-4-(1,2,4,5-tetrahydro-benzo[d]azepin-3-yl)-1,6-dihydro-pyrimidine-5-carbonitrile, 2,2,2-trifluoroethyl iodide, potassium carbonate, N,N-dimethylformamide, as yellowish solid; m.p. 186-188 °C; MS: [M+H]⁺ = 363; see example 79.

### Example 79

### 2-Methyl-4-(1,2,4,5-tetrahydro-benzo[d]azepin-3-yl)-6-(2,2,2-trifluoro-ethoxy)-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with 2,2,2-trifluoroethyl iodide in N,N-dimethylformamide in the presence of potassium carbonate at 80 °C to yield the 2-methyl-6-oxo-4-(1,2,4,5-tetrahydro-benzo[d]azepin-3-yl)-1-(2,2,2-trifluoro-ethyl)-1,6-dihydro-pyrimidine-5-carbonitrile as yellowish solid; m.p. 186-188 °C; MS: [M+H]⁺ = 363; and the 2-methyl-4-(1,2,4,5-tetrahydro-benzo[d]azepin-3-yl)-6-(2,2,2-trifluoro-ethoxy)-pyrimidine-5-carbonitrile as colorless solid; m.p. 108-110 °C; MS: [M+H]⁺ = 363.

### Example 80

### 2-(2-Methylsulfanyl-ethyl)-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 3-hydroxy-propionamidine hydrochloride (1:1) [Tetrahedron Lett. (1990), 31(14), 1969-72] and 1,8-diazabicyclo[5.4.0]undec-7-en in N,N-dimethylformamide at 100 °C to yield beside 2-(2-hydroxy-ethyl)-6-oxo-4-(1,2,4,5-tetrahydro-benzo[d]azepin-3-yl)-1,6-dihydro-pyrimidine-5-carbonitrile (example 24) the 2-(2-methylsulfanyl-ethyl)-6-oxo-4-(1,2,4,5-tetrahydro-benzo[d]azepin-3-yl)-1,6-dihydro-pyrimidine-5-carbonitrile as colorless solid; m.p. 215-218 °C (decomposition); MS: [M+H]⁺ = 341.

### Example 81

### 1-Ethyl-2-(2-methylsulfanyl-ethyl)-6-oxo-4-(1,2,4,5-tetrahydro-benzo[d]azepin-3-yl)-1,6-dihydro-pyrimidine-5-carbonitrile

From 2-(2-methylsulfanyl-ethyl)-6-oxo-4-(1,2,4,5-tetrahydro-benzo[d]azepin-3-yl)-1,6-dihydro-pyrimidine-5-carbonitrile, ethyliodide, potassium carbonate, N,N-dimethylformamide, as colorless solid; m.p.154-159 °C; MS: [M+H]⁺ = 369; see example 82.

### Example 82

### 4-Ethoxy-2-(2-methylsulfanyl-ethyl)-6-(1,2,4,5-tetrahydro-benzo[d]azepin-3-yl)-pyrimidine-5-carbonitrile

In analogy to the procedure described in example 3 the 2-(2-methylsulfanyl-ethyl)-6-oxo-4-(1,2,4,5-tetrahydro-benzo[d]azepin-3-yl)-1,6-dihydro-pyrimidine-5-carbonitrile (example 80) was treated with ethyliodide in N,N-dimethylformamide in the presence of potassium carbonate to yield the 1-ethyl-2-(2-methylsulfanyl-ethyl)-6-oxo-4-(1,2,4,5-tetrahydro-benzo[d]azepin-3-yl)-1,6-dihydro-pyrimidine-5-carbonitrile as colorless solid; m.p. 154-159 °C; MS: [M+H]⁺ = 369; and the 4-ethoxy-2-(2-methylsulfanyl-ethyl)-6-(1,2,4,5-tetrahydro-benzo[d]azepin-3-yl)-pyrimidine-5-carbonitrile as colorless solid; m.p. 102-104 °C; MS: [M+H]⁺ = 369.

### Example 83

### 1-(2-Hydroxy-ethyl)-2-(2-methylsulfanyl-ethyl)-6-oxo-4-(1,2,4,5-tetrahydro-benzo[d]azepin-3-yl)-1,6-dihydro-pyrimidine-5-carbonitrile

From 2-(2-methylsulfanyl-ethyl)-6-oxo-4-(1,2,4,5-tetrahydro-benzo[d]azepin-3-yl)-1,6-dihydro-pyrimidine-5-carbonitrile, potassium carbonate, N,N-dimethylformamide, bromoethanol, as yellowish amorphous solid; MS: [M+H]⁺ = 385; see example 84.

### Example 84

### 4-(2-Hydroxy-ethoxy)-2-(2-methylsulfanyl-ethyl)-6-(1,2,4,5-tetrahydro-benzo[d]azepin-3-yl)-pyrimidine-5-carbonitrile

In analogy to the procedure described in example 3 the 2-(2-methylsulfanyl-ethyl)-6-oxo-4-(1,2,4,5-tetrahydro-benzo[d]azepin-3-yl)-1,6-dihydro-pyrimidine-5-carbonitrile (example 80) was treated with 2-bromo-ethanol in N,N-dimethylformamide in the presence of potassium carbonate to yield the 1-(2-hydroxy-ethyl)-2-(2-methylsulfanyl-ethyl)-6-oxo-4-(1,2,4,5-tetrahydro-benzo[d] azepin-3-yl)-1,6-dihydro-pyrimidine-5-carbonitrile as yellowish amorphous solid; MS: [M+H]⁺ = 385; and the 4-(2-hydroxy-ethoxy)-2-(2-methylsulfanyl-ethyl)-6-(1,2,4,5-tetrahydro-benzo[d]azepin-3-yl)-pyrimidine-5-carbonitrile as yellowish solid; m.p. 93-99 °C; MS: [M+H]⁺ = 385.

### Example 85

### 3-(2-Hydroxy-ethyl)-2-methyl-5-nitro-6-(1,2,4,5-tetrahydro-benzo[d]azepin-3-yl)-3H-pyrimidin-4-one

From 2-methyl-5-nitro-6-(1,2,4,5-tetrahydro-benzo[d]azepin-3-yl)-pyrimidin-4-ol and 2-bromoethanol, potassium carbonate in N,N- dimethylformamide, as yellow solid; m.p. 151-155 °C; MS: [M+H]⁺ = 345; see example 86.

### Example 86

### 2-[2-Methyl-5-nitro-6-(1,2,4,5-tetrahydro-benzo[d]azepin-3-yl)-pyrimidin-4-yloxy]-ethanol

In analogy to the procedure described in example 3 the 2-methyl-5-nitro-6-(1,2,4,5-tetrahydro-benzo[d]azepin-3-yl)-pyrimidin-4-ol (example 1) was treated with 2-bromo-ethanol in N,N-dimethylformamide in the presence of potassium carbonate to yield the 3-(2-hydroxy-ethyl)-2-methyl-5-nitro-6-(1,2,4,5-tetrahydro-benzo[d]azepin-3-yl)-3H-pyrimidin-4-one as yellow solid; m.p. 151-155 °C; MS: [M+H]⁺ = 345; and 2-[2-methyl-5-nitro-6-(1,2,4,5-tetrahydro-benzo[d]azepin-3-yl)-pyrimidin-4-yloxy]-ethanol as light yellow solid; m.p. 113 °C (decomposition); MS: [M+H]⁺ = 345.

### Example 87

### 3-(5-Methyl-3-nitro-pyridin-2-yl)-2,3,4,5-tetrahydro-1H-benzo[d]azepine

In analogy to the procedure described in example 4 the 2-chloro-5-methyl-3-nitro-pyridine [J. Organomet. Chem. (1996), 517(1-2), 25-36] was treated with 2,3,4,5-tetrahydro-1H-benzo[d]azepine hydrochloride [J. Heterocycl. Chem. (1971), 8(5), 779-83] in N,N-dimethylformamide in the presence of N-ethyl-N,N-diisopropylamine at room temperature to yield the 3-(5-methyl-3-nitro-pyridin-2-yl)-2,3,4,5-tetrahydro-1H-benzo[d]azepine as yellow solid; MS: [M+H]⁺ = 284; m.p. 87-88 °C.

### Example 88

### 2-(2-Methylsulfanyl-ethyl)-4-(1,2,4,5-tetrahydro-benzo[d]azepin-3-yl)-6-(2,2,2-trifluoro-ethoxy)-pyrimidine-5-carbonitrile

In analogy to the procedure described in example 3 the 2-(2-methylsulfanyl-ethyl)-6-oxo-4-(1,2,4,5-tetrahydro-benzo[d]azepin-3-yl)-1,6-dihydro-pyrimidine-5-carbonitrile (example 80) was treated with 2,2,2-trifluoroethyl trifluoromethanesulfonate in N,N-dimethylformamide in the presence of potassium carbonate to yield the 2-(2-methylsulfanyl-ethyl)-4-(1,2,4,5-tetrahydro-benzo[d]azepin-3-yl)-6-(2,2,2-trifluoro-ethoxy)-pyrimidine-5-carbonitrile as colorless solid; m.p. 97-100 °C; MS: [M+H]⁺ = 423.

### Example 89

### 2-Methylsulfanyl-6-oxo-4-(1,2,4,5-tetrahydro-benzo[d]azepin-3-yl)-1,6-dihydro-pyrimidine-5-carbonitrile

a) 0.475 g (1.50 mmol) of E and/or Z 2-cyano-3-methylsulfanyl-3-(1,2,4,5-tetrahydro-benzo[d]azepin-3-yl)-acrylic acid ethyl ester [example 13 a)] were dissolved in 8.0 ml of dichloromethane and treated with 0.712 g (3.30 mmol) of m-chloroperbenzoic acid. After stirring at room temperature for 18 hours, the reaction mixture was poured into 30 ml of an ice/dil. aq. sodium carbonate mixture and extracted three times with 20 ml of dichloromethane. The combined organic phases were dried over magnesium sulphate and evaporated under reduced pressure. The thus obtained crude product was purified by chromatography on silica gel using a 1:1 v/v mixture of ethylacetate and hexane as eluent to yield after crystallization from ether 0.115 g (0.330 mmol), 22 %, of E and/or Z 2-cyano-3-methanesulfonyl-3-(1,2,4,5-tetrahydro-benzo[d]azepin-3-yl)-acrylic acid ethyl ester as yellowish solid; MS: [M+H]⁺ = 349; m.p. 80 °C.
b) 0.100 g (0.287 mmol) of E and/or Z 2-cyano-3-methanesulfonyl-3-(1,2,4,5-tetrahydro-benzo[d]azepin-3-yl)-acrylic acid ethyl ester, 0.082 mg (0.287 mmol) S-methylisothioureasulfate and 0.107 mg (1.03 mmol) triethylamine were dissolved in 2.0 ml of ethanol and heated under reflux for 6 hours. After cooling to room temperature the reaction mixture was evaporated to dryness, poured into 1 N HCl solution and extracted three times with 20 ml of dichloromethane. The combined organic phases were dried over magnesium sulphate and evaporated under reduced pressure. The thus obtained crude product was purified by chromatography on silica gel using a 95:5 v/v mixture of dichloromethane and methanol as eluent to yield after crystallization from ethyl acetate 0.012 g (0.039 mmol), 14%, of 2-methylsulfanyl-6-oxo-4-(1,2,4,5-tetrahydro-benzo[d]azepin-3-yl)-1,6-dihydro-pyrimidine-5-carbonitrile as colorless solid; MS: [M+H]⁺ = 313; m.p. > 250 °C.

### Example 90

### 6-(7-Methoxy-1,2,4,5-tetrahydro-benzo[d]azepin-3-yl)-2-methyl-5-nitro-3H-pyrimidin-4-one

In analogy to the procedure described in example 4 the 6-bromo-2-methyl-5-nitro-3H-pyrimidin-4-one (prepared from 2-methyl-4-methoxy-5-nitro-6-chloro-pyrimidine [Helv. (1958), 41, 1806] and hydrobromic acid (48% in water) in acetic acid at room temperature) was treated with the 7-methoxy-2,3,4,5-tetrahydro-1H-benzo[d]azepine [J. Heterocycl. Chem. (1971), 8(5), 779-83] in N,N-dimethylformamide in the presence of N-ethyl-N,N-diisopropylamine at room temperature to yield the 6-(7-methoxy-1,2,4,5-tetrahydro-benzo[d]azepin-3-yl)-2-methyl-5-nitro-3H-pyrimidin-4-one as yellow solid; m.p. 243 °C (decomposition); MS: [M+H]⁺ = 331.

### Example 91

### Dimethyl-{2-[2-methyl-5-nitro-6-(1,2,4,5-tetrahydro-benzo[d]azepin-3-yl)-pyrimidin-4-yloxy]-ethyl}-amine

From 2-methyl-5-nitro-6-( 1,2,4,5-tetrahydro-benzo[ d] azepin-3-yl)-pyrimidin-4-ol with 1-chloro-2-dimethylamino-ethane hydrochloride, potassium carbonate, N,N-dimethylformamide, as yellow oil; MS: [M+H]⁺ = 372; see example 92.

### Example 92

### 3-(2-Dimethylamino-ethyl)-2-methyl-5-nitro-6-(1,2,4,5-tetrahydro-benzo[d]azepin-3-yl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol (example 1) was treated with 1-chloro-2-dimethylamino-ethane hydrochloride in N,N-dimethylformamide in the presence of potassium carbonate at 50 °C to yield the dimethyl-{2-[2-methyl-5-nitro-6-(1,2,4,5-tetrahydro-benzo[d]azepin-3-yl)-pyrimidin-4-yloxy]-ethyl}-amine as yellow oil; MS: [M+H]⁺ = 372; and the 3-(2-dimethylamino-ethyl)-2-methyl-5-nitro-6-(1,2,4,5-tetrahydro-benzo[d]azepin-3-yl)-3H-pyrimidin-4-one as yellow amorphous solid; MS: [M+H]⁺ = 372.

### Example 93

### 3-[2-Methyl-6-(2-morpholin-4-yl-ethoxy)-5-nitro-pyrimidin-4-yl]-2,3,4,5-tetrahydro-1H-benzo[d]azepine

From 2-methyl-5-nitro-6-(1,2,4,5-tetrahydro-benzo[d]azepin-3-yl)-pyrimidin-4-ol with 4-(2-chloroethyl)-morpholine hydrochloride, potassium carbonate, N,N-dimethylformamide, as yellow oil; MS: [M+H]⁺ = 414; see example 94.

### Example 94

### 2-Methyl-3-(2-morpholin-4-yl-ethyl)-5-nitro-6-(1,2,4,5-tetrahydro-benzo[d]azepin-3-yl)- 3H-pyrimidin-4-one

In analogy to the procedure described in example 3 the 2-methyl-5-nitro-6-(1,2,4,5-tetrahydro-benzo[d]azepin-3-yl)-pyrimidin-4-ol (example 1) was treated with 4-(2-chloroethyl)-morpholine hydrochloride in N,N-dimethylformamide in the presence of potassium carbonate at 50 °C to yield the 3-[2-methyl-6-(2-morpholin-4-yl-ethoxy)-5-nitro-pyrimidin-4-yl]-2,3,4,5-tetrahydro-1H-benzo[d]azepine as yellow oil; MS: [M+H]⁺ = 414; and the 2-methyl-3-(2-morpholin-4-yl-ethyl)-5-nitro-6-(1,2,4,5-tetrahydro-benzo[d]azepin-3-yl)-3H-pyrimidin-4-one as yellowsolid; m.p. 143-145 °C; MS: [M+H]⁺ = 414.

### Example 95

### 4-(1,2,4,5-Tetrahydro-benzo[d]azepin-3-yl)-2-[2-(tetrahydro-pyran-2-yloxy)-ethyl]-6-(2,2,2-trifluoro-ethoxy)-pyrimidine-5-carbonitrile

In analogy to the procedure described in example 3 the 6-oxo-4-(1,2,4,5-tetrahydro-benzo[d]azepin-3-yl)-2-[2-(tetrahydro-pyran-2-yloxy)-ethyl]-1,6-dihydro-pyrimidine-5-carbonitrile (example 67) was treated with 2,2,2-trifluoroethyl trifluoromethanesulfonate in N,N-dimethylformamide in the presence of potassium carbonate to yield the 4-(1,2,4,5-tetrahydro-benzo [d] azepin-3-yl)-2-[2-(tetrahydro-pyran-2-yloxy)-ethyl]-6-(2,2,2-trifluoro-ethoxy)-pyrimidine-5-carbonitrile as yellow oil; MS: [M+H]⁺ = 477.

### Example 96

### 2-(2-Hydroxy-ethyl)-4-(1,2,4,5-tetrahydro-benzo[d]azepin-3-yl)-6-(2,2,2-trifluoro-ethoxy)-pyrimidine-5-carbonitrile

In analogy to the procedure described in example 70 the 4-(1,2,4,5-tetrahydro-benzo [d]azepin-3-yl)-2-[2-(tetrahydro-pyran-2-yloxy)-ethyl]-6-(2,2,2-trifluoro-ethoxy)-pyrimidine-5-carbonitrile (example 95) was treated with 1.5 N HCl in methanol to yield the 2-(2-hydroxy-ethyl)-4-(1,2,4,5-tetrahydro-benzo[d]azepin-3-yl)-6-(2,2,2-trifluoro-ethoxy)-pyrimidine-5-carbonitrile as colorless solid; MS: [M+H]⁺ = 393; m.p. 114-116 °C.

### Example 97

### 2-Hydroxymethyl-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with glycolamidine hydrochloride [J. Amer. Chem. Soc. 68, 2393-2395 (1946)] and 1,8-diazabicyclo[5.4.0]undec-7-en in N,N-dimethylformamide at 110 °C to yield the 2-hydroxymethyl-6-oxo-4-(1,2,4,5-tetrahydro-benzo[d]azepin-3-yl)-1,6-dihydro-pyrimidine-5-carbonitrile as colorless solid; m.p. 196-198 °C; MS: [M]⁺ = 296.

### Example 98

### 2,3-Diethyl-5-nitro-6-(1,2,4,5-tetrahydro-benzo[d]azepin-3-yl)-3H-pyrimidin-4-one

From 3-ethyl-2-methyl-5-nitro-6-(1,2,4,5-tetrahydro-benzo[d]azepin-3-yl)-3H-pyrimidin-4-one and methyl iodide, lithium-bis-(trimethylsilyl) amide in tetrahydrofuran, as yellow oil; MS: [M+H]⁺ = 343; see example 99.

### Example 99

### 3-Ethyl-2-isopropyl-5-nitro-6-(1,2,4,5-tetrahydro-benzo[d]azepin-3-yl)-3H-pyrimidin-4-one

0.328 g (1.0 mmol) of 3-ethyl-2-methyl-5-nitro-6-(1,2,4,5-tetrahydro-benzo[d]azepin-3-yl)-3H-pyrimidin-4-one (example 2) were dissolved in 10.0 ml of tetrahydrofuran, the solution cooled in an argon atmosphere to -70 °C and treated with 1.2 ml of an 1.0 M solution of lithium-bis-(trimethylsilyl)amide in tetrahydrofuran, stirred at -70 °C for 120 minutes and treated with 0.095 ml (1.5 mmol) of methyliodide. The reaction mixture was then allowed to come to room temperature and stirring continued overnight, subsequently poured into 50 ml of an ice/water mixture and extracted three times with 100 ml of ethylacetate. The combined organic phases were washed twice with 50 ml of water, dried over magnesium sulphate, evaporated under reduced pressure and dried in a high vacuum. The thus obtained crude product was purified by chromatography on silica gel using a 9:1 to 1:1 v/v mixture of hexane and ethyl acetate as eluent to yield 0.055 g (0.161 mmol), 16.1%, of 2,3-diethyl-5-nitro-6-(1,2,4,5-tetrahydro-benzo[d]azepin-3-yl)-3H-pyrimidin-4-one as yellow oil; MS: [M+H]⁺ = 343; and 0.012 g (0.034 mmol), 3.4 %, of 3-ethyl-2-isopropyl-5-nitro-6-( 1,2,4,5-tetrahydro-benzo[d]azepin-3-yl)-3H-pyrimidin-4-one as yellow oil; MS: [M+H]⁺ = 357.

### Example 100

### 3-(4-Butyl-5-methyl-3-nitro-pyridin-2-yl)-2,3,4,5-tetrahydro-1H-benzo[d]azepine

0.283 g (1.0 mmol) of 3-(5-methyl-3-nitro-pyridin-2-yl)-2,3,4,5-tetrahydro-1H-benzo[d]azepine (example 87) were dissolved in 10.0 ml of tetrahydrofuran, the solution cooled in an argon atmosphere to -70 °C and treated with 0.94 ml of an 1.6 M solution of n-butyllithium in n-hexane, stirred at -70 °C for 120 minutes, then allowed to come to room temperature and stirring continued overnight. Subsequently it was poured into 50 ml of an ice/water mixture and extracted three times with 100 ml of ethylacetate. The combined organic phases were washed twice with 50 ml of water, dried over magnesium sulphate, evaporated under reduced pressure and dried in a high vacuum. The thus obtained crude product was purified by chromatography on silica gel using a 98:2 to 9:1 v/v mixture of hexane and ethyl acetate as eluent to yield 0.071 g (0.21 mmol), 21 %, of 3-(4-butyl-5-methyl-3-nitro-pyridin-2-yl)-2,3,4,5-tetrahydro-1H-benzo[d]azepine as yellow oil; MS: [M+H]⁺ = 340.

### Example 101

### 6-(6-Methoxy-1,2,4,5-tetrahydro-benzo[d]azepin-3-yl)-2-methyl-5-nitro-3H-pyrimidin-4-one

In analogy to the procedure described in example 4 the 6-bromo-2-methyl-5-nitro-3H-pyrimidin-4-one (see example 66) was treated with the 6-methoxy-2,3,4,5-tetrahydro-1H-benzo[d]azepine [J. Med. Chem. (1984), 27(7), 918-21] in N,N-dimethylformamide in the presence of N-ethyl-N,N-diisopropylamine at room temperature to yield the 6-(6-methoxy-1,2,4,5-tetrahydro-benzo[d]azepin-3-yl)-2-methyl-5-nitro-3H-pyrimidin-4-one as yellow solid; m.p. >200 °C; MS: [M+H]⁺ = 331.

### Example 102

### 2-[2-Methyl-5-nitro-6-(1,2,4,5-tetrahydro-benzo[d]azepin-3-yl)-pyrimidin-4-yloxy]-ethylamine

a) 0.300 g (1.00 mmol) of the 2-methyl-5-nitro-6-(1,2,4,5-tetrahydro-benzo[d]azepin-3-yl)-pyrimidin-4-ol (example 1), 0.194 g (1.20 mmol) of N-tert-butoxycarbonyl-aminoethanol and 0.371 g (1.40 mmol) of triphenylphosphin were dissolved in 15.0 ml of tetrahydrofuran, the solution cooled to 2 °C and treated with 0.306 g (1.30 mmol) of di-tert-butyl azodicarboxylate. The reaction mixture was then allowed to come to room temperature and stirring continued for 22 hours. Subsequently it was poured into 50 ml of an ice/water mixture and extracted three times with 100 ml of ethylacetate. The combined organic phases were washed twice with 50 ml of water, dried over magnesium sulphate, evaporated under reduced pressure and dried in a high vacuum. The thus obtained crude product was purified by chromatography on silica gel using a 95:5 to 4:1 v/v mixture of hexane and ethyl acetate as eluent to yield impure {2-[2-methyl-5-nitro-6-(1,2,4,5-tetrahydro-benzo[d]azepin-3-yl)-pyrimidin-4-yloxy]-ethyl}-carbamic acid tert-butyl ester as yellow solid; MS: [M+H]⁺ = 444.
b) The impure {2-[2-methyl-5-nitro-6-(1,2,4,5-tetrahydro-benzo[d]azepin-3-yl)-pyrimidin-4-yloxy]-ethyl}-carbamic acid tert-butyl ester prepared in a) was dissolved in 10.0 ml of methanol and treated with 5.96 ml of an 1.56 M solution of hydrogen chloride in methanol and the reaction mixture stirred at room temperature for 72 hours. Subsequently it was poured into 50 ml of an ice/water mixture, neutralized with sodium hydrogen carbonate solution and extracted three times with 100 ml of diochloromethane. The combined organic phases were washed twice with 50 ml of water, dried over magnesium sulphate, evaporated under reduced pressure and dried in a high vacuum. The thus obtained crude product was purified by chromatography on silica gel using a 9:1 to 0:1 v/v mixture of hexane and ethyl acetate as eluent to yield 0.058 g (0.169 mmol), 16.9% over two steps, of the 2-[2-methyl-5-nitro-6-(1,2,4,5-tetrahydro-benzo[d]azepin-3-yl)-pyrimidin-4-yloxy]-ethylamine as light yellow oil; MS: [M+H]⁺ = 344.

### Example 103

### 4-(Bromo-difluoro-methoxy)-6-(1,2,4,5-tetrahydro-benzo[d]azepin-3-yl)-pyrimidine-5-carbonitrile

In analogy to the procedure described in example 60 the 6-oxo-4-(1,2,4,5-tetrahydro-benzo[d]azepin-3-yl)-1,6-dihydro-pyrimidine-5-carbonitrile (example 15) was treated with dibromo-difluoro-methane in N,N-dimethylformamide in the presence of sodium hydride at room temperature to yield the 4-(bromo-difluoro-methoxy)-6-(1,2,4,5-tetrahydro-benzo[d]azepin-3-yl)-pyrimidine-5-carbonitrile as colorless solid; m.p. 95-100 °C; MS: [M+H]⁺ = 396.

### Example 104

### 6-Oxo-4-(1,2,4,5-tetrahydro-benzo[d]azepin-3-yl)-2-(tetrahydro-pyran-2-yloxymethyl)-1,6-dihydro-pyrimidine-5-carbonitrile

In analogy to the procedure described in example 67 the 2-hydroxymethyl-6-oxo-4-(1,2,4,5-tetrahydro-benzo[d]azepin-3-yl)-1,6-dihydro-pyrimidine-5-carbonitrile (example 97) was treated with 3,4-dihydro-2H-pyran and pyridinium-(toluene-4-sulfonate) in dichloromethane at room temperature to yield the 6-oxo-4-(1,2,4,5-tetrahydro-benzo[d]azepin-3-yl)-2-(tetrahydro-pyran-2-yloxymethyl)-1,6-dihydro-pyrimidine-5-carbonitrile as colorless solid; m.p. 179.5-183 °C; MS: [M+H]⁺ = 381.

### Example 105

### 4-(1,2,4,5-Tetrahydro-benzo[d]azepin-3-yl)-2-(tetrahydro-pyran-2-yloxymethyl)-6-(2,2,2-trifluoro-ethoxy)-pyrimidine-5-carbonitrile

From the 6-oxo-4-(1,2,4,5-tetrahydro-benzo[d]azepin-3-yl)-2-(tetrahydro-pyran-2-yloxymethyl)-1,6-dihydro-pyrimidine-5-carbonitrile (example 104) and 2,2,2-trifluoroethyl trifluoromethanesulfonate in N,N-dimethylformamide in the presence of potassium carbonate as colorless amorphous solid; MS: [M+H]⁺ = 463; see example 106.

### Example 106

### 6-Oxo-4-(1,2,4,5-tetrahydro-benzo[d]azepin-3-yl)-2-(tetrahydro-pyran-2-yloxymethyl)-1-(2,2,2-trifluoro-ethyl)-1,6-dihydro-pyrimidine-5-carbonitrile

In analogy to the procedure described in example 3 the 6-oxo-4-(1,2,4,5-tetrahydro-benzo[d]azepin-3-yl)-2-(tetrahydro-pyran-2-yloxymethyl)-1,6-dihydro-pyrimidine-5-carbonitrile (example 104) was treated with 2,2,2-trifluoroethyl trifluoromethanesulfonate in N,N-dimethylformamide in the presence of potassium carbonate to yield the 4-(1,2,4,5-tetrahydro-benzo[d]azepin-3-yl)-2-(tetrahydro-pyran-2-yloxymethyl)-6-(2,2,2-trifluoro-ethoxy)-pyrimidine-5-carbonitrile as colorless amorphous solid; MS: [M+H]⁺ = 463; and the 6-oxo-4-(1,2,4,5-tetrahydro-benzo[d]azepin-3-yl)-2-(tetrahydro-pyran-2-yloxymethyl)-1-(2,2,2-trifluoro-ethyl)-1,6-dihydro-pyrimidine-5-carbonitrile as colorless amorphous solid; MS: [M+H]⁺ = 463.

### Example 107

### 2-Hydroxymethyl-4-(1,2,4,5-tetrahydro-benzo[d]azepin-3-yl)-6-(2,2,2-trifluoro-ethoxy)-pyrimidine-5-carbonitrile

In analogy to the procedure described in example 70 the 4-(1,2,4,5-tetrahydro-benzo[d]azepin-3-yl)-2-(tetrahydro-pyran-2-yloxymethyl)-6-(2,2,2-trifluoro-ethoxy)-pyrimidine-5-carbonitrile (example 105) was treated with hydrogen chloride in methanol at room temperature to yield the 2-hydroxymethyl-4-(1,2,4,5-tetrahydro-benzo[d]azepin-3-yl)-6-(2,2,2-trifluoro-ethoxy)-pyrimidine-5-carbonitrile as colorless solid; m.p. 106-110 °C; MS: [M]⁺ = 378.

### Example 108

### 2-Hydroxymethyl-6-oxo-4-(1,2,4,5-tetrahydro-benzo[d]azepin-3-yl)-1-(2,2,2-trifluoro-ethyl)-1,6-dihydro-pyrimidine-5-carbonitrile

In analogy to the procedure described in example 70 the 6-oxo-4-(1,2,4,5-tetrahydro-benzo[d]azepin-3-yl)-2-(tetrahydro-pyran-2-yloxymethyl)-1-(2,2,2-trifluoro-ethyl)-1,6-dihydro-pyrimidine-5-carbonitrile (example 106) was treated with hydrogen chloride in methanol at room temperature to yield the 2-hydroxymethyl-6-oxo-4-(1,2,4,5-tetrahydro-benzo[d]azepin-3-yl)-1-(2,2,2-trifluoro-ethyl)-1,6-dihydro-pyrimidine-5-carbonitrile as colorless solid; m.p. 181-185 °C; MS: [M]⁺ = 378.

### Example 109

### 1-Allyl-2-amino-6-oxo-4-(1,2,4,5-tetrahydro-benzo[d]azepin-3-yl)-1,6-dihydro-pyrimidine-5-carbonitrile

From the 2-amino-6-oxo-4-(1,2,4,5-tetrahydro-benzo[d]azepin-3-yl)-1,6-dihydro-pyrimidine-5-carbonitrile (example 13) with allylbromide in N,N-dimethylformamide in the presence of potassium carbonate at room temperature as colorless solid; m.p. >200 °C; MS: [M]⁺ = 321; see example 111.

### Example 110

### 1-Allyl-2-allylamino-6-oxo-4-(1,2,4,5-tetrahydro-benzo[d]azepin-3-yl)-1,6-dihydro-pyrimidine-5-carbonitrile

From the 2-amino-6-oxo-4-(1,2,4,5-tetrahydro-benzo[d]azepin-3-yl)-1,6-dihydro-pyrimidine-5-carbonitrile (example 13) with allylbromide in N,N-dimethylformamide in the presence of potassium carbonate at room temperature as colorless solid; m.p. 181-182.5 °C; MS: [M]⁺ = 361; see example 111.

### Example 111

### 4-Allyloxy-2-amino-6-(1,2,4,5-tetrahydro-benzo[d]azepin-3-yl)-pyrimidine-5-carbonitrile

In analogy to the procedure described in example 3 the 2-amino-6-oxo-4-(1,2,4,5-tetrahydro-benzo[d]azepin-3-yl)-1,6-dihydro-pyrimidine-5-carbonitrile (example 13) was treated with allylbromide in N,N-dimethylformamide in the presence of potassium carbonate at room temperature to yield the 1-allyl-2-amino-6-oxo-4-(1,2,4,5-tetrahydro-benzo[d]azepin-3-yl)-1,6-dihydro-pyrimidine-5-carbonitrile as colorless solid; m.p. >200 °C; MS: [M]⁺ = 321; the 1-allyl-2-allylamino-6-oxo-4-(1,2,4,5-tetrahydro-benzo[d]azepin-3-yl)-1,6-dihydro-pyrimidine-5-carbonitrile as colorless solid; m.p. 181-182.5 °C; MS: [M]⁺ = 361; and the 4-allylox-2-amino-6-(1,2,4,5-tetrahydro-benzo[d]azepin-3-yl)-pyrimidine-5-carbonitrile as colorless amorphous solid; MS: [M+H]⁺ = 322.

### Example 112

### 1-Allyl-2-ethylamino-6-oxo-4-(1,2,4,5-tetrahydro-benzo[d]azepin-3-yl)-1,6-dihydro-pyrimidine-5-carbonitrile

In analogy to the procedure described in example 3 the 1-allyl-2-amino-6-oxo-4-(1,2,4,5-tetrahydro-benzo[d]azepin-3-yl)-1,6-dihydro-pyrimidine-5-carbonitrile (example 109) was treated with ethyliodide in N,N-dimethylformamide in the presence of potassium carbonate at room temperature to yield the 1-allyl-2-ethylamino-6-oxo-4-(1,2,4,5-tetrahydro-benzo[d]azepin-3-yl)-1,6-dihydro-pyrimidine-5-carbonitrile as colorless solid; m.p. 172-177 °C; MS: [M+H]⁺ = 350.

### Example 113

### 1-Allyl-2-(allyl-ethyl-amino)-6-oxo-4-(1,2,4,5-tetrahydro-benzo[d]azepin-3-yl)-1,6-dihydro-pyrimidine-5-carbonitrile

In analogy to the procedure described in example 3 the 1-allyl-2-allylamino-6-oxo-4-(1,2,4,5-tetrahydro-benzo[d]azepin-3-yl)-1,6-dihydro-pyrimidine-5-carbonitrile (example 110) was treated with ethylbromide in N,N-dimethylformamide in the presence of potassium carbonate at room temperature to yield the 1-allyl-2-(allyl-ethyl-amino)-6-oxo-4-(1,2,4,5-tetrahydro-benzo[d]azepin-3-yl)-1,6-dihydro-pyrimidine-5-carbonitrile as colorless solid; m.p. 138.5-140.5 °C; MS: [M+H]⁺ = 390.

### Example 114

### 1-Cyclopropyl-2-methyl-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with N-1-cyclopropyl acetamidine hydrochloride (from ethyl acetimidate hydrochloride and cyclopropylamine in ethanol at reflux, used as crude reaction product) and 1,8-diazabicyclo[5.4.0]undec-7-en in N,N-dimethylformamide at 100 °C to yield the 1-cyclopropyl-2-methyl-6-oxo-4-(1,2,4,5-tetrahydro-benzo[d]azepin-3-yl)-1,6-dihydro-pyrimidine-5-carbonitrile as light yellow solid; m.p. 177-179 °C; MS: [M+H]⁺ = 321.

### Example 115

### 5-Ethyl-6-methyl-2-(1,2,4,5-tetrahydro-benzo[d]azepin-3-yl)-nicotinonitrile

In analogy to the procedure described in example 1 the 2-choro-5-ethyl-6-methyl-nicotinonitrile [J. Med. Chem. (1992), 35(21), 3784-91] was treated with the 2,3,4,5-tetrahydro-1H-benzo[d]azepine hydrochloride [J. Heterocycl. Chem. (1971), 8(5), 779-83] in the presence of potassium carbonate in N,N-dimethylformamide at 120 °C to yield the 5-ethyl-6-methyl-2-(1,2,4,5-tetrahydro-benzo[d]azepin-3-yl)-nicotinonitrile as a light yellow oil; MS: [M+H]⁺ = 292.

### Example 116

### 5-Oxo-7-(1,2,4,5-tetrahydro-benzo[d]azepin-3-yl)-1,2,3,5-tetrahydro-imidazo[1,2-a]pyrimidine-6-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-iminoimidazolidine hydrobromide and 1,8-diazabicyclo[5.4.0]undec-7-en in N,N-dimethylformamide at 100 °C to yield the 5-oxo-7-(1,2,4,5-tetrahydro-benzo[d]azepin-3-yl)-1,2,3,5-tetrahydro-imidazo[1,2-a]pyrimidine-6-carbonitrile as colorless solid; m.p. >200 °C; MS: [M+H]⁺ = 308.

### Example 117

### 5-Oxo-7-(1,2,4,5-tetrahydro-benzo[d]azepin-3-yl)-1,5-dihydro-imidazo[1,2-a]pyrimidine-6-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-aminoimidazole sulfate and 1,8-diazabicyclo[5.4.0]undec-7-en in N,N-dimethylformamide at 100 °C to yield the 5-oxo-7-(1,2,4,5-tetrahydro-benzo[d]azepin-3-yl)-1,5-dihydro-imidazo[1,2-a]pyrimidine-6-carbonitrile as brownish solid; m.p. >200 °C; MS: [M+H]⁺ = 306.

### Example 118

### 1-Methyl-5-oxo-7-(1,2,4,5-tetrahydro-benzo[d]azepin-3-yl)-1,2,3,5-tetrahydro-imidazo[1,2-a]pyrimidine-6-carbonitrile

In analogy to the procedure described in example 3 the 5-oxo-7-(1,2,4,5-tetrahydro-benzo[d]azepin-3-yl)-1,2,3,5-tetrahydro-imidazo[1,2-a]pyrimidine-6-carbonitrile (example 116) was treated with dimethylsulfate in N,N-dimethylformamide in the presence of potassium carbonate at room temperature to yield the 1-methyl-5-oxo-7-(1,2,4,5-tetrahydro-benzo[d]azepin-3-yl)-1,2,3,5-tetrahydro-imidazo[1,2-a]pyrimidine-6-carbonitrile as colorless solid; m.p. 174-177 °C; MS: [M+H]⁺ = 322.

### Example 119

### 3-[2-Methyl-5-nitro-6-(2,2,2-trifluoro-ethoxy)-pyrimidin-4-yl]-2,3,4,5-tetrahydro-1H-benzo[d]azepine

In analogy to the procedure described in example 3 the 2-methyl-5-nitro-6-(1,2,4,5-tetrahydro-benzo[d]azepin-3-yl)-pyrimidin-4-ol (example 1) was treated with 2,2,2-trifluoroethyl iodide in N,N-dimethylformamide in the presence of potassium carbonate at 80 °C to yield beside the 2-methyl-5-nitro-6-(1,2,4,5-tetrahydro-benzo[d]azepin-3-yl)-3-(2,2,2-trifluoro-ethyl)-3H-pyrimidin-4-one (example 11) the 3-[2-methyl-5-nitro-6-(2,2,2-trifluoro-ethoxy)-pyrimidin-4-yl]-2,3,4,5-tetrahydro-1H-benzo[d]azepine as light yellow oil; MS: [M+H]⁺ = 383.

### Example 120

### 1-Allyl-6-oxo-4-(1,2,4,5-tetrahydro-benzo[d]azepin-3-yl)-2-(2,2,2-trifluoro-ethylamino)-1,6-dihydro-pyrimidine-5-carbonitrile

In analogy to the procedure described in example 3 the 1-allyl-2-amino-6-oxo-4-(1,2,4,5-tetrahydro-benzo[d]azepin-3-yl)-1,6-dihydro-pyrimidine-5-carbonitrile (example 109) was treated with 2,2,2-trifluoroethyl-trifluoromethanesulfonate in N,N-dimethylformamide in the presence of potassium carbonate at room temperature to yield the 1-allyl-6-oxo-4-(1,2,4,5-tetrahydro-benzo[d]azepin-3-yl)-2-(2,2,2-trifluoro-ethylamino)-1,6-dihydro-pyrimidine-5-carbonitrile as colorless solid; m.p. 186-191 °C; MS: [M+H]⁺ = 404.

### Example 121

### 6-Oxo-4-(1,2,4,5-tetrahydro-benzo[d]azepin-3-yl)-1-(2,2,2-trifluoro-ethyl)-2-(2,2,2-trifluoro-ethylamino)-1,6-dihydro-pyrimidine-5-carbonitrile

From the 2-amino-6-oxo-4-(1,2,4,5-tetrahydro-benzo[d]azepin-3-yl)-1,6-dihydro-pyrimidine-5-carbonitrile (example 13) with 2,2,2-trifluoroethyl-trifluoromethanesulfonate in N,N-dimethylformamide in the presence of potassium carbonate at room temperature as colorless solid; m.p. >200 °C; MS: [M]⁺ = 446; see example 122.

### Example 122

### 2-Amino-4-(1,2,4,5-tetrahydro-benzo[d]azepin-3-yl)-6-(2,2,2-trifluoro-ethoxy)-pyrimidine-5-carbonitrile

In analogy to the procedure described in example 3 the 2-amino-6-oxo-4-(1,2,4,5-tetrahydro-benzo[d]azepin-3-yl)-1,6-dihydro-pyrimidine-5-carbonitrile (example 13) was treated with 2,2,2-trifluoroethyl-trifluoromethanesulfonate in N,N-dimethylformamide in the presence of potassium carbonate at room temperature to yield the 2-amino-4-(1,2,4,5-tetrahydro-benzo[d]azepin-3-yl)-6-(2,2,2-trifluoro-ethoxy)-pyrimidine-5-carbonitrile as colorless solid; m.p. 145.3-146.9 °C; MS: [M+H]⁺ = 364; and the 6-oxo-4-(1,2,4,5-tetrahydro-benzo[d]azepin-3-yl)-1-(2,2,2-trifluoro-ethyl)-2-(2,2,2-trifluoro-ethylamino)-1,6-dihydro-pyrimidine-5-carbonitrile as colorless solid; m.p. >200 °C; MS: [M+H]⁺ = 446.

### Example 123

### 6-(7-Fluoro-1,2,4,5-tetrahydro-benzo[d]azepin-3-yl)-2-methyl-5-nitro-3H-primidin-4-one

In analogy to the procedure described in example 4 the 6-bromo-2-methyl-5-nitro-3H-pyrimidin-4-one (see example 66) was treated with the 7-fluoro-2,3,4,5-tetrahydro-1H-benzo[d]azepine in N,N-dimethylformamide in the presence of N-ethyl-N,N-diisopropylamine at room temperature to yield the 6-(7-fluoro-1,2,4,5-tetrahydro-benzo[d]azepin-3-yl)-2-methyl-5-nitro-3H-pyrimidin-4-one as light yellow solid; m.p. >200 °C; MS: [M+H]⁺ = 319.

### Preparation of the 7-fluoro-2,3,4,5-tetrahydro-1H-benzo[d]azepine

The 7-fluoro-2,3,4,5-tetrahydro-1H-benzo[d]azepine used above was prepared by the following reaction sequence: i) catalytic reduction of the 1-(7-nitro-1,2,4,5-tetrahydro-benzo[d]azepin-3-yl)-ethanone [J. Heterocycl. Chem. (1971), 8(5), 779-83] with hydrogen in methanol in the presence of palladium on charcoal to yield the 1-(7-amino-1,2,4,5-tetrahydro-benzo[d]azepin-3-yl)-ethanone; ii) treatment of the 1-(7-amino-1,2,4,5-tetrahydro-benzo[d]azepin-3-yl)-ethanone with nitrosonium tetrafluoroborate in dichloromethane and decomposition of the thus formed diazonium tetrafluoroborate in boiling 1,2-dichlorobenzene at 180°C to give the 1-(7-fluoro-1,2,4,5-tetrahydro-benzo[d]azepin-3-yl)-ethanone; iii) conversion of the 1-(7-fluoro-1,2,4,5-tetrahydro-benzo[d]azepin-3-yl)-ethanone into the 7-fluoro-2,3,4,5-tetrahydro-1H-benzo[d]azepine by removal of the acetyl function with hydrogen chloride (37% in water) in methanol at reflux.

### Example 124

### 1-(2-Hydroxy-ethyl)-2-methyl-6-oxo-4-(1,2,4,5-tetrahydro-benzo[d]azepin-3-yl)-1,6-dihydro-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) with 2-bromoethanol in acetonitrile in the presence of potassium carbonate at reflux as colorless solid; m.p. 164-167.5 °C; MS: [M]⁺ = 325; see example 125.

### Example 125

### 4-(2-Hydroxy-ethoxy)-2-methyl-6-(1,2,4,5-tetrahydro-benzo[d]azepin-3-yl)-pyrimidine-5-carbonitrile

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with 2-bromoethanol in acetonitrile in the presence of potassium carbonate at reflux to yield the 4-(2-hydroxy-ethoxy)-2-methyl-6-(1,2,4,5-tetrahydro-benzo[d]azepin-3-yl)-pyrimidine-5-carbonitrile as yellow solid; m.p. 86.7-92.3 °C; MS: [M]⁺ = 325; and the 1-(2-hydroxy-ethyl)-2-methyl-6-oxo-4-(1,2,4,5-tetrahydro-benzo[d]azepin-3-yl)-1,6-dihydro-pyrimidine-5-carbonitrile as colorless solid; m.p. 164-167.5 °C; MS: [M]⁺ = 325.

### Example 126

### 4-Oxo-2-(1,2,4,5-tetrahydro-benzo[d]azepin-3-yl)-4,6,7,8-tetrahydro-pyrrolo[1,2-a]pyrimidine-3-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-iminopyrrolidine hydrochloride [J. Med. Chem. (1996), 39, 669-672] and 1,8-diazabicyclo[5.4.0]undec-7-en in N,N-dimethylformamide at 100 °C to yield the 4-oxo-2-(1,2,4,5-tetrahydro-benzo[d]azepin-3-yl)-4,6,7,8-tetrahydro-pyrrolo[1,2-a]pyrimidine-3-carbonitrile as colorless solid; m.p. 184.5-190.5 °C; MS: [M+H]⁺ = 307.

### Example 127

### 4-Oxo-2-(1,2,4,5-tetrahydro-benzo[d]azepin-3-yl)-4,6,7,8,9,10-hexahydro-pyrimido[1,2-a]azepine-3-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-iminohomopiperidine hydroiodid [J. Med. Chem. (1996), 39, 669-672] and 1,8-diazabicyclo[5.4.0]undec-7-en in N,N-dimethylformamide at 100 °C to yield the 4-oxo-2-(1,2,4,5-tetrahydro-benzo[d]azepin-3-yl)-4,6,7,8,9,10-hexahydro-pyrimido[1,2-a]azepine-3-carbonitrile as colorless amorphous solid; MS: [M+H]⁺ = 335.

### Example 128

### 4-Oxo-2-(1,2,4,5-tetrahydro-benzo[d]azepin-3-yl)-6,7,8,9-tetrahydro-4H-pyrido[1,2-a]pyrimidine-3-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with 2-iminopiperidine hydrochlorid [J. Med. Chem. (1996), 39, 669-672] and 1,8-diazabiryclo[5.4.0]undec-7-en in N,N-dimethylformamide at 100 °C to yield the 4-oxo-2-(1,2,4,5-tetrahydro-benzo[d]azepin-3-yl)-6,7,8,9-tetrahydro-4H-pyrido[1,2-a]pyrimidine-3-carbonitrile as yellow solid; m.p. 136.5-139.5 °C; MS: [M+H]⁺ = 321.

### Example 129

### 5-Ethyl-6-hydroxymethyl-2-(1,2,4,5-tetrahydro-benzo[d]azepin-3-yl)-nicotinonitrile

From the 5-ethyl-6-methyl-1-oxy-2-(1,2,4,5-tetrahydro-benzo[d]azepin-3-yl)-nicotinonitrile with trifluoroacetic anhydride in dichloromethane at reflux as light brown oil; MS: [M+H]⁺ = 308; see example 130.

### Example 130

### Trifluoro-acetic acid 5-cyano-3-ethyl-6-(1,2,4,5-tetrahydro-benzo[d]azepin-3-yl)-pyridin-2-ylmethyl ester

A solution of 0.30 g (0.98 mmol) of 5-ethyl-6-methyl-1-oxy-2-(1,2,4,5-tetrahydro-benzo[d]azepin-3-yl)-nicotinonitrile and 3.0 g (14.2 mmol) of trifluoroacetic anhydride in 3.0 ml of dichloromethane was heated at reflux for 20 hours. The reaction mixture was then evaporated and the residue chromatographed on silica gel using a 99:1 v/v mixture of dichloromethane and methanol as eluent. Thus, 0.32 g (0.80 mmol), 82 %, trifluoro-acetic acid 5-cyano-3-ethyl-6-(1,2,4,5-tetrahydro-benzo[d]azepin-3-yl)-pyridin-2-ylmethyl ester were obtained as a yellow oil; MS: [M+H]⁺ = 404; and 0.053 g (0.17 mmol), 18 %, 5-ethyl-6-hydroxymethyl-2-(1,2,4,5-tetrahydro-benzo[d]azepin-3-yl)-nicotinonitrile as light brown oil; MS: [M+H]⁺ = 308.

### Preparation of the 5-ethyl-6-methyl-1-oxy-2-(1,2,4,5-tetrahydro-benzo[d]azepin-3-yl)-nicotinonitrile

The 5-ethyl-6-methyl-1-oxy-2-(1,2,4,5-tetrahydro-benzo[d]azepin-3-yl)-nicotinonitrile used above was prepared by the following reaction sequence: i) treatment of the 2-chloro-5-ethyl-6-methyl-nicotinonitrile [J. Med. Chem. (1992), 35(21), 3784-91] with hydrogen peroxide in trifluoroacetic acid at reflux to yield the 2-chloro-5-ethyl-6-methyl-1-oxy-nicotinonitrile; ii) treatment of the 2-chloro-5-ethyl-6-methyl-1-oxy-nicotinonitrile in analogy to the procedure described in example 4 with the 2,3,4,5-tetrahydro-1H-benzo[d]azepine hydrochloride [J. Heterocycl. Chem. (1971), 8(5), 779-83] in N,N-dimethylformamide in the presence of N-ethyl-N,N-diisopropylamine at room temperature to yield the 5-ethyl-6-methyl-1-oxy-2-(1,2,4,5-tetrahydro-benzo[d]azepin-3-yl)-nicotinonitrile as yellow solid; m.p. 162-166 °C; MS: [M]⁺ = 307.

### Example 131

### 3-(6-Ethoxy-2-methyl-5-nitro-pyrimidin-4-yl)-7-fluoro-2,3,4,5-tetrahydro-1H-benzo[d]azepine

From the 6-(7-fluoro-1,2,4,5-tetrahydro-benzo[d]azepin-3-yl)-2-methyl-5-nitro-3H-pyrimidin-4-one (example 123) with iodoethane and potassium carbonate in N,N-dimethylformamide at room temperature as light yellow amorphous solid; MS: [M+H]⁺ = 347; see example 132.

### Example 132

### 3-Ethyl-6-(7-fluoro-1,2,4,5-tetrahydro-benzo[d]azepin-3-yl)-2-methyl-5-nitro-3H-pyrimidin-4-one

In analogy to the procedure described in example 3 the 6-(7-fluoro-1,2,4,5-tetrahydro-benzo[d]azepin-3-yl)-2-methyl-5-nitro-3H-pyrimidin-4-one (example 123) was treated with iodoethane in N,N-dimethylformamide in the presence of potassium carbonate at room temperature to yield the 3-ethyl-6-(7-fluoro-1,2,4,5-tetrahydro-benzo[d]azepin-3-yl)-2-methyl-5-nitro-3H-pyrimidin-4-one as yellow oil; MS: [M+H]⁺ = 347; and the 3-(6-ethoxy-2-methyl-5-nitro-pyrimidin-4-yl)-7-fluoro-2,3,4,5-tetrahydro-1H-benzo[d]azepine as light yellow amorphous solid; MS: [M+H]⁺ = 347.

### Example 133

### 1-Allyl-6-oxo-2-(2-phenoxy-ethylamino)-4-(1,2,4,5-tetrahydro-benzo[d]azepin-3-yl)-1,6-dihydro-pyrimidine-5-carbonitrile

A suspension of 0.100 g (0.311 mmol) of the 1-allyl-2-amino-6-oxo-4-(1,2,4,5-tetrahydro-benzo[d]azepin-3-yl)-1,6-dihydro-pyrimidine-5-carbonitrile (example 109), 0.0738 g (0.367 mmol) of 2-phenoxyethyl bromide and 0.0887 g (0.662 mmol) of potassium carbonate in 1.0 ml of N,N-dimethylformamide was stirred at room temperature for 48 hours. The reaction mixture was then poured into 30 ml of ice-water, acidified with 1 N hydrogen chloride and extracted 3 times with 50 ml of ethylacetate. The combined ethylacetate phases were dried over magnesium sulfate and evaporated under reduced pressure. The residue was chromatographed on silica gel using a 1:1 v/v mixture of dichloromethane and ethylacetate as eluent and then crystallized from ether. There was thus obtained 0.063 g (0.143 mmol), 46 %, 1-allyl-6-oxo-2-(2-phenoxy-ethylamino)-4-(1,2,4,5-tetrahydro-benzo[d]azepin-3-yl)-1,6-dihydro-pyrimidine-5-carbonitrile as colorless solid; m.p. 157-160 °C; MS: [M+H]⁺ = 442.

### Example 134

### 6-Oxo-2-(2-phenoxy-ethylamino)-4-(1,2,4,5-tetrahydro-benzo[d]azepin-3-yl)-1,6-dihydro-pyrimidine-5-carbonitrile

0.0065 g (0.283 mmol) of lithium borohydride were added to a suspension of 0.050 g (0.113 mmol) 1-allyl-6-oxo-2-(2-phenoxy-ethylamino)-4-(1,2,4,5-tetrahydro-benzo[d]azepin-3-yl)-1,6-dihydro-pyrimidine-5-carbonitrile (example 133), 0.0005 g (0.0023 mmol) of palladium(II)acetate and 0.0012 g (0.0045 mmol) of triphenylphoshin in 2.0 ml of tetrahydrofuran and the reaction mixture was stirred at room temperature for 1 hour. It was then treated with a few drops of acetone, poured into 30 ml of ice-water and extracted 3 times with 50 ml of ethylacetate. The combined ethylacetate phases were dried over magnesium sulfate and evaporated under reduced pressure. The residue formed was then chromatographed on silica gel using a 95:5 v/v mixture of dichloromethane and methanol as eluent and crystallized from ethylacetate/ether. There was thus obtained 0.028 g (0.070 mmol), 62 %, 6-oxo-2-(2-phenoxy-ethylamino)-4-(1,2,4,5-tetrahydro-benzo[d]azepin-3-yl)-1,6-dihydro-pyrimidine-5-carbonitrile as beige solid; m.p. >200 °C; MS: [M+H]⁺ = 402.

### Example 135

### 4-Allyloxy-2-diallylamino-6-(1,2,4,5-tetrahydro-benzo[d]azepin-3-yl)-pyrimidine-5-carbonitrile

In analogy to the procedure described in example 3 the 2-amino-6-oxo-4-(1,2,4,5-tetrahydro-benzo[d]azepin-3-yl)-1,6-dihydro-pyrimidine-5-carbonitrile [example 13 b)] was treated with 3-bromo-1-propene in N,N-dimethylformamide in the presence of potassium carbonate to yield the 4-allyloxy-2-diallylamino-6-(1,2,4,5-tetrahydro-benzo[d]azepin-3-yl)-pyrimidine-5-carbonitrile as colorless solid; m.p. 125.9-130.0 °C; MS: [M+H]⁺ = 402.

### Example 136

### (S)-1-(2,2-Dimethyl-[1,3]dioxolan-4-ylmethyl)-2-methyl-6-oxo-4-(1,2,4,5-tetrahydro-benzo[d]azepin-3-yl)-1,6-dihydro-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) with (R)-(-)-2,2-dimethyl-4-(hydroxymethyl)-1,3-dioxolane-p-toluene-sulfonate and potassium carbonate in N,N-dimethylformamide at room temperature as colorless solid; m.p. 166.2-168.3 °C; MS: [M+H]⁺ = 395; see example 137.

### Example 137

### (R)-4-(2,2-Dimethyl-[1,3]dioxolan-4-ylmethoxy)-2-methyl-6-(1,2,4,5-tetrahydro-benzo[d]azepin-3-yl)-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with the (R)-(-)-2,2-dimethyl-4-(hydroxymethyl)-1,3-dioxolane-p-toluene-sulfonate in N,N-dimethylformamide in the presence of potassium carbonate to yield the (R)-4-(2,2-dimethyl-[1,3]dioxolan-4-ylmethoxy)-2-methyl-6-(1,2,4,5-tetrahydro-benzo[d]azepin-3-yl)-pyrimidine-5-carbonitrile as colorless solid; m. p. 109.8-111.5 °C; MS: [M+H]⁺ = 395; and the (S)-1-(2,2-dimethyl-[1,3]dioxolan-4-ylmethyl)-2-methyl-6-oxo-4-(1,2,4,5-tetrahydro-benzo[d]azepin-3-yl)-1,6-dihydro-pyrimidine-5-carbonitrile as colorless solid; m.p. 166.2-168.3 °C; MS: [M+H]⁺ = 395.

### Example 138

### N-[1-Allyl-5-cyano-6-oxo-4-(1,2,4,5-tetrahydro-benzo[d]azepin-3-yl)-1,6-dihydro-pyrimidin-2-yl]-formamide

From the 1-allyl-2-amino-6-oxo-4-(1,2,4,5-tetrahydro-benzo[d]azepin-3-yl)-1,6-dihydro-pyrimidine-5-carbonitrile (example 109) and potassium carbonate in N,N-dimethylformamide at 130 °C as yellow amorphous solid; MS: [M]⁺ = 349; see example 139.

### Example 139

### 2-Amino-6-oxo-1-[Z]-propenyl-4-(1,2,4,5-tetrahydro-benzo[d]azepin-3-yl)-1,6-dihydro-pyrimidine-5-carbonitrile

Heating of the 1-allyl-2-amino-6-oxo-4-(1,2,4,5-tetrahydro-benzo[d]azepin-3-yl)-1,6-dihydro-pyrimidine-5-carbonitrile (example 109) in the presence of potassium carbonate in N,N-dimethylformamide at 130 °C yielded the N-[1-allyl-5-cyano-6-oxo-4-(1,2,4,5-tetrahydro-benzo[d]axepin-3-yl)-1,6-dihydro-pyrimidin-2-yl)-formamide as yellow amorphous solid; MS: [M]⁺ = 349; and the 2-amino-6-oxo-1-[2]-propenyl-4-(1,2,4,5-tetrahydro-benzo[d]azepin-3-yl)-1,6-dihydro-pyrimidine-5-carbonitrile as colorless solid; m.p. >200 °C; MS: [M+H]⁺ = 322.

### Example 140

### 1-Allyl-6-oxo-2-(3-phenoxy-propylamino)-4-(1,2,4,5-tetrahydro-benzo[d]azepin-3-yl)-1,6-dihydro-pyrimidine-5-carbonitrile

In analogy to the procedure described in example 133 the 1-allyl-2-amino-6-oxo-4-(1,2,4,5-tetrahydro-benzo[d]azepin-3-yl)-1,6-dihydro-pyrimidine-5-carbonitrile (example 109) was treated with the 3-phenoxypropyl bromide in N,N-dimethylformamide in the presence of potassium carbonate at room temperature to yield the 1-allyl-6-oxo-2-(3-phenoxy-propylamino)-4-(1,2,4,5-tetrahydro-benzo[d]azepin-3-yl)-1,6-dihydro-pyrimidine-5-carbonitrile as colorless solid; m.p. 157-159 °C; MS: [M+H]⁺ = 456.

### Example 141

### 6-Oxo-2-(3-phenoxy-propylamino)-4-(1,2,4,5-tetrahydro-benzo[d]azepin-3-yl)-1,6-dihydro-pyrimidine-5-carbonitrile

In analogy to the procedure described in example 134 the 1-allyl-6-oxo-2-(3-phenoxy-propylamino)-4-(1,2,4,5-tetrahydro-benzo[d]azepin-3-yl)-1,6-dihydro-pyrimidine-5-carbonitrile (example 140) was treated with lithium borohydride, palladium(II)acetate and triphenylphosphin in tetrahydrofuran to yield the 6-oxo-2-(3-phenoxy-propylamino)-4-(1,2,4,5-tetrahydro-benzo[d]azepin-3-yl)-1,6-dihydro-pyrimidine-5-carbonitrile as colorless solid; m.p. 175-180 °C; MS: [M+H]⁺ = 416.

### Example 142

### 4-Allyloxy-2-(3-phenoxy-propylamino)-6-(1,2,4,5-tetrahydro-benzo[d]azepin-3-yl)-pyrimidine-5-carbonitrile

In analogy to the procedure described in example 133 the 4-allyloxy-2-amino-6-(1,2,4,5-tetrahydro-benzo[d]azepin-3-yl)-pyrimidine-5-carbonitrile (example 111) was treated with the 3-phenoxypropyl bromide in N,N-dimethylformamide in the presence of potassium carbonate at 130 °C to yield the 4-allyloxy-2-(3-phenoxy-propylamino)-6-(1,2,4,5-tetrahydro-benzo[d]azepin-3-yl)-pyrimidine-5-carbonitrile as colorless amorphous solid; MS: [M+H]⁺ = 456.

### Example 143

### 4-Allyloxy-6-(1,2,4,5-tetrahydro-benzo[d]azepin-3-yl)-2-(2,2,2-trifluoro-ethylamino)-pyrimidine-5-carbonitrile

In analogy to the procedure described in example 133 the 4-allyloxy-2-amino-6-(1,2,4,5-tetrahydro-benzo[d]azepin-3-yl)-pyrimidine-5-carbonitrile (example 111) was treated with the 2,2,2-trifluoroethyl-trifluoromethanesulfonate in N,N-dimethylformamide in the presence of sodium hydride at room temperature to yield the 4-allyloxy-6-(1,2,4,5-tetrahydro-benzo[d]azepin-3-yl)-2-(2,2,2-trifluoro-ethylamino)-pyrimidine-5-carbonitrile as colorless amorphous solid; MS: [M+H]⁺ = 404.

### Example 144

### (S)-1-(2,3-Dihydroxy-propyl)-2-methyl-6-oxo-4-(1,2,4,5-tetrahydro-benzo[d]azepin-3-yl)-1,6-dihydro-pyrimidine-5-carbonitrile

In analogy to the procedure described in example 70 the (S)-1-(2,2-dimethyl-[1,3]dioxolan-4-ylmethyl)-2-methyl-6-oxo-4-(1,2,4,5-tetrahydro-benzo[d]azepin-3-yl)-1,6-dihydro-pyrimidine-5-carbonitrile (example 136) was treated with 1.5N hydrogen chloride in methanol at room temperature to yield the (S)-1-(2,3-dihydroxy-propyl)-2-methyl-6-oxo-4-(1,2,4,5-tetrahydro-benzo[d]azepin-3-yl)-1,6-dihydro-pyrimidine-5-carbonitrile as colorless amorphous solid; MS: [M+H]⁺ = 355.

### Example 145

### (R)-4-(2,3-Dihydroxy-propoxy)-2-methyl-6-(1,2,4,5-tetrahydro-benzo[d]azepin-3-yl)-pyrimidine-5-carbonitrile

In analogy to the procedure described in example 70 the (R)-4-(2,2-dimethyl-[1,3]dioxolan-4-ylmethoxy)-2-methyl-6-(1,2,4,5-tetrahydro-benzo[d]azepin-3-yl)-pyrimidine-5-carbonitrile (example 137) was treated with 1.5N hydrogen chloride in methanol at room temperature to yield the (R)-4-(2,3-dihydroxy-propoxy)-2-methyl-6-(1,2,4,5-tetrahydro-benzo[d]azepin-3-yl)-pyrimidine-5-carbonitrile as colorless amorphous solid; MS: [M+H]⁺ = 355.

### Example 146

### 1-Allyl-2-(cyanomethyl-amino)-6-oxo-4-(1,2,4,5-tetrahydro-benzo[d]azepin-3-yl)-1,6-dihydro-pyrimidine-5-carbonitrile

In analogy to the procedure described in example 3 the 1-allyl-2-amino-6-oxo-4-(1,2,4,5-tetrahydro-benzo[d]azepin-3-yl)-1,6-dihydro-pyrimidine-5-carbonitrile (example 109) was treated with the bromoacetonitrile in N,N-dimethylformamide in the presence of sodium hydride at room temperature to yield the 1-allyl-2-(cyanomethyl-amino)-6-oxo-4-(1,2,4,5-tetrahydro-benzo[d]azepin-3-yl)-1,6-dihydro-pyrimidine-5-carbonitrile as colorless solid; m.p. 198-203 °C; MS: [M+H]⁺ = 361.

### Example 147

### 4-Amino-2-methyl-6-(1,2,4,5-tetrahydro-benzo[d]azepin-3-yl)-pyrimidine-5-carbonitrile

a) In analogy to the procedure described in example 13 a) the [bis(methylthio)methylene]propanedinitrile was treated with the 2,3,4,5-tetrahydro-1H-benzo[d]azepine hydrochloride [J. Heterocycl. Chem. (1971), 8(5), 779-83] in dimethylsulfoxide in the presence of potassium carbonate at 80 °C to yield the 2-[methylsulfanyl-(1,2,4,5-tetrahydro-benzo[d]azepin-3-yl)-methylene]-malononitrile as a yellowish solid; m.p. 129-132.5 °C ; MS: [M+H]⁺ = 270.
b) In analogy to the procedure described in example 13 b) the 2-[methylsulfanyl-(1,2,4,5-tetrahydro-benzo[d]azepin-3-yl)-methylene]-malononitrile was treated with the acetamidine hydrochloride and 1,8-diazabicyclo[5.4.0]undec-7-ene in N,N-dimethylformamide at 100 °C to yield the 4-amino-2-methyl-6-(1,2,4,5-tetrahydro-benzo[d]azepin-3-yl)-pyrimidine-5-carbonitrile as a light yellowsolid; m.p. >200 °C; MS: [M+H]⁺ = 280.

### Example 148

### 2-Methyl-4-(1,2,4,5-tetrahydro-benzo[d]azepin-3-yl)-6-(2,2,2-trifluoro-ethylamino)-pyrimidine-5-carbonitrile

In analogy to the procedure described in example 133 the 4-amino-2-methyl-6-(1,2,4,5-tetrahydro-benzo[d]azepin-3-yl)-pyrimidine-5-carbonitrile (example 147) was treated with the 2,2,2-trifluoroethyl-trifluoromethanesulfonate in N,N-dimethylformamide in the presence of sodium hydride at 60 °C to yield the 2-methyl-4-(1,2,4,5-tetrahydro-benzo[d]azepin-3-yl)-6-(2,2,2-trifluoro-ethylamino)-pyrimidine-5-carbonitrile as colorless amorphous solid; MS: [M+H]⁺ = 362.

### Example 149

### 4-Chloro-2-methylsulfanyl-6-(1,2,4,5-tetrahydro-benzo[d]azepin-3-yl)-pyrimidine-5-carbonitrile

In analogy to the procedure described in example 46 a) the 4,6-dichloro-2-methylsulfanyl-pyrimidine-5-carbonitrile [J. Heterocycl. Chem. (1971), 8, 445-453] was treated with the 2,3,4,5-tetrahydro-1H-benzo[d]azepine hydrochloride [J. Heterocycl. Chem. (1971), 8(5), 779-83] in N,N-dimethylformamide in the presence of potassium carbonate at 50 °C to yield the 4-chloro-2-methylsulfanyl-6-(1,2,4,5-tetrahydro-benzo[d]azepin-3-yl)-pyrimidine-5-carbonitrile as colorless solid; m.p. 144-150 °C; MS: [M+H]⁺ = 331.

### Example 150

### 3-[2-Ethyl-5-nitro-6-(2,2,2-trifluoro-ethoxy)-pyrimidin-4-yl]-2,3,4,5-tetrahydro-1H-benzo[d]azepine

In analogy to the procedure described in example 99 the 3-[2-methyl-5-nitro-6-(2,2,2-trifluoro-ethoxy)-pyrimidin-4-yl]-2,3,4,5-tetrahydro-1H-benzo[d]azepine (example 119) was treated with the lithium-bis-(trimethylsilyl)amid and methyliodide in tetrahydrofuran between -70 °C and room temperature to yield the 3-[2-ethyl-5-nitro-6-(2,2,2-trifluoro-ethoxy)-pyrimidin-4-yl]-2,3,4,5-tetrahydro-1H-benzo[d]azepine as colorless oil; MS: [M+H]⁺ = 397.

### Example 151

### 1-Allyl-6-oxo-2-[(pyridin-3-ylmethyl)-amino]-4-(1,2,4,5-tetrahydro-benzo[d]azepin-3-yl)-1,6-dihydro-pyrimidine-5-carbonitrile

In analogy to the procedure described in example 133 the 1-allyl-2-amino-6-oxo-4-(1,2,4,5-tetrahydro-benzo[d]azepin-3-yl)-1,6-dihydro-pyrimidine-5-carbonitrile (example 109) was treated with the 3-(chloromethyl)pyridine hydrochloride and sodium hydride in N,N-dimethylformamide at room temperature to yield the 1-allyl-6-oxo-2-[(pyridin-3-ylmethyl)-amino]-4-(1,2,4,5-tetrahydro-benzo[d]azepin-3-yl)-1,6-dihydro-pyrimidine-5-carbonitrile as yellowish solid; m.p. >200 °C; MS: [M+H]⁺ = 413.

### Example 152

### 1-Allyl-2-(2-ethoxy-ethylamino)-6-oxo-4-(1,2,4,5-tetrahydro-benzo[d]azepin-3-yl)-1,6-dihydro-pyrimidine-5-carbonitrile

In analogy to the procedure described in example 133 the 1-allyl-2-amino-6-oxo-4-(1,2,4,5-tetrahydro-benzo[d]azepin-3-yl)-1,6-dihydro-pyrimidine-5-carbonitrile (example 109) was treated with the 2-ethoxyethyl methanesulfonate and potassium carbonate in N,N-dimethylformamide at 100 °C to yield the 1-allyl-2-(2-ethoxy-ethylamino)-6-oxo-4-(1,2,4,5-tetrahydro-benzo[d]azepin-3-yl)-1,6-dihydro-pyrimidine-5-carbonitrile as yellowish amorphous solid; MS: [M+H]⁺ = 394.

### Example 153

### 2-(Cyanomethyl-amino)-6-oxo-4-(1,2,4,5-tetrahydro-benzo[d]azepin-3-yl)-1,6-dihydro-pyrimidine-5-carbonitrile

In analogy to the procedure described in example 134 the 1-allyl-2-(cyanomethyl-amino)-6-oxo-4-(1,2,4,5-tetrahydro-benzo[d]azepin-3-yl)-1,6-dihydro-pyrimidine-5-carbonitrile (example 146) was treated with lithium borohydride, palladium(II)acetate and triphenylphoshin in tetrahydrofuran at room temperature to yield the 2-(cyanomethylamino)-6-oxo-4-(1,2,4,5-tetrahydro-benzo[d]azepin-3-yl)-1,6-dihydro-pyrimidine-5-carbonitrile as brownish solid; m.p. >200 °C; MS: [M+H]⁺ = 321.

### Example 154

### N-[5-cyano-1-ethyl-6-oxo-4-(1,2,4,5-tetrahydro-benzo[d]azepin-3-yl)-1,6-dihydro-pyrimidin-2-yl]-formamide

A suspension of 0.104 g (0.336 mmol) of 2-amino-1-ethyl-6-oxo-4-(1,2,4,5-tetrahydro-benzo[d]azepin-3-yl)-1,6-dihydro-pyrimidine-5-carbonitrile (example 30), 0.6 ml of ethylformiate and 0.093g (0.67 mmol) of potassium carbonate in 1.2 ml of N,N-dimethylformamide was stirred at 120 °C for 30 hours. The reaction mixture was then poured into 30 ml of ice/1 N hydrogen chloride solution and filtered. The residue was chromatographed on silica gel using a 1:1 v/v mixture of dichloromethane and ethylacetate as eluent and crystallized from ether. There was thus obtained 0.038 g (0.113 mmol), 33.5 %, N-[5-cyano-1-ethyl-6-oxo-4-(1,2,4,5-tetrahydro-benzo[d]azepin-3-yl)-1,6-dihydro-pirimidin-2-yl]-formamide as colorless solid; m.p. >200 °C; MS: [M+H]⁺ = 338.

### Example 155

### 4-(2-Hydroxy-ethylamino)-2-methylsulfanyl-6-(1,2,4,5-tetrahydro-benzo[d]azepin-3-yl)-pyrimidine-5-carbonitrile

In analogy to the procedure described in example 48 the 4-chloro-2-methylsulfanyl-6-(1,2,4,5-tetrahydro-benzo[d]azepin-3-yl)-pyrimidine-5-carbonitrile (example 149) was treated with the ethanolamine in ethanol at 80 °C to yield the 4-(2-hydroxy-ethylamino)-2-methylsulfanyl-6-(1,2,4,5-tetrahydro-benzo[d]azepin-3-yl)-pyrimidine-5-carbonitrile as colorless foam; MS: [M+H]⁺ = 356.

### Example 156

### 4-(3-Imidazol-1-yl-propylamino)-2-methylsulfanyl-6-(1,2,4,5-tetrahydro-benzo[d]azepin-3-yl)-pyrimidine-5-carbonitrile

In analogy to the procedure described in example 48 the 4-chloro-2-methylsulfanyl-6-(1,2,4,5-tetrahydro-benzo[d]azepin-3-yl)-pyrimidine-5-carbonitrile (example 149) was treated with the 1-(3-aminopropyl)-imidazole in dioxane in the presence of potassium carbonate at 90 °C to yield the 4-(3-imidazol-1-yl-propylamino)-2-methylsulfanyl-6-(1,2,4,5-tetrahydro-benzo[d]azepin-3-yl)-pyrimidine-5-carbonitrile as colorless solid; m.p. 131.0-133.5 °C; MS: [M+H]⁺ = 420.

### Example 157

### 5-Methylsulfanyl-7-(1,2,4,5-tetrahydro-benzo[d]azepin-3-yl)-2,3-dihydro-imidazo[1,2-c]pyrimidine-8-carbonitrile hydrochloride

A solution of 0.160 g (0.41 mmol) of 4-(2-hydroxy-ethylamino)-2-methylsulfanyl-6-(1,2,4,5-tetrahydro-benzo[d]azepin-3-yl)-pyrimidine-5-carbonitrile (example 155) and 0.5 ml (6.8 mmol) of thionyl chloride in 5.0 ml of chloroform was stirred at 90 °C for 2 hours. The reaction mixture was then evaporated under reduced pressure, suspended in dichloromethane and the not soluble crystals filtered off. There was thus obtained 0.050 g (0.134 mmol), 33 %, of the 5-methylsulfanyl-7-(1,2,4,5-tetrahydro-benzo[d]azepin-3-yl)-2,3-dihydro-imidazo[1,2-c]pyrimidine-8-carbonitrile hydrochloride as yellow solid; m.p. >200 °C; MS: [M+H]⁺ = 374.

### Example 158

### 3-(2-Methyl-5-nitro-6-oxo-1,6-dihydro-pyrimidin-4-yl)-2,3,4,5-tetrahydro-benzo[d]azepin-1-one

In analogy to the procedure described in example 4 the 6-bromo-2-methyl-5-nitro-3H-pyrimidin-4-one (see example 66) was treated with the 2,3,4,5-tetrahydro-benzo[d]azepin-1-one hydrochloride (1:1) [J. Chem. Soc., Perkin Trans. 1 (1975), (7), 622-6] in N,N-dimethylformamide in the presence of N-aethyldiisopropylamine at room temperature to yield the 3-(2-methyl-5-nitro-6-oxo-1,6-dihydro-pyrimidin-4-yl)-2,3,4,5-tetrahydro-benzo[d]azepin-1-one as light yellow amorphous solid; MS: [M+H]⁺ = 315.

### Example 159

### [rac]-6-(1-Hydroxy-1,2,4,5-tetrahydro-benzo[d]azepin-3-yl)-2-methyl-5-nitro-3H-pyrimidin-4-one

In analogy to the procedure described in example 4 the (6-bromo-2-methyl-5-nitro-3H-pyrimidin-4-one (see example 66) was treated with the [rac]-2,3,4,5-tetrahydro-1H-benzo[d]azepin-1-ol [J. Chem. Soc., Perkin Trans. 1 (1975), (7), 622-6] in N,N-dimethylformamide in the presence of N-aethyldiisopropylamine at room temperature to yield the [rac]-6-(1-hydroxy-1,2,4,5-tetrahydro-benzo[d]azepin-3-yl)-2-methyl-5-nitro-3H-pyrimidin-4-one as light yellow solid; m.p. >200 °C; MS: [M-H]⁻ = 315.

### Example 160

### 2-Methylsulfanyl-4-(3-pyridin-2-yl-propoxy)-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the 3-(3-pyridyl)-1-propanol in tetrahydrofuran in the presence of sodium hydride at room temperature to yield the 2-methylsulfanyl-4-(3-pyridin-2-yl-propoxy)-6-(1,2,4,5-tetrahydro-benzo[d]azepin-3-yl)-pyrimidine-5-carbonitrile as light brown oil; MS: [M+H]⁺ = 432.

### Example 161

### 2-Methylsulfanyl-4-[2-(2-oxo-pyrrolidin-1-yl)-ethoxy]-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the 1-(2-hydroxyethyl)-2-pyrrolidone in tetrahydrofuran in the presence of sodium hydride at room temperature to yield the 2-methylsulfanyl-4-[2-(2-oxo-pyrrolidin-1-yl)-ethoxy]-6-(1,2,4,5-tetrahydro-benzo[d]azepin-3-yl)-pyrimidine-5-carbonitrile as colorless solid; m.p. 126-128.5 °C; MS: [M+H]⁺ = 424.

### Example 162

### 4-(4-Methyl-pentyloxy)-2-methylsulfanyl-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the 4-methyl-1-pentanol in tetrahydrofuran in the presence of sodium hydride at room temperature to yield the 4-(4-methyl-pentyloxy)-2-methylsulfanyl-6-(1,2,4,5-tetrahydro-benzo[d]azepin-3-yl)-pyrimidine-5-carbonitrile as colorless oil; MS: [M+H]⁺ = 397.

### Example 163

### 2-Methylsulfanyl-4-(2-morpholin-4-yl-ethoxy)-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the 4-(2-hydroxyethyl)-morpholine in tetrahydrofuran in the presence of sodium hydride at room temperature to yield the 2-methylsulfanyl-4-(2-morpholin-4-yl-ethoxy)-6-(1,2,4,5-tetrahydro-benzo[d]azepin-3-yl)-pyrimidine-5-carbonitrile as colorless oil; MS: [M+H]⁺ = 426.

### Example 164

### 4-Amino-2-methylsulfanyl-6-(1,2,4,5-tetrahydro-benzo[d]azepin-3-yl)-pyrimidine-5-carbonitrile

In analogy to the procedure described in example 48 the 4-chloro-2-methylsulfanyl-6-(1,2,4,5-tetrahydro-benzo[d]azepin-3-yl)-pyrimidine-5-carbonitrile (example 149) was treated with aqueous ammonia at room temperature to yield the 4-amino-2-methylsulfanyl-6-(1,2,4,5-tetrahydro-benzo[d]azepin-3-yl)-pyrimidine-5-carbonitrile as colorless solid; m.p. >200 °C; MS: [M+H]⁺ = 312.

### Example 165

### 4-Allyloxy-2-methylsulfanyl-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the allylalkohol in tetrahydrofuran in the presence of sodium hydride at room temperature to yield the 4-allyloxy-2-methylsulfanyl-6-(1,2,4,5-tetrahydro-benzo[d]azepin-3-yl)-pyrimidine-5-carbonitrile as colorless solid; m.p. 102.6-104.8 °C; MS: [M+H]⁺ = 353.

### Example 166

### 2-Methylsulfanyl-4-(1,2,4,5-tetrahydro-benzo[d]azepin-3-yl)-6-(2,2,2-trifluoro-ethoxy)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the trifluoroethanol in tetrahydrofuran in the presence of sodium hydride at room temperature to yield the 2-methylsulfanyl-4-(1,2,4,5-tetrahydro-benzo[d]azepin-3-yl)-6-(2,2,2-trifluoro-ethoxy)-pyrimidine-5-carbonitrile as colorless solid; m.p. 107.9-110.5 °C; MS: [M+H]⁺ = 395.

### Example 167

### 2-Methylsulfanyl-4-(2-morpholin-4-yl-ethylamino)-6-(1,2,4,5-tetrahydro-benzo[d]azepin-3-yl)-pyrimidine-5-carbonitrile

In analogy to the procedure described in example 48 the 4-chloro-2-methylsulfanyl-6-(1,2,4,5-tetrahydro-benzo[d]azepin-3-yl)-pyrimidine-5-carbonitrile (example 149) was treated with the aminoethyl-morpholine in dioxane in the presence of potassium carbonate at 90 °C to yield the 2-methylsulfanyl-4-(2-morpholin-4-yl-ethylamino)-6-(1,2,4,5-tetrahydro-benzo[d]azepin-3-yl)-pyrimidine-5-carbonitrile as colorless amorphous solid; MS: [M+H]⁺ = 425.

### Example 168

### 4-(2-Methoxy-ethoxy)-2-methylsulfanyl-6-(1,2,4,5-tetrahydro-benzo[d]azepin-3-yl)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the diethylen-glycol-monomethylether in tetrahydrofuran in the presence of sodium hydride at room temperature to yield the 4-(2-methoxy-ethoxy)-2-methylsulfanyl-6-(1,2,4,5-tetrahydro-benzo[d]azepin-3-yl)-pyrimidine-5-carbonitrile as colorless amorphous solid; MS: [M+H]⁺ = 371.

### Example 169

### [rac]-3-(6-Ethoxy-2-methyl-5-nitro-pyrimidin-4-yl)-2,3,4,5-tetrahydro-1H-benzo[d]azepin-1-ol

From [rac]-6-(1-hydroxy-1,2,4,5-tetrahydro-benzo[d]azepin-3-yl)-2-methyl-5-nitro-3H-pyrimidin-4-one (example 159) and iodoethane, potassium carbonate in N,N-dimethylformamide at room temperature as light yellow oil; MS: [M+H]⁺ = 345; see example 170.

### Example 170

### [rac]-3-Ethyl-6-(1-hydroxy-1,2,4,5-tetrahydro-benzo[d]azepin-3-yl)-2-methyl-5-nitro-3H-pyrimidin-4-one

In analogy to the procedure described in example 3 the [rac]-6-(1-hydroxy-1,2,4,5-tetrahydro-benzo[d]azepin-3-yl)-2-methyl-5-nitro-3H-pyrimidin-4-one (example 159) was treated with the iodoethane in N,N-dimethylformamide in the presence of potassium carbonate at room temperature to yield the [rac]-3-ethyl-6-(1-hydroxy-1,2,4,5-tetrahydro-benzo[d]azepin-3-yl)-2-methyl-5-nitro-3H-pyrimidin-4-one as yellow amorphous solid; MS: [M+H]⁺ = 345; and the [rac]-3-(6-ethoxy-2-methyl-5-nitro-pyrimidin-4-yl)-2,3,4,5-tetrahydro-1H-benzo[d]azepin-1-ol as light yellow oil; MS: [M+H]⁺ = 345.

### Example 171

### 4-Chloro-6-(1,2,4,5-tetrahydro-benzo[d]azepin-3-yl)-2-(2,2,2-trifluoro-ethoxy)-pyrimidine-5-carbonitrile

In analogy to the procedure described in example 47 a) the 4-chloro-2-methanesulfonyl-6-(1,2,4,5-tetrahydro-benzo[d]azepin-3-yl)-pyrimidine-5-carbonitrile was treated with the 2,2,2-trifluoroethanol in tetrahydrofuran in the presence of sodium hydride at room temperature to yield the 4-chloro-6-(1,2,4,5-tetrahydro-benzo[d]azepin-3-yl)-2-(2,2,2-trifluoro-ethoxy)-pyrimidine-5-carbonitrile as colorless solid; m.p. 165-167 °C; MS: [M+H]⁺ = 383.

### Preparation of the 4-chloro-2-methanesulfonyl-6-(1,2,4,5-tetrahydro-benzo[d]azepin-3-yl)-pyrimidine-5-carbonitrile

Oxidation of the 4-chloro-2-methylsulfanyl-6-(1,2,4,5-tetrahydro-benzo[d]azepin-3-yl)-pyrimidine-5-carbonitrile (example 149) with m-chloroperbenzoic acid in dichloromethane at room temperature yielded the 4-chloro-2-methanesulfonyl-6-(1,2,4,5-tetrahydro-benzo[d]azepin-3-yl)-pyrimidine-5-carbonitrile as colorless solid; MS: [M+H]⁺ = 363.

### Example 172

### 2-(3-Phenoxy-propoxy)-4-(1,2,4,5-tetrahydro-benzo[d]azepin-3-yl)-6-(2,2,2-trifluoro-ethoxy)-pyrimidine-5-carbonitrile

In analogy to the procedure described in example 47 a) the 2-methanesulfonyl-4-(1,2,4,5-tetrahydro-benzo[d]azepin-3-yl)-6-(2,2,2-trifluoro-ethoxy)-pyrimidine-5-carbonitrile was treated with the 3-phenoxypropanol in tetrahydrofuran in the presence of sodium hydride at room temperature to yield the 2-(3-phenoxy-propoxy)-4-(1,2,4,5-tetrahydro-benzo[d]azepin-3-yl)-6-(2,2,2-trifluoro-ethoxy)-pyrimidine-5-carbonitrile as colorless oil; MS: [M+H]⁺ = 499.

### Preparation of the 2-methanesulfonyl-4-(1,2,4,5-tetrahydro-benzo[d]azepin-3-yl)-6-(2,2,2-trifluoro-ethoxy)-pyrimidine-5-carbonitrile

Oxidation of the 2-methylsulfanyl-4-(1,2,4,5-tetrahydro-benzo[d]azepin-3-yl)-6-(2,2,2-trifluoro-ethoxy)-pyrimidine-5-carbonitrile (example 166) with m-chloroperbenzoic acid in dichloromethane at room temperature yielded the 2-methanesulfonyl-4-(1,2,4,5-tetrahydro-benzo[d]azepin-3-yl)-6-(2,2,2-trifluoro-ethoxy)-pyrimidine-5-carbonitrile as colorless solid; m.p. 166-169 °C; MS: [M+H]⁺ = 427.

### Example 173

### 2-(3-Pyridin-2-yl-propoxy)-4-(1,2,4,5-tetrahydro-benzo[d]azepin-3-yl)-6-(2,2,2-trifluoro-ethoxy)-pyrimidine-5-carbonitrile

In analogy to the procedure described in example 47 a) the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 3-(3-pyridyl)-1-propanol in tetrahydrofuran in the presence of sodium hydride at room temperature to yield the 2-(3-pyridin-2-yl-propoxy)-4-(1,2,4,5-tetrahydro-benzo[d]azepin-3-yl)-6-(2,2,2-trifluoro-ethoxy)-pyrimidine-5-carbonitrile as colorless oil; MS: [M+H]⁺ = 484.

### Example 174

### 2-(3-Morpholin-4-yl-propylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 4-(3-aminopropyl)-morpholine in tetrahydrofuran at 50 °C to yield the 2-(3-morpholin-4-yl-propylamino)-4-(1,2,4,5-tetrahydro-benzo[d]azepin-3-yl)-6-(2,2,2-trifluoro-ethoxy)-pyrimidine-5-carbonitrile as colorless oil; MS: [M+H]⁺ = 491.

### Example 175

### 5-Methylsulfanyl-7-(1,2,4,5-tetrahydro-benzo[d]azepin-3-yl)-[1,2,4]triazolo[4,3-c]pyrimidine-8-carbonitrile

0.30 g (0.83 mmol) of 4-hydrazino-2-methylsulfanyl-6-(1,2,4,5-tetrahydro-benzo[d]azepin-3-yl)-pyrimidine-5-carbonitrile were suspended in 1.0 ml of triethyl orthoformiate and the reaction mixture was stirred at 110°C during 24 hours. Subsequently, it was evaporated under reduced pressure and the residue obtained was purified by chromatography on silica gel using a 95:5 v/v mixture of dichloromethane and methanol as eluent to yield 0.183 g (0.54 mmol), 66 %, of the 5-methylsulfanyl-7-(1,2,4,5-tetrahydro-benzo[d]azepin-3-yl)-[1,2,4]triazolo[4,3-c]pyrimidine-8-carbonitrile as light brown solid; m.p. >200 °C; MS: [M]⁺ = 336.

### Preparation of the 4-hydrazino-2-methylsulfanyl-6-(1,2,4,5-tetrahydro-benzo[d]azepin-3-yl)-pyrimidine-5-carbonitrile

The 4-chloro-2-methylsulfanyl-6-(1,2,4,5-tetrahydro-benzo[d]azepin-3-yl)-pyrimidine-5-carbonitrile (example 149) and hydrazine hydrate were heated in a mixture of dioxane and water at 100 °C to yield the 4-hydrazino-2-methylsulfanyl-6-(1,2,4,5-tetrahydro-benzo[d]azepin-3-yl)-pyrimidine-5-carbonitrile as orange solid; m.p.. >200 °C; MS: [M+H]⁺ = 327.

### Example 176

### 2-Methyl-4-(1,1,2,2-tetrafluoro-ethoxy)-6-(1,2,4,5-tetrahydro-benzo[d]azepin-3-yl)-pyrimidine-5-carbonitrile

3.0 ml of tetrafluoroethylene were condensed at -180 °C into a mixture of 0.20 g (0.71 mmol) of 2-methyl-6-oxo-4-(1,2,4,5-tetrahydro-benzo[d]azepin-3-yl)-1,6-dihydro-pyrimidine-5-carbonitrile (example 17) and 0.037 g (0.86 mmol) of sodium hydride dispersion (55% in mineral oil) in 2.0 ml of N,N-dimethylformamide and the reaction mixture was then heated in an autoclave at 120 °C for 16 hours. Subsequently, it was poured into 50 ml of an ice/1N HCl mixture and extracted three times with 50 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a hexane/ethyl acetate mixture as eluent to yield 0.025 g (0.066 mmol), 9.3 %, of 2-methyl-4-(1,1,2,2-tetrafluoro-ethoxy)-6-(1,2,4,5-tetrahydro-benzo[d]azepin-3-yl)-pyrimidine-5-carbonitrile as yellow amorphous solid; MS: [M+H]⁺ = 381.

### Example 177

### 1-Dimethylamino-2-methyl-6-oxo-4-(1,2,4,5-tetrahydro-benzo[d]azepin-3-yl)-1,6-dihydro-pyrimidine-5-carbonitrile

In analogy to the procedure described in example 17 the Z and/or E 2-cyano-3-methylsulfanyl-3-(1,2,4,5-tetrahydro-benzo[d]azepin-3-yl)-acrylic acid ethyl ester [example 13 a)] was treated with the acetamide dimethylhydrazone [J. Heterocycl. Chem. 18, 319 (1981)] and 1,8-diazabicyclo[5.4.0]undec-7-en in N,N-dimethylformamide at 100 °C to yield the 1-dimethylamino-2-methyl-6-oxo-4-(1,2,4,5-tetrahydro-benzo[d]azepin-3-yl)-1,6-dihydro-pyrimidine-5-carbonitrile as colorless solid; m.p. 134.4-136.3 °C; MS: [M]⁺ = 324.

### Example 178

### 4-Fluoro-2-methyl-6-(1,2,4,5-tetrahydro-benzo[d]azepin-3-yl)-pyrimidine-5-carbonitrile

3.0 ml of tetrafluoroethylene were condensed at -180 °C into a mixture of 0.40 g (1.42 mmol) of 2-methyl-6-oxo-4-(1,2,4,5-tetrahydro-benzo[d]azepin-3-yl)-1,6-dihydro-pyrimidine-5-carbonitrile (example 17) and 0.296 g (2.14 mmol) of potassium carbonate in 3.0 ml of N,N-dimethylformamide and the reaction mixture was then heated in an autoclave at 100 °C for 72 hours. Subsequently, it was poured into 50 ml of an ice/1N HCl mixture and extracted three times with 50 ml of dichloromethane. The combined organic phases were washed twice with 50 ml of water, dried over magnesium sulphate, evaporated under reduced pressure and dried in a high vacuum. The thus obtained crude product was purified by chromatography on silica gel using dichloromethane as eluent to yield 0.097 g (0.034 mmol), 2.4 %, of 4-fluoro-2-methyl-6-(1,2,4,5-tetrahydro-benzo[d]azepin-3-yl)-pyrimidine-5-carbonitrile as yellowish amorphous solid; MS: [M]⁺ = 282.

### Example 179

### 2-Cyclopropyl-4-(1,2,4,5-tetrahydro-benzo[d]azepin-3-yl)-6-(2,2,2-trifluoro-ethoxy)-pyrimidine-5-carbonitrile

From the 2-cyclopropyl-6-oxo-4-(1,2,4,5-tetrahydro-benzo[d]azepin-3-yl)-1,6-dihydro-pyrimidine-5-carbonitrile (example 18) and 2,2,2-trifluoroethyl trifluoromethanesulfonate in N,N-dimethylformamide in the presence of potassium carbonate as colorless solid; m.p. 116.5-118 °C; MS: [M+H]⁺ = 389; see example 180.

### Example 180

### 2-Cyclopropyl-6-oxo-4-(1,2,4,5-tetrahydro-benzo[d]azepin-3-yl)-1-(2,2,2-trifluoro-ethyl)-1,6-dihydro-pyrimidine-5-carbonitrile

In analogy to the procedure described in example 3 the 2-cyclopropyl-6-oxo-4-(1,2,4,5-tetrahydro-benzo[d]azepin-3-yl)-1,6-dihydro-pyrimidine-5-carbonitrile (example 18) was treated with the 2,2,2-trifluoroethyl trifluoromethanesulfonate in N,N-dimethylformamide in the presence of potassium carbonate to yield the 2-cyclopropyl-6-oxo-4-(1,2,4,5-tetrahydro-benzo[d]azepin-3-yl)-1-(2,2,2-trifluoro-ethyl)-1,6-dihydro-pyrimidine-5-carbonitrile as colorless solid; m.p. 176-179.5 °C; MS: [M+H]⁺ = 389; and the 2-cyclopropyl-4-(1,2,4,5-tetrahydro-benzo[d]azepin-3-yl)-6-(2,2,2-trifluoro-ethoxy)-pyrimidine-5-carbonitrile as colorless solid; m.p. 116.5-118 °C; MS: [M+H]⁺ = 389.

### Example 181

### 2-(2-Morpholin-4-yl-ethylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 2-morpholin-4-yl-ethylamine in tetrahydrofuran at 80 °C to yield the 2-(2-morpholin-4-yl-ethylamino)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 477.

### Example 182

### 2-Oxo-4-(1,2,4,5-tetrahydro-benzo[d]azepin-3-yl)-6-(2,2,2-trifluoro-ethoxy)-1,2-dihydro-pyrimidine-5-carbonitrile

0.050 g (0.12 mmol) of the 2-methanesulfonyl-4-(1,2,4,5-tetrahydro-benzo[d]azepin-3-yl)-6-(2,2,2-trifluoro-ethoxy)-pyrimidine-5-carbonitrile (example 172) were dissolved in 3.0 ml of dioxane, treated with 0.04 ml of 6M aqueous potassium hydroxide solution and the reaction mixture was stirred at 80 °C during 16 hours. Subsequently, it was evaporated under reduced pressure, 50 ml of water were added and the mixture extracted three times with 50 ml of dichloromethane. The combined organic phases were washed twice with 50 ml of water, dried over magnesium sulphate, evaporated under reduced pressure and dried in a high vacuum. The residue obtained was crystallized from dichlomethane to yield 0.030 g (0.082 mmol), 69 %, of the 2-oxo-4-(1,2,4,5-tetrahydro-benzo[d]azepin-3-yl)-6-(2,2,2-trifluoro-ethoxy)-1,2-dihydro-pyrimidine-5-carbonitrile as colorless solid; m.p. 196-198 °C; MS: [M]⁺ = 365.

### Example 183

### 6-Oxo-2-propyl-4-(1,2,4,5-tetrahydro-benzo[d]azepin-3-yl)-1-(2,2,2-trifluoro-ethyl)-1,6-dihydro-pyrimidine-5-carbonitrile

From the 6-oxo-2-propyl-4-(1,2,4,5-tetrahydro-benzo[d]azepin-3-yl)-1,6-dihydro-pyrimidine-5-carbonitrile (example 23) and 2,2,2-trifluoroethyl trifluoromethanesulfonate in N,N-dimethylformamide in the presence of potassium carbonate as colorless solid; m.p. 165-167 °C; MS: [M+H]⁺ = 391; see example 184.

### Example 184

### 2-Propyl-4-(1,2,4,5-tetrahydro-benzo[d]azepin-3-yl)-6-(2,2,2-trifluoro-ethoxy)-pyrimidine-5-carbonitrile

In analogy to the procedure described in example 3 the 6-oxo-2-propyl-4-(1,2,4,5-tetrahydro-benzo[d]azepin-3-yl)-1,6-dihydro-pyrimidine-5-carbonitrile (example 23) was treated with the 2,2,2-trifluoroethyl trifluoromethanesulfonate in N,N-dimethylformamide in the presence of potassium carbonate at room temperature to yield the 6-oxo-2-propyl-4-(1,2,4,5-tetrahydro-benzo[d]azepin-3-yl)-1-(2,2,2-trifluoro-ethyl)-1,6-dihydro-pyrimidine-5-carbonitrile as colorless solid; m.p. 165-167 °C; MS: [M+H]⁺ = 391; and the 2-propyl-4-(1,2,4,5-tetrahydro-benzo[d]azepin-3-yl)-6-(2,2,2-trifluoro-ethoxy)-pyrimidine-5-carbonitrile as colorless solid; m.p. 104-106 °C; MS: [M+H]⁺ = 391.

### Example 185

### [rac]-Acetic acid 3-(1-ethyl-2-methyl-5-nitro-6-oxo-1,6-dihydro-pyrimidin-4-yl)-2,3,4,5-tetrahydro-1H-benzo[d]azepin-1-yl ester

Treatment of the [rac]-3-ethyl-6-(1-hydroxy-1,2,4,5-tetrahydro-benzo[d]azepin-3-yl)-2-methyl-5-nitro-3H-pyrimidin-4-one (example 170) with acetic anhydride and trietylamine in dichloromethane at room temperature yielded the [rac]-acetic acid 3-(1-ethyl-2-methyl-5-nitro-6-oxo-1,6-dihydro-pyrimidin-4-yl)-2,3,4,5-tetrahydro-1H-benzo[d]azepin-1-yl ester as light yellow foam; MS: [M+H]⁺ = 387.

### Example 186

### 3-(3-Hydroxy-propyl)-2-methyl-5-nitro-6-(1,2,4,5-tetrahydro-benzo[d]azepin-3-yl)-3H-pyrimidin-4-one

From the 2-methyl-5-nitro-6-(1,2,4,5-tetrahydro-benzo[d]azepin-3-yl)-pyrimidin-4-ol (example 1) and 3-chloro-1-propanol, potassium iodide, potassium carbonate in N,N-dimethylformamide at 50 °C as yellow oil; MS: [M+H]⁺ = 359; see example 187.

### Example 187

### 3-[2-Methyl-5-nitro-6-(1,2,4,5-tetrahydro-benzo[d]azepin-3-yl)-pyrimidin-4-yloxy]-propan-1-ol

In analogy to the procedure described in example 3 the 2-methyl-5-nitro-6-(1,2,4,5-tetrahydro-benzo[d]azepin-3-yl)-pyrimidin-4-ol (example 1) was treated with 3-chloro-1-propanol, potassium iodide and potassium carbonate in N,N-dimethylformamide at 50 °C to yield the 3-[2-methyl-5-nitro-6-(1,2,4,5-tetrahydro-benzo[d]azepin-3-yl)-pyrimidin-4-yloxy]-propan-1-ol as yellow oil; MS: [M+H]⁺ = 359; and the 3-(3-hydroxy-propyl)-2-methyl-5-nitro-6-(1,2,4,5-tetrahydro-benzo[d]azepin-3-yl)-3H-pyrimidin-4-one as yellow oil; MS: [M+H]⁺ = 359.

### Example 188

### 2-(2-Morpholin-4-yl-ethoxy)-4-(2-morpholin-4-yl-ethylamino)-6-(1,2,4,5-tetrahydro-benzo[d]azepin-3-yl)-pyrimidine-5-carbonitrile

In analogy to the procedure described in example 48 the 4-chloro-2-(2-morpholin-4-yl-ethoxy)-6-(1,2,4,5-tetrahydro-benzo[d]azepin-3-yl)-pyrimidine-5-carbonitrile was treated with the 4-(2-aminoethyl)-morpholine in dioxane at 80 °C during 15 hours to yield the 2-(2-morpholin-4-yl-ethoxy)-4-(2-morpholin-4-yl-ethylamino)-6-(1,2,4,5-tetrahydro-benzo[d]azepin-3-yl)-pyrimidine-5-carbonitrile as a orange amorphous solid; MS: [M+H]⁺ = 508.

### Preparation of the 4-chloro-2-(2-morpholin-4-yl-ethoxy)-6-(1,2,4,5-tetrahydro-benzo[d]azepin-3-yl)-pyrimidine-5-carbonitrile

In analogy to the procedure described in example 47 a) the 4-chloro-2-methanesulfonyl-6-(1,2,4,5-tetrahydro-benzo[d]azepin-3-yl)-pyrimidine-5-carbonitrile (example 171) was treated with 4-(2-hydroxyethyl)-morpholine in tetrahydrofuran in the presence of sodium hydride at 40 °C to yield the 4-chloro-2-(2-morpholin-4-yl-ethoxy)-6-(1,2,4,5-tetrahydro-benzo[d]azepin-3-yl)-pyrimidine-5-carbonitrile as a colorless amorphous solid; MS: [M+H]⁺ = 414.

### Example 189

### 2-[2-(Pyridin-2-yloxy)-ethylamino]-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 2-(pyridin-2-yloxy)-ethylamine in dioxan at 80 °C to yield the 2-[2-(pyridin-2-yloxy)-ethylamino]-4-(1,2,4,5-tetrahydro-benzo[d]azepin-3-yl)-6-(2,2,2-trifluoro-ethoxy)-pyrimidine-5-carbonitrile as amorphous colorless solid; MS: [M+H]⁺ = 485.

### Example 190

### 2-(2-Hydroxy-ethylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the ethanolamine in dioxan at 80 °C to yield the 2-(2-hydroxy-ethylamino)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 408.

### Example 191

### (3-Imidazol-1-yl-propylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 1-(3-aminopropyl)-imidazole in dioxan at 80 °C to yield the (3-imidazol-1-yl-propylamino)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 472.

### Example 192

### 5-Methylsulfanyl-7-(1,2,4,5-tetrahydro-benzo[d]azepin-3-yl)-imidazo[1,2-c]pyrimidine-8-carbonitrile

A supension of 0.120 g (0.27 mmol) of the 4-amino-2-methylsulfanyl-6-(1,2,4,5-tetrahydro-benzo[d]azepin-3-yl)-pyrimidine-5-carbonitrile (example 164) in 0.390 ml (2.7 mmol) of chloroacetaldehyde solution (45% in water) was stirred at 80 °C for 2 hours. The reaction mixture was then evaporated under reduced pressure, suspended in an ether and hexane mixture and the not soluble crystals filtered off. The residue obtained was further purified by chromatography on silica gel using a 95:5 v/v mixture of dichloromethane and methanol as eluent to yield 0.065 g (0.194 mmol), 72.3 %, of the 5-methylsulfanyl-7-(1,2,4,5-tetrahydro-benzo[d]azepin-3-yl)-imidazo[1,2-c]pyrimidine-8-carbonitrile as amorphous light brown solid; MS: [M+H]⁺ = 336.

### Example 193

### 3-[2-Methyl-5-nitro-6-(3-phenyl-propoxy)-pyrimidin-4-yl]-2,3,4,5-tetrahydro-1H-benzo[d]azepine

From the 2-methyl-5-nitro-6-(1,2,4,5-tetrahydro-benzo[d]azepin-3-yl)-pyrimidin-4-ol (example 1) with the 3-chloro-1-phenyl-propane in N,N-dimethylformamide in the presence of potassium carbonate at 80 °C as light yellow oil; MS: [M+H]⁺ = 419; see example 194.

### Example 194

### 2-Methyl-5-nitro-3-(3-phenyl-propyl)-6-(1,2,4,5-tetrahydro-benzo[d]azepin-3-yl)-3H-pyrimidin-4-one

In analogy to the procedure described in example 3 the 2-methyl-5-nitro-6-( 1,2,4,5-tetrahydro-benzo[d]azepin-3-yl)-pyrimidin-4-ol (example 1) was treated with the 3-chloro-1-phenyl-propane in N,N-dimethylformamide in the presence of potassium carbonate 80 °C to yield the 2-methyl-5-nitro-3-(3-phenyl-propyl)-6-(1,2,4,5-tetrahydro-benzo[d]azepin-3-yl)-3H-pyrimidin-4-one as yellow oil; MS: [M+H]⁺ = 419; and the 3-[2-methyl-5-nitro-6-(3-phenyl-propoxy)-pyrimidin-4-yl]-2,3,4,5-tetrahydro-1H-benzo[d]azepine as light yellow oil; MS: [M+H]⁺ = 419.

### Example 195

### (2-Pyrrolidin-1-yl-ethylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the N-(2-aminoethyl)-pyrrolidine in dioxan at 80 °C to yield the (2-pyrrolidin-1-yl-ethylamino)-4-(1,2,4,5-tetrahydro-benzo[d]azepin-3-yl)-6-(2,2,2-trifluoro-ethoxy)-pyrimidine-5-carbonitrile as light yellow amorphous solid; MS: [M+H]⁺ = 461.

### Example 196

### 2-Methyl-5-nitro-3-(3-pyridin-3-yl-propyl)-6-(1,2,4,5-tetrahydro-benzo[d]azepin-3-yl)-3H-pyrimidin-4-one

From the 2-methyl-5-nitro-6-(1,2,4,5-tetrahydro-benzo[d]azepin-3-yl)-pyrimidin-4-ol (example 1) with the toluene-4-sulfonic acid 3-(3-pyridyl)-propyl ester in N,N-dimethylformamide in the presence of potassium carbonate at 120 °C as yellow oil; MS: [M+H]⁺ = 420; see example 197.

### Example 197

### 3-[2-Methyl-5-nitro-6-(3-pyridin-3-yl-propoxy)-pyrimidin-4-yl]-2,3,4,5-tetrahydro-1H-benzo[d]azepine

In analogy to the procedure described in example 3 the 2-methyl-5-nitro-6-( 1,2,4,5-tetrahydro-benzo[d]azepin-3-yl)-pyrimidin-4-ol (example 1) was treated with the toluene-4-sulfonic acid 3-(3-pyridyl)-propyl ester in N,N-dimethylformamide in the presence of potassium carbonate at 120 °C to yield the 3-[2-methyl-5-nitro-6-(3-pyridin-3-yl-propoxy)-pyrimidin-4-yl)-2,3,4,5-tetrahydro-1H-benzo[d]azepine as yellow oil; MS: [M+H]⁺ = 420; and the 2-methyl-5-nitro-3-(3-pyridin-3-yl-propyl)-6-(1,2,4,5-tetrahydro-benzo[d]azepin-3-yl)-3H-pyrimidin-4-one as yellow oil; MS: [M+H]⁺ = 420.

### Example 198

### 3-(6-Chloro-2-methyl-5-nitro-pyrimidin-4-yl)-2,3,4,5-tetrahydro-1H-benzo[d]azepine

0.266 g (1.25 mmol) of phosphorus pentachloride were added to a suspension of 0.30 g (1.0 mmol) of the 2-methyl-5-nitro-6-(1,2,4,5-tetrahydro-benzo[d]azepin-3-yl)-pyrimidin-4-ol (example 1) in 0.66 g (5.0 mmol) of N-ethyldiisopropylamine under argon at 0° C. Then, 0.63 g (4.0 mmol) of phosphorus oxychloride were added and the reaction mixture stirred at 100 °C for 3 h. It was then poured into 50 ml of an ice/water mixture and extracted three times with 100 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 1:1 v/v mixture of hexane and ethyl acetate as eluent to yield 0.161 g (0.505 mmol), 50.5 %, of the 3-(6-chloro-2-methyl-5-nitro-pyrimidin-4-yl)-2,3,4,5-tetrahydro-1H-benzo[d]azepine as light brown solid; m.p. 161-165 °C; MS: [M+H]⁺ = 319.

### Example 199

### 1-Hydroxy-2-methyl-6-oxo-4-(1,2,4,5-tetrahydro-benzo[d]azepin-3-yl)-1,6-dihydro-pyrimidine-5-carbonitrile

A suspension of 0.30 g (1.07 mmol) of the 2-methyl-6-oxo-4-(1,2,4,5-tetrahydro-benzo[d]azepin-3-yl)-1,6-dihydro-pyrimidine-5-carbonitrile (example 17) in 10 ml of hexamethyldisilazane and 3.0 ml of trimethylchlorosilane was heated at reflux for 3 hours.

The solution formed was then evaporated under reduced pressure. The residue was dissolved in 10 ml of dichloromethane and treated with 0.93 g (2.14 mmol) of the oxodiperoxymolybdenum (pyridine) (HMPA) complex [J. Org. Chem. 43 (1978),188-196] and the reaction mixture was stirred at room temperature for 20 hours. Then, 0.63 g (2.14 mmol) of ethylenediamine-tetraacetic acid and 8.4 ml of 1N sodium hydroxide solution were added and stirring continued for 30 minutes. The aqueous phase was then adjusted to pH 7 with 1N hydrogen chloride solution, and the reaction mixture extracted three times with 50 ml of dichloromethane. The combined organic phases were washed twice with 50 ml of water, dried over magnesium sulphate, evaporated under reduced pressure and dried in a high vacuum. The thus obtained crude product was crystallized from ether to yield 0.115 g (0.388 mmol), 36.3 %, of the 1-hydroxy-2-methyl-6-oxo-4-(1,2,4,5-tetrahydro-benzo[d]azepin-3-yl)-1,6-dihydro-pyrimidine-5-carbonitrile as yellowish solid; m.p. 168.5-172 °C; MS: [M+H]⁺ = 297.

### Example 200

### 1-Amino-2-methyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with the O-mesitylenesulfonylhydroxylamine [Synthesis 1972, 140] in N,N-dimethylformamide in the presence of potassium carbonate at room temperature to yield the 1-amino-2-methyl-6-oxo-4-(1,2,4,5-tetrahydro-benzo[d]azepin-3-yl)-1,6-dihydro-pyrimidine-5-carbonitrile as colorless solid; m.p. 137-142 °C; MS: [M+H]⁺ = 296.

### Example 201

### 2-(4-Ethyl-piperazin-1-yl)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the ethyl-piperazine in dioxan at 80 °C to yield the 2-(4-ethyl-piperazin-1-yl)-4-(1,2,4,5-tetrahydro-benzo[d] azepin-3-yl)-6-(2,2,2-trifluoro-ethoxy)-pyrimidine-5-carbonitrile as light yellow amorphous solid; MS: [M+H]⁺ = 461.

### Example 202

### 3-(3-Imidazol-1-yl-propyl)-2-methyl-5-nitro-6-(1,2,4,5-tetrahydro-benzo[d]azepin-3-yl)-3H-pyrimidin-4-one

A solution of 0.437 g (1.0 mmol) of the methanesulfonic acid 3-[2-methyl-5-nitro-6-oxo-4-(1,2,4,5-tetrahydro-benzo[d]azepin-3-yl)-6H-pyrimidin-1-yl]-propyl ester [prepared from the 3-(3-hydroxy-propyl)-2-methyl-5-nitro-6-(1,2,4,5-tetrahydro-benzo[d]azepin-3-yl)-3H-pyrimidin-4-one (example 186) and methanesulfonyl chloride/triethylamine in dichloromethane, -70 °C to room temperature], 0.103 g (1.5 mmol) of imidiazole and 0.396 g (3.0 mmol) of N-ethyl-diisopropylamine in 10.0 ml N,N-dimethylformamide was stirred at 80 °C for 16 hours. The reaction mixture was then poured into 50 ml of an ice/water mixture and extracted three times with 60 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 10:0 to 9:1 v/v mixture of dichloromethane and methanol as eluent to yield 0.134 g (0.328 mmol), 33%, 3-(3-imidazol-1-yl-propyl)-2-methyl-5-nitro-6-(1,2,4,5-tetrahydro-benzo[d]azepin-3-yl)-3H-pyrimidin-4-one as light yellow oil; MS: [M-H]⁻ = 407.

### Example 203

### 2-Methyl-3-(3-morpholin-4-yl-prophl)-5-nitro-6-(1,2,4,5-tetrahydro-benzo[d]azepin-3-yl)-3H-pyrimidin-4-one

In analogy to the procedure described in example 202 the methanesulfonic acid 3-[2-methyl-5-nitro-6-oxo-4-(1,2,4,5-tetrahydro-benzo[d]azepin-3-yl)-6H-pyrimidin-1-yl]-propyl ester (example 202) was treated with the morpholine (excess) at room temperature to yield the 2-methyl-3-(3-morpholin-4-yl-propyl)-5-nitro-6-(1,2,4,5-tetrahydro-benzo[d]azepin-3-yl)-3H-pyrimidin-4-one as light yellow amorphous solid; MS: [M+H]⁺ = 428.

### Example 204

### 2-Methyl-5-nitro-6-(1,2,4,5-tetrahydro-benzo[d]azepin-3-yl)-3-(3-[1,2,4]triazol-1-yl-propyl)-3H-pyrimidin-4-one

In analogy to the procedure described in example 202 the methanesulfonic acid 3-[2-methyl-5-nitro-6-oxo-4-( 1,2,4,5-tetrahydro-benzo[d]azepin-3-yl)-6H-pyrimidin-1-yl]-propyl ester (example 202) was treated with the 1,2,4-triazole and sodium hydride in N,N-dimethylformamide at room temperature to yield the 2-methyl-5-nitro-6-(1,2,4,5-tetrahydro-benzo[d]azepin-3-yl)-3-(3-[1,2,4]triazol-1-yl-propyl)-3H-pyrimidin-4-one as light yellow amorphous solid; MS: [M+H]⁺ = 410.

### Example 205

### 3-[3-(2-(R)-Hydroxymethyl-pyrrolidin-1-yl)-propyl]-2-methyl-5-nitro-6-(1,2,4,5-tetrahydro-benzo[d]azepin-3-yl)-3H-pyrimidin-4-one

In analogy to the procedure described in example 202 the methanesulfonic acid 3-[2-methyl-5-nitro-6-oxo-4-(1,2,4,5-tetrahydro-benzo[d]azepin-3-yl)-6H-pyrimidin-1-yl]-propyl ester (example 202) was treated with the (R)-(-)-2-hydroxymethyl-pyrrolidine and N-ethyl-diisopropylamine in tetrahydrofuran at reflux to yield the 3-[3-(2-(R)-hydroxymethyl-pyrrolidin-1-yl)-propyl]-2-methyl-5-nitro-6-(1,2,4,5-tetrahydro-benzo[d]azepin-3-yl)-3H-pyrimidin-4-one as light yellow oil; MS: [M+H]⁺ = 442.

### Example 206

### 3-[2-Methyl-5-nitro-6-(3-[1,2,4]triazol-1-yl-propoxy)-pyrimidin-4-yl]-2,3,4,5-tetrahydro-1H-benzo[d]azepine

In analogy to the procedure described in example 202 the methanesulfonic acid 3-[2-methyl-5-nitro-6-(1,2,4,5-tetrahydro-benzo[d]azepin-3-yl)-pyrimidin-4-yloxy]-propyl ester [prepared from 3-[2-methyl-5-nitro-6-(1,2,4,5-tetrahydro-benzo[d]azepin-3-yl)-pyrimidin-4-yloxy]-propan-1-ol (example 187) and methanesulfonyl chloride/triethylamine in dichloromethane, -70 °C to room temperature] was treated with the 1,2,4-triazole and N-ethyl-diisopropylamine in N,N-dimethylformamide at 50 °C to yield the 3-[2-methyl-5-nitro-6-(3-[1,2,4]triazol-1-yl-propoxy)-pyrimidin-4-yl]-2,3,4,5-tetrahydro-1H-benzo[d]azepine as light yellow oil; MS: [M-H]⁻ = 408.

### Example 207

### 3-[6-(3-Imidazol-1-yl-propoxy)-2-methyl-5-nitro-pyrimidin-4-yl]-2,3,4,5-tetrahydro-1H-benzo[d]azepine

In analogy to the procedure described in example 202 the methanesulfonic acid 3-[2-methyl-5-nitro-6-(1,2,4,5-tetrahydro-benzo[d]azepin-3-yl)-pyrimidin-4-yloxy]-propyl ester (example 206) was treated with the imidazole and N-ethyl-diisopropylamine in N,N-dimethylformamide at 50 °C to yield the 3-[6-(3-imidazol-1-yl-propoxy)-2-methyl-5-nitro-pyrimidin-4-yl]-2,3,4,5-tetrahydro-1H-benzo[d]azepine as light yellow amorphous solid; MS: [M+H]⁺ = 409.

### Example 208

### 3-[2-Methyl-5-nitro-6-(3-pyridin-2-yl-propoxy)-pyrimidin-4-yl]-2,3,4,5-tetrahydro-1H-benzo[d]azepine

In analogy to the procedure described in example 47 a) the 3-(6-chloro-2-methyl-5-nitro-pyrimidin-4-yl)-2,3,4,5-tetrahydro-1H-benzo[d]azepine (example 198) was treated with the 3-pyridin-2-yl-propanol in N,N-dimethylformamide in the presence of sodium hydride at 50 °C to yield the 3-[2-methyl-5-nitro-6-(3-pyridin-2-yl-propoxy)-pyrimidin-4-yl]-2,3,4,5-tetrahydro-1H-benzo[d]azepine as light brown oil; MS: [M+H]⁺ = 420.

### Example 209

### 3-[2-Methyl-5-nitro-6-(3-pyridin-4-yl-propoxy)-pyrimidin-4-yl]-2,3,4,5-tetrahydro-1H-benzo[d]azepine

In analogy to the procedure described in example 47 a) the 3-(6-chloro-2-methyl-5-nitro-pyrimidin-4-yl)-2,3,4,5-tetrahydro-1H-benzo[d]azepine (example 198) was treated with the 3-pyridin-4-yl-propanol in N,N-dimethylformamide in the presence of sodium hydride at room temperature to yield the 3-[2-methyl-5-nitro-6-(3-pyridin-4-yl-propoxy)-pyrimidin-4-yl]-2,3,4,5-tetrahydro-1H-benzo[d]azepine as light brown oil; MS: [M+H]⁺ = 420.

### Example 210

### [2-Methyl-5-nitro-6-(1,2,4,5-tetrahydro-benzo[d]azepin-3-yl)-pyrimidin-4-yl]-(3-morpholin-4-yl-propyl)-amine

In analogy to the procedure described in example 48 the 3-(6-chloro-2-methyl-5-nitro-pyrimidan-4-yl)-2,3,4,5-tetrahydro-1H-benzo[d]azepine (example 198) was treated with the 4-(3-aminopropyl)-morpholine (excess) at room temperature to yield the [2-methyl-5-nitro-6-(1,2,4,5-tetrahydro-benzo[d]azepin-3-yl)-pyrimidin-4-yl]-(3-morpholin-4-yl-propyl)-amine as yellow oil; MS: [M+H]⁺ = 427.

### Example 211

### (3-Imidazol-1-yl-propyl)-[2-methyl-5-nitro-6-(1,2,4,5-tetrahydro-benzo[d]azepin-3-yl)-pyrimidin-4-yl]-amine

In analogy to the procedure described in example 48 the 3-(6-chloro-2-methyl-5-nitro-pyrimidin-4-yl)-2,3,4,5-tetrahydro-1H-benzo[d]azepine (example 198) was treated with the 1-(3-aminopropyl)-imidazole (excess) at room temperature to yield the (3-imidazol-1-yl-propyl)-[2-methyl-5-nitro-6-(1,2,4,5-tetrahydro-benzo[d]azepin-3-yl)-pyrimidin-4-yl]-amine as yellow oil; MS: [M+H]⁺ = 408.

### Example 212

### 4-[2-Methyl-5-nitro-6-(1,2,4,5-tetrahydro-benzo[d]azepin-3-yl)-pyrimidin-4-ylamino]-butan-1-ol

In analogy to the procedure described in example 48 the 3-(6-chloro-2-methyl-5-nitro-pyrimidin-4-yl)-2,3,4,5-tetrahydro-1H-benzo[d]azepine (example 198) was treated with the 4-amino-1-butanol (excess) at room temperature to yield the 4-[2-methyl-5-nitro-6-(1,2,4,5-tetrahydro-benzo[d]azepin-3-yl)-pyrimidin-4-ylamino]-butan-1-ol as light yellow oil; MS: [M-H]⁻ = 370.

### Example 213

### 3-[2-Methyl-5-nitro-6-(1,2,4,5-tetrahydro-benzo[d]azepin-3-yl)-pyrimidin-4-ylamino]-propan-1-ol

In analogy to the procedure described in example 48 the 3-(6-chloro-2-methyl-5-nitro-pyrimidin-4-yl)-2,3,4,5-tetrahydro-1H-benzo[d]azepine (example 198) was treated with the 3-amino-1-propanol (excess) at room temperature to yield the 3-[2-methyl-5-nitro-6-(1,2,4,5-tetrahydro-benzo[d]azepin-3-yl)-pyrimidin-4-ylamino]-propan-1-ol as light yellow solid; m.p. 104-108 °C; MS: [M-H]⁻ = 356.

### Example 214

### 2-Methyl-1-methylamino-6-oxo-4-(1,2,4,5-tetrahydro-benzo[d]azepin-3-yl)-1,6-dihydro-pyrimidine-5-carbonitrile

From the 1-amino-2-methyl-6-oxo-4-(1,2,4,5-tetrahydro-benzo[d]azepin-3-yl)-1,6-dihydro-pyrimidine-5-carbonitrile (example 200) and methyl iodide, potassium carbonate in N,N-dimethylformamide as colorless solid; m.p. > 200 °C; MS: [M+H]⁺ = 310; see example 215.

### Example 215

### [5-Cyano-2-methyl-6-oxo-4-(1,2,4,5-tetrahydro-benzo[d]azepin-3-yl)-6H-pyrimidin-1-yl]-methyl-carbamic acid methyl ester

A solution of 0.110 g (0.37 mmol) of the 1-amino-2-methyl-6-oxo-4-(1,2,4,5-tetrahydro-benzo[d]azepin-3-yl)-1,6-dihydro-pyrimidine-5-carbonitrile (example 200), 0.112 ml (4.8 mmol) of methyl iodide and 0.162 g (1.17 mmol) of potassium carbonate in 1.0 ml of N,N-dimethylformamide was stirred at room temperature for 60 hours. The reaction mixture was then poured into 25 ml of an ice/water mixture and extracted three times with 50 ml of ethyl acetate. The combined organic phases were washed twice with 50 ml of water, dried over magnesium sulphate, evaporated under reduced pressure and dried in a high vacuum. The thus obtained crude product was purified by chromatography on silica gel using a 1:1 v/v mixture of hexane and ethyl acetate as eluent to yield 0.034 g (0.094 mmol), 25%, of the [5-cyano-2-methyl-6-oxo-4-(1,2,4,5-tetrahydro-benzo[d]azepin-3-yl)-6H-pyrimidin-1-yl]-methyl-carbamic acid methyl ester as colorless solid; m.p. 120-122.5 °C; MS: [M+H]⁺ = 368; and 0.008 g (0.026 mmol), 7%, of the 2-methyl-1-methylamino-6-oxo-4-(1,2,4,5-tetrahydro-benzo[d]azepin-3-yl)-1,6-dihydro-pyrimidine-5-carbonitrile as colorless solid; m.p. > 200 °C; MS: [M+H]⁺ = 310.

### Example 216

### [5-Cyano-2-methyl-6-oxo-4-(1,2,4,5-tetrahydro-benzo[d]azepin-3-yl)-6H-pyrimidin-1-yl]-ethyl-carbamic acid ethyl ester

In analogy to the procedure described in example 215 the 1-amino-2-methyl-6-oxo-4-(1,2,4,5-tetrahydro-benzo[d]azepin-3-yl)-1,6-dihydro-pyrimidine-5-carbonitrile (example 200) was treated with the iodoethane in N,N-dimethylformamide in the presence of potassium carbonate at room temperature to yield the [5-cyano-2-methyl-6-oxo-4-(1,2,4,5-tetrahydro-benzo[d]azepin-3-yl)-6H-pyrimidin-1-yl]-ethyl-carbamic acid ethyl ester as colorless amorphous solid; MS: [M+H]⁺ = 396.

### Example 217

### [5-Cyano-2-methyl-6-oxo-4-(1,2,4,5-tetrahydro-benzo[d]azepin-3-yl)-6H-pyrimidin-1-yl]-dicarbamic acid tert-butylester

4.2 mg (0.034 mmol) of 4-dimethylamino pyridine were added to a solution of 0.100 g (0.34 mmol) of the 1-amino-2-methyl-6-oxo-4-(1,2,4,5-tetrahydro-benzo[d]azepin-3-yl)-1,6-dihydro-pyrimidine-5-carbonitrite (example 200) and 0.141 mg (0.65 mmol) of di-tert-butyldicarbonate in 1.5 ml of dichloromethane and the reaction mixture was stirred at room temperature for 16 hours. It was then evaporated under reduced pressure and dried in a high vacuum. The thus obtained crude product was purified by chromatography on silica gel using a 9:1 v/v mixture of dichloromethane and diethyl ether as eluent to yield 0.074 g (0.015 mmol), 44%, of the [5-cyano-2-methyl-6-oxo-4-(1,2,4,5-tetrahydro-benzo[d]azepin-3-yl)-6H-pyrimidin-1-yl]-dicarbamic acid tert-butylester as colorless solid; m.p. 148-150 °C; MS: [M+H]⁺ = 496.

### Example 218

### [5-Cyano-2-methyl-6-oxo-4-(1,2,4,5-tetrahydro-benzo[d]azepin-3-yl)-6H-pyrimidin-1-yl]-carbamic acid tert-butyl ester

670 mg (1.35 mmol) of the [5-cyano-2-methyl-6-oxo-4-(1,2,4,5-tetrahydro-benzo[d]azepin-3-yl)-6H-pyrimidin-1-yl]-dicarbamic acid tert-butylester (example 217) and 3.5 g silica gel were suspended in 10 ml of dichloromethane and slurry stirred for 16 hours. It was then transferred on a silica gel chromatography column and the desired product eluted with a 9:1 v/v mixture of dichloromethane and diethyl ether yielding 0.395 g (1.0 mmol), 74%, of the [5-cyano-2-methyl-6-oxo-4-(1,2,4,5-tetrahydro-benzo[d]azepin-3-yl)-6H-pyrimidin-1-yl]-carbamic acid tert-butyl ester as colorless solid; m.p. 150-152 °C; MS: [M+H]⁺ = 396.

### Example 219

### 2-(2-Pyridin-3-yl-ethylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 2-pyridin-3-yl-ethylamine in dioxan at 80 °C to yield the 2-(2-pyridin-3-yl-ethylamino)-4-(1,2,4,5-tetrahydro-benzo[d]azepin-3-yl)-6-(2,2,2-trifluoro-ethoxy)-pyrimidine-5-carbonitrile as light yellow amorphous solid; MS: [M+H]⁺ = 469.

### Example 220

### 2-Methyl-6-oxo-4-(1,2,4,5-tetrahydro-benzo[d]azepin-3-yl)-1-(2,2,2-trifluoro-ethoxy)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with the 2,2,2-trifluoroethyl trifluoromethanesulfonate in N,N-dimethylformamide in the presence of potassium carbonate to yield the 2-methyl-6-oxo-4-(1,2,4,5-tetrahydro-benzo[d]azepin-3-yl)-1-(2,2,2-trifluoro-ethoxy)-1,6-dihydro-pyrimidine-5-carbonitrile as colorless solid; m.p. >200 °C; MS: [M+H]⁺, = 379.

### Example 221

### 1-{3-[2-Methyl-5-nitro-6-(1,2,4,5-tetrahydro-benzo[d]azepin-3-yl)-pyrimidin-4-ylamino]-propyl}-pyrrolidin-2-one

In analogy to the procedure described in example 48 the 3-(6-chloro-2-methyl-5-nitro-pyrimidin-4-yl)-2,3,4,5-tetrahydro-1H-benzo[d]azepine (example 198) was treated with the 1-(3-aminopropyl)-2-pyrrolidinone in tetrahydrofuran at room temperature to yield the 1-{3-[2-methyl-5-nitro-6-(1,2,4,5-tetrahydro-benzo[d]azepin-3-yl)-pyrimidin-4-ylamino]-propyl}-pyrrolidin-2-one as light yellow oil; MS: [M+H]⁺ = 425.

### Example 222

### 3-[3-(3-(R)-Hydroxy-pyrrolidin-1-yl)-propyl]-2-methyl-5-nitro-6-(1,2,4,5-tetrahydro-benzo[d]azepin-3-yl)-3H-pyrimidin-4-one

In analogy to the procedure described in example 202 the methanesulfonic acid 3-[2-methyl-5-nitro-6-oxo-4-(1,2,4,5-tetrahydro-benzo[d]azepin-3-yl)-6H-pyrimidin-1-yl]-propyl ester (example 202) was treated with the (R)-3-hydroxypyrrolidine and N-ethyldiisopropyl amine in tetrahydrofuran between room temperature and reflux to yield the 3-[3-(3-(R)-hydroxy-pyrrolidin-1-yl)-propyl]-2-methyl-5-nitro-6-(1,2,4,5-tetrahydro-benzo[d]azepin-3-yl)-3H-pyrimidin-4-one as light yellow oil; MS: [M+H]⁺ = 428.

### Example 223

### N'-[5-Cyano-2-methyl-6-oxo-4-(1,2,4,5-tetrahydro-benzo[d]azepin-3-yl)-6H-pyrimidin-1-yl]-hydrazinecarboxylic acid tert-butyl ester

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with a mixture of the O-mesitylenesulfonylhydroxylamine [Synthesis 1972, 140] and the t-butyl N-mesitylenesulfonyloxycarbamate [Synthesis 1972, 140] in N,N-dimethylformamide in the presence of potassium carbonate at room temperature to yield beside the 1-amino-2-methyl-6-oxo-4-(1,2,4,5-tetrahydro-benzo[d]azepin-3-yl)-1,6-dihydro-pyrimidine-5-carbonitrile (example 200) the N'-[5-cyano-2-methyl-6-oxo-4-(1,2,4,5-tetrahydro-benzo[d]azepin-3-yl)-6H-pyrimidin-1-yl]-hydrazinecarboxylic acid tert-butyl ester as colorless solid; m.p. 170-171.5 °C; MS: [M+H]⁺ = 411.

### Example 224

### 1-Methoxy-2-methyl-6-oxo-4-(1,2,4,5-tetrahydro-benzo[d]azepin-3-yl)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with the methly iodide in N,N-dimethylformamide in the presence of potassium carbonate to yield the 1-methoxy-2-methyl-6-oxo-4-(1,2,4,5-tetrahydro-benzo[d]azepin-3-yl)-1,6-dihydro-pyrimidine-5-carbonitrile as colorless solid; m.p. 144-145 °C; MS: [M]⁺ = 310.

### Example 225

### 1-Ethoxy-2-methyl-6-oxo-4-(1,2,4,5-tetrahydro-benzo[d]azepin-3-yl)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with the ethyl iodide in N,N-dimethylformamide in the presence of potassium carbonate at room temperature to yield the 1-ethoxy-2-methyl-6-oxo-4-(1,2,4,5-tetrahydro-benzo[d]azepin-3-yl)-1,6-dihydro-pyrimidine-5-carbonitrile as colorless solid; m.p. 149-152 °C; MS: [M]⁺ = 324.

### Example 226

### 2-Methyl-6-oxo-1-propoxy-4-(1,2,4,5-tetrahydro-benzo[d]azepin-3-yl)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with n-propyl iodide in N,N-dimethylformamide in the presence of potassium carbonate at room temperature to yield the 2-methyl-6-oxo-1-propoxy-4-(1,2,4,5-tetrahydro-benzo[d]azepin-3-yl)-1,6-dihydro-pyrimidine-5-carbonitrile as colorless solid; m.p. 132.5-134 °C; MS: [M]⁺ = 338.

### Example 227

### 4-Methoxy-2-methyl-6-(1,2,4,5-tetrahydro-benzo[d]azepin-3-yl)-pyrimidine-5-carbonitrile

In analogy to the procedure described in example 3 the 2-methyl-6-oxo-4-(1,2,4,5-tetrahydro-bentzo[d]azepin-3-yl)-1,6-dihydro-pyrimidine-5-carbonitrile (example 17) was treated with methyl iodide in N,N-dimethylformamide in the presence of potassium carbonate to yield the beside the 1,2-dimethyl-6-oxo-4-(1,2,4,5-tetrahydro-benzo[d]azepin-3-yl)-1,6-dihydro-pyrimidine-5-carbonitrile (example 26) the 4-methoxy-2-methyl-6-(1,2,4,5-tetrahydro-benzo[d]azepin-3-yl)-pyrimidine-5-carbonitrile as colorless amorphous solid; MS: [M]⁺ = 294.

### Example 228

### 2-Methyl-4-propoxy-6-(1,2,4,5-tetrahydro-benzo[d]azepin-3-yl)-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) with n-propyl iodide in N,N-dimethylformamide in the presence of potassium carbonate as colorless amorphous solid; MS: [M+H]⁺ = 323; see example 229.

### Example 229

### 2-Methyl-6-oxo-1-propyl-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with n-propyl iodide in N,N-dimethylformamide in the presence of potassium carbonate to yield the 2-methyl-6-oxo-1-propyl-4-(1,2,4,5-tetrahydro-benzo[d]azepin-3-yl)-1,6-dihydro-pyrimidine-5-carbonitrile as colorless amorphous solid; MS: [M+H]⁺ = 323; and the 2-methyl-4-propoxy-6-(1,2,4,5-tetrahydro-benzo[d]azepin-3-yl)-pyrimidine-5-carbonitrile as colorless amorphous solid; MS: [M+H]⁺ = 323.

### Example 230

### (1,2-Diamino-6-oxo-4-(1,2,4,5-tetrahydro-benzo[d]azepin-3-yl)-1,6-dihydro-pyrimidine-5-carbonitrile

In analogy to the procedure described in example 3 the 2-amino-6-oxo-4-(1,2,4,5-tetrahydro-benzo[d]azepin-3-yl)-1,6-dihydro-pyrimidine-5-carbonitrile (example 13) was treated with the O-mesitylenesulfonylhydroxylamine [Synthesis 1972, 140] in N,N-dimethylformamide in the presence of potassium carbonate at room temperature to yield the (1,2-diamino-6-oxo-4-(1,2,4,5-tetrahydro-benzo[d] azepin-3-yl)-1,6-dihydro-pyrimidine-5-carbonitrile as colorless solid; m.p. >200 °C; MS: [M+H]⁺ = 297.

### Example 231

### 3-{6-[4-(tert-Butyl-dimethyl-silanyloxy)-butoxy]-2-methyl-5-nitro-pyrimidin-4-yl}-2,3,4,5-tetrahydro-1H-benzo[d]azepine

From the 2-methyl-5-nitro-6-(1,2,4,5-tetrahydro-benzo[d]azepin-3-yl)-pyrimidin-4-ol (example 1) and the tert.-butyl(4-chlorobutoxy)dimethylsilane in N,N-dimethylformamide in the presence of potassium carbonate at 120 °C as light yellow oil; MS: [M-C₄H₉]⁺ = 429; see example 232.

### Example 232

### 3-[4-(tert-Butyl-dimethyl-silanyloxy)-butyl]-2-methyl-5-nitro-6-(1,2,4,5-tetrahydro-benzo[d]azepin-3-yl)-3H-pyrimidin-4-one

In analogy to the procedure described in example 3 the 2-methyl-5-nitro-6-(1,2,4,5-tetrahydro-benzo[d]azepin-3-yl)-pyrimidin-4-ol (example 1) was treated with the tert.-butyl(4-chlorobutoxy)dimethylsilane in N,N-dimethylformamide in the presence of potassium carbonate at 120 °C to yield the 3-[4-(tert-butyl-dimethyl-silanyloxy)-butyl]-2-methyl-5-nitro-6-(1,2,4,5-tetrahydro-benzo[d]azepin-3-yl)-3H-pyrimidin-4-one as light yellow oil; MS: [M-C₄H₉]⁺ = 429; and the 3-{6-[4-(tert-butyl-dimethyl-silanyloxy)-butoxy]-2-methyl-5-nitro-pyrimidin-4-yl}-2,3,4,5-tetrahydro-1H-benzo[d]azepine as light yellow oil; MS: [M-C₄H₉]⁺ = 429.

### Example 233

### 4-[2-Methyl-5-nitro-6-(1,2,4,5-tetrahydro-benzo[d]azepin-3-yl)-pyrimidin-4-yloxy]-butan-1-ol

A solution of 4.01 g (8.23 mmol) of the 3-{6-[4-(tert-butyl-dimethyl-silanyloxy)-butoxy]-2-methyl-5-nitro-pyrimidin-4-yl}-2,3,4,5-tetrahydro-1H-benzo[d]azepine (example 231) in 45 ml of a 2:1 v/v mixture of acetonitrile and dichloromethane was treated with stirring with 5.2 ml of hydrogen fluoride solution (40% in water) and the reaction mixture was stirred at room temperature for 1 hour. It was then poured into 150 ml of an ice/water mixture and extracted three times with 150 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 9:1 to 1:1 v/v mixture of hexane and ethyl acetate as eluent to yield 2.18 g (5.85 mmol), 71 %, of the 4-[2-methyl-5-nitro-6-(1,2,4,5-tetrahydro-benzo[d]azepin-3-yl)-pyrimidin-4-yloxy]-butan-1-ol as yellow oil; MS: [M+H]⁺ = 373.

### Example 234

### 3-(4-Hydroxy-butyl)-2-methyl-5-nitro-6-(1,2,4,5-tetrahydro-benzo[d]azepin-3-yl)-3H-pyrimidin-4-one

In analogy to the procedure described in example 233 the 3-[4-(tert-butyl-dimethyl-silanyloxy)-butyl]-2-methyl-5-nitro-6-(1,2,4,5-tetrahydro-benzo[d]azepin-3-yl)-3H-pyrimidin-4-one (example 232) was treated with a hydrogen fluoride solution (40% in water) in a 2:1 v/v mixture of acetonitrile and dichloromethane at room temperature to yield the 3-(4-hydroxy-butyl)-2-methyl-5-nitro-6-(1,2,4,5-tetrahydro-benzo[d]azepin-3-yl)-3H-pyrimidin-4-one as yellow oil; [M+H]⁺ = 373.

### Example 235

### Methanesulfonic acid 4-[2-methyl-5-nitro-6-(1,2,4,5-tetrahydro-benzo[d]azepin-3-yl)-pyrimidin-4-yloxy]-butyl ester

Treatment of the 4-[2-methyl-5-nitro-6-(1,2,4,5-tetrahydro-benzo[d]azepin-3-yl)-pyrimidin-4-yloxy]-butan-1-ol (example 233) with methanesulfonyl chloride in dichloromethane (-70 °C to room temperature) gave the methanesulfonic acid 4-[2-methyl-5-nitro-6-(1,2,4,5-tetrahydro-benzo[d]azepin-3-yl)-pyrimidin-4-yloxy]-butyl ester as yellow amorphous solid; [M+H]⁺ = 451.

### Example 236

### Methanesulfonic acid 4-[2-methyl-5-nitro-6-oxo-4-(1,2,4,5-tetrahydro-benzo[d]azepin-3-yl)-6H-pyrimidin-1-yl]-butyl ester

Treatment of the 3-(4-hydroxy-butyl)-2-methyl-5-nitro-6-(1,2,4,5-tetrahydro-benzo[d]azepin-3-yl)-3H-pyrimidin-4-one (example 234) with methanesulfonyl chloride in dichloromethane (-70 °C to room temperature) gave the methanesulfonic acid 4-[2-methyl-5-nitro-6-oxo-4-(1,2,4,5-tetrahydro-benzo[d]azepin-3-yl)-6H-pyrimidin-1-yl]-butyl ester as yellow amorphous solid; [M+H]⁺ = 451.

### Example 237

### 3-[3-Nitro-2-(1,2,4,5-tetrahydro-benzo[d]azepin-3-yl)-6,7,8,9-tetrahydro-5H-cyclohepta[b]pyridin-4-ylamino]-propan-1-ol

In analogy to the procedure described in example 4 the trifluoro-methanesulfonic acid 4-(3-hydroxy-propylamino)-3-nitro-6,7,8,9-tetrahydro-5H-cyclohepta[b]pyridin-2-yl ester was treated with the 2,3,4,5-tetrahydro-1H-benzo[d]azepine hydrochloride [J. HeterocycL Chem. (1971), 8(5), 779-83] in N,N-dimethylformamide in the presence of N-ethyl-N,N-diisopropylamine at room temperature to yield the 3-[3-nitro-2-(1,2,4,5-tetrahydro-benzo[d]azepin-3-yl)-6,7,8,9-tetrahydro-5H-cyclohepta[b]pyridin-4-ylamino]-propan-1-ol as yellow oil; MS: [M+H]⁺ = 411.

### Preparation of the trifluoro-methanesulfonic acid 4-(3-hydroxy-propylamino)-3-nitro-6,7,8,9-tetrahydro-5H-cyclohepta[b]pyridin-2-yl ester

The trifluoro-methanesulfonic acid 4-(3-hydroxy-propylamino)-3-nitro-6,7,8,9-tetrahydro-5H-cyclohepta[b]pyridin-2-yl ester used above was prepared by the following reaction sequence: i) treatment of the 3-nitro-6,7,8,9-tetrahydro-5H-cyclohepta[b]pyridine-2,4-diol [US 5352784 A (1994)] with trifluoromethanesulfonic anhydride and triethylamine in dichloromethane at 3 °C gave the trifluoro-methanesulfonic acid 3-nitro-2-trifluoromethanesulfonyloxy-6,7,8,9-tetrahydro-5H-cyclohepta[b]pyridin-4-yl ester; ii) treatment of the trifluoro-methanesulfonic acid 3-nitro-2-trifluoromethanesulfonyloxy-6,7,8,9-tetrahydro-5H-cyclohepta[b]pyridin-4-yl ester with 3-amino-1-propanol and triethylamine in dichloromethane at room temperature yielded the trifluoro-methanesulfonic acid 4-(3-hydroxy-propylamino)-3-nitro-6,7,8,9-tetrahydro-5H-cyclohepta[b]pyridin-2-yl ester.

### Example 238

### 3-Nitro-2-(1,2,4,5-tetrahydro-benzo[d]azepin-3-yl)-6,7,8,9-tetrahydro-5H-cyclohepta[b]pyridin-4-ol

In analogy to the procedure described in example 4 the trifluoro-methanesulfonic acid 4-hydroxy-3-nitro-6,7,8,9-tetrahydro-5H-cyclohepta[b]pyridin-2-yl ester [prepared from the trifluoro-methanesulfonic acid 3-nitro-2-trifluoromethanesulfonyloxy-6,7,8,9-tetrahydro-5H-cyclohepta[b]pyridin-4-yl ester (example 237) and potassium carbonate in a mixture of tetrahydrofuran and water at room temperature] was treated with the 2,3,4,5-tetrahydro-1H-benzo[d]azepine hydrochloride [J. Heterocycl. Chem. (1971), 8(5), 779-83] in N,N-dimethylformamide in the presence of N-ethyl-N,N-diisopropylamine at 50 °C to yield the 3-nitro-2-(1,2,4,5-tetrahydro-benzo[d]azepin-3-yl)-6,7,8,9-tetrahydro-5H-cyclohepta[b]pyridin-4-ol as light yellow solid; m.p. >200 °C; MS: [M+H]⁺ = 354.

### Example 239

### [5-Cyano-2-methyl-6-oxo-4-(1,2,4,5-tetrahydro-benzo[d]azepin-3-yl)-6H-pyrimidin-1-yl]-methyl-carbamic acid tert-butyl ester

In analogy to the procedure described in example 3 the [5-cyano-2-methyl-6-oxo-4-(1,2,4,5-tetrahydro-benzo[d]azepin-3-yl)-6H-pyrimidin-1-yl]-carbamic acid tert-butyl ester (example 218) was treated with methyl iodide in N,N-dimethylformamide in the presence of potassium carbonate at room temperature to yield the [5-cyano-2-methyl-6-oxo-4-(1,2,4,5-tetrahydro-benzo[d]azepin-3-yl)-6H-pyrimidin-1-yl]-methyl-carbamic acid tert-butyl ester as colorless solid; m.p. 164-166 °C; MS: [M+H]⁺ = 410.

### Example 240

### [5-Cyano-2-methyl-6-oxo-4-(1,2,4,5-tetrahydro-benzo[d]azepin-3-yl)-6H-pyrimidin-1-yl]-ethyl-carbamic acid tert-butyl ester

In analogy to the procedure described in example 3 the [5-cyano-2-methyl-6-oxo-4-(1,2,4,5-tetrahydro-benzo[d]azepin-3-yl)-6H-pyrimidin-1-yl]-carbamic acid tert-butyl ester (example 218) was treated with ethyl iodide in N,N-dimethylformamide in the presence of potassium carbonate at room temperature to yield the [5-cyano-2-methyl-6-oxo-4-(1,2,4,5-tetrahydro-benzo[d]azepin-3-yl)-6H-pyrimidin-1-yl]-ethyl-carbamic acid tert-butyl ester as colorless solid; m.p. 86-88 °C; MS: [M+H]⁺ = 424.

### Example 241

### 5-Cyano-2-methyl-6-oxo-4-(1,2,4,5-tetrahydro-benzo[d]azepin-3-yl)-6H-pyrimidin-1-yl]-(2,2,2-trifluoro-ethyl)-carbamic acid tert-butyl ester

In analogy to the procedure described in example 3 the [5-cyano-2-methyl-6-oxo-4-(1,2,4,5-tetrahydro-benzo[d]azepin-3-yl)-6H-pyrimidin-1-yl]-carbamic acid tert-butyl ester (example 218) was treated with the 2,2,2-trifluoroethyl-trifluoromethanesulfonate in N,N-dimethylformamide in the presence of potassium carbonate at room temperature to yield the [5-cyano-2-methyl-6-oxo-4-(1,2,4,5-tetrahydro-benzo[d]azepin-3-yl)-6H-pyrimidin-1-yl]-(2,2,2-trifluoro-ethyl)-carbamic acid tert-butyl ester as colorless solid; m.p. 86-88 °C; MS: [M+H]⁺ = 478.

### Example 242

### [5-Cyano-2-methyl-6-oxo-4-(1,2,4,5-tetrahydro-benzo[d]azepin-3-yl)-6H-pyrimidin-1-yl]-isopropyl-carbamic acid tert-butyl ester

In analogy to the procedure described in example 3 the [5-cyano-2-methyl-6-oxo-4-(1,2,4,5-tetrahydro-benzo[d]azepin-3-yl)-6H-pyrimidin-1-yl]-carbamic acid tert-butyl ester (example 218) was treated with the 2-iodopropane in N,N-dimethylformamide in the presence of potassium carbonate at room temperature to yield the [5-cyano-2-methyl-6-oxo-4-(1,2,4,5-tetrahydro-benzo[d]azepin-3-yl)-6H-pyrimidin-1-yl]-isopropyl-carbamic acid tert-butyl ester as colorless amorphous solid; MS: [M+H]⁺ = 438.

### Example 243

### 1-Isopropylamino-2-methyl-6-oxo-4-(1,2,4,5-tetrahydro-benzo[d]azepin-3-yl)-1,6-dihydro-pyrimidine-5-carbonitrile

In analogy to the procedure described in example 70 the [5-cyano-2-methyl-6-oxo-4-(1,2,4,5-tetrahydro-benzo[d]azepin-3-yl)-6H-pyrimidin-1-yl]-isopropyl-carbamic acid tert-butyl ester (example 242) was treated with 1.5N hydrogen chloride in methanol at room temperature to yield the 1-isopropylamino-2-methyl-6-oxo-4-(1,2,4,5-tetrahydro-benzo[d]azepin-3-yl)-1,6-dihydro-pyrimidine-5-carbonitrile as colorless solid; m.p. 142-146 °C; MS: [M+H]⁺ = 338.

### Example 244

### 1-Ethylamino-2-methyl-6-oxo-4-(1,2,4,5-tetrahydro-benzo[d]azepin-3-yl)-1,6-dihydro-pyrimidine-5-carbonitrile

In analogy to the procedure described in example 70 the [5-cyano-2-methyl-6-oxo-4-(1,2,4,5-tetrahydro-benzo[d]azepin-3-yl)-6H-pyrimidin-1-yl]-ethyl-carbamic acid tert-butyl ester (example 240) was treated with 1.5N hydrogen chloride in methanol at room temperature to yield the 1-ethylamino-2-methyl-6-oxo-4-(1,2,4,5-tetrahydro-benzo[d]azepin-3-yl)-1,6-dihydro-pyrimidine-5-carbonitrile as colorless solid; m.p. 172-174 °C; MS: [M+H]⁺ = 324.

### Example 245

### 2-Methyl-6-oxo-4-(1,2,4,5-tetrahydro-benzo[d]azepin-3-yl)-1-(2,2,2-trifluoro-ethylamino)-1,6-dihydro-pyrimidine-5-carbonitrile

In analogy to the procedure described in example 70 the [5-cyano-2-methyl-6-oxo-4-(1,2,4,5-tetrahydro-benzo[d]azepin-3-yl)-6H-pyrimidin-1-yl]-(2,2,2-trifluoro-ethyl)-carbamic acid tert-butyl ester (example 241) was treated with 1.5N hydrogen chloride in methanol at room temperature to yield the 2-methyl-6-oxo-4-(1,2,4,5-tetrahydro-benzo[d]azepin-3-yl)-1-(2,2,2-trifluoro-ethylamino)-1,6-dihydro-pyrimidine-5-carbonitrile as colorless solid; m.p. 144-146 °C; MS: [M+H]⁺ = 378.

### Example 246

### 2-(2-Methoxy-ethoxy)-4-methylsulfanyl-6-(1,2,4,5-tetrahydro-benzo[d]azepin-3-yl)-pyrimidine-5-carbonitrile

0.011 g (0.25 mmol) of sodium hydride dispersion (50% in mineral oil) was added to a solution of 0.019 g (0.25 mmol) of the 2-methoxy-ethanol in 2 ml of tetrahydrofuran and the reaction mixture was stirred for 15 minutes at room temperature. Then, a solution of 0.10 g (0.25 mmol) of the 2-methylsulfanyl-4-(1,2,4,5-tetrahydro-benzo[d]azepin-3-yl)-6-(2,2,2-trifluoro-ethoxy)-pyrimidine-5-carbonitrile (example 166) in 3.0 ml of tetrahydrofuran was added and stirring continued at 40 °C for 16 hours. The reaction mixture was then poured into 150 ml of an ice/water mixture and extracted three times with 150 ml of dichloromethane. The combined organic phases were washed twice with 50 ml of water, dried over magnesium sulphate, evaporated under reduced pressure and dried in a high vacuum. The thus obtained crude product was purified by chromatography on silica gel using a 9:1 v/v mixture of hexane and ethyl acetate as eluent to yield 0.051 g (0.14 mmol), 55%, of the 2-(2-methoxy-ethoxy)-4-methylsulfanyl-6-(1,2,4,5-tetrahydro-benzo[d]azepin-3-yl)-pyrimidine-5-carbonitrile as colorless solid; m.p. 108-111 °C; MS: [M+H]⁺ = 371.

### Example 247

### 4-Amino-2-methanesulfonyl-6-(1,2,4,5-tetrahydro-benzo[d]azepin-3-yl)-pyrimidine-5-carbonitrile

Oxidation of the 4-amino-2-methylsulfanyl-6-(1,2,4,5-tetrahydro-benzo[d]azepin-3-yl)-pyrimidine-5-carbonitrile (example 164) with m-chloroperbenzoic acid in dichloromethane at room temperature yielded the 4-amino-2-methanesulfonyl-6-(1,2,4,5-tetrahydro-benzo[d]azepin-3-yl)-pyrimidine-5-carbonitrile as colorless solid; m.p. 184-185.5 °C; MS: [M+H]⁺ = 344.

### Example 248

### 4-(1,2,4,5-Tetrahydro-benzo[d]azepin-3-yl)-2,6-bis-(2,2,2-trifluoro-ethoxy)-pyrimidine-5-carbonitrile

In analogy to the procedure described in example 47 a) the 4-chloro-2-methylsulfanyl-6-(1,2,4,5-tetrahydro-benzo[d]azepin-3-yl)-pyrimidine-5-carbonitrile (example 149) was treated with the trifluoroethanol in tetrahydrofuran in the presence of sodium hydride at room temperature to yield beside the 2-methylsulfanyl-4-(1,2,4,5-tetrahydro-benzo[d]azepin-3-yl)-6-(2,2,2-trifluoro-ethoxy)-pyrimidine-5-carbonitrile (example 166) the 4-(1,2,4,5-tetrahydro-benzo[d]azepin-3-yl)-2,6-bis-(2,2,2-trifluoro-ethoxy)-pyrimidine-5-carbonitrile as colorless solid; m.p. 130-133.5 °C; MS: [M+H]⁺ = 447.

### Example 249

### 2-Amino-4-methylsulfanyl-6-(1,2,4,5-tetrahydro-benzo[d]azepin-3-yl)-pyrimidine-5-carbonitrile

In analogy to the procedure described in example 4 the 2-amino-4-bromo-6-methylsulfanyl-pyrimidine-5-carbonitrile [prepared from the 2,2-dicyano-1-methylsulfanyl-vinyl-cyanamide sodium salt [EP 244360 A2 (1987)] with excess hydrogen bromide in acetic acid between 0 °C and room temperature] was treated with the 2,3,4,5-tetrahydro-1H-benzo[d]azepine hydrochloride [J. Heterocycl. Chem. (1971), 8(5), 779-83] in dioxan in the presence of aqueous sodium hydroxide solution at room temperature to yield the 2-amino-4-methylsulfanyl-6-(1,2,4,5-tetrahydro-benzo[d]azepin-3-yl)-pyrimidine-5-carbonitrile as colorless solid; m.p. 164-168 °C; MS: [M]⁺ = 311.

### Example 250

### N-[5-Cyano-4-methylsulfanyl-6-(1,2,4,5-tetrahydro-benzo[d]azepin-3-yl)-pyrimidin-2-yl]-2-methoxy-acetamide

A solution of 0.10 g (0.32 mmol) of the 2-amino-4-methylsulfanyl-6-(1,2,4,5-tetrahydro-benzo[d]azepin-3-yl)-pyrimidine-5-carbonitrile (example 249) in 5 ml of pyridine was treated with 0.072 g (0.64 mmol) of the methoxyacetyl-chloride and the reaction mixture was stirred at 60°C for 16 hours. It was then poured into 150 ml of an ice/water mixture and extracted three times with 150 ml of dichloromethane. The combined organic phases were washed three times with 50 ml of 1N hydrogen chloride solution, twice with 50 ml of water, dried over magnesium sulphate, evaporated under reduced pressure and dried in a high vacuum. The thus obtained crude product was purified by chromatography on silica gel using a 1:1 v/v mixture of hexane and ethyl acetate as eluent to yield 0.070 g (0.18 mmol), 57%, of the N-[5-cyano-4-methylsulfanyl-6-(1,2,4,5-tetrahydro-benzo[d]azepin-3-yl)-pyrimidin-2-yl]-2-methoxy-acetamide as light yellow amorphous solid; MS: [M+H]⁺ = 384.

### Example 251

### [rac]-2-(2-Hydroxy-propylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the [rac]-1-amino-2-hydroxypropane in dioxan at 60 °C to yield the [rac]-2-(2-hydroxy-propylamino)-4-(1,2,4,5-tetrahydro-benzo[d]azepin-3-yl)-6-(2,2,2-trifluoro-ethoxy)-pyrimidine-5-carbonitrile as colorless solid; m.p. 141-147 °C; MS: [M+H]⁺ = 422.

### Example 252

### 2-[2-(2-Hydroxy-ethoxy)-ethylamino]-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 2-(2-aminoethoxy)-ethanol in dioxan at 40 °C to yield the 2-[2-(2-hydroxy-ethoxy)-ethylamino]-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 452.

### Example 253

### [rac]-2-[(2,2-Dimethyl-[1,3]dioxolan-4-ylmethyl)-amino]-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d] azepin-3-yl)-6-(2,2,2-trifluoro-ethoxy)-pyrimidine-5-carbonitrile (example 172) was treated with the [rac]-2,2-dimethyl-1,3-dioxolane-4-methanamine in dioxan at 40 °C to yield the [rac]-2-[(2,2-dimethyl-[1,3]dioxolan-4-ylmethyl)-amino]-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 478.

### Example 254

### 3-[5-Cyano-4-(1,2,4,5-tetrahydro-benzo[d]azepin-3-yl)-6-(2,2,2-trifluoro-ethoxy)-pyrimidin-2-ylamino]-propionic acid tert-butyl ester

In analogy to the procedure described in example 48 the 2-methanesulfonyl-4-(1,2,4,5-tetrahydro-benzo [d] azepin-3-yl)-6-(2,2,2-trifluoro-ethoxy)-pyrimidine-5-carbonitrile (example 172) was treated with the beta-alanine-tert-butylester in dioxan at 50 °C to yield the 3-[5-cyano-4-(1,2,4,5-tetrahydro-benzo[d]azepin-3-yl)-6-(2,2,2-trifluoro-ethoxy)-pyrimidin-2-ylamino]-propionic acid tert-butyl ester as colorless amorphous solid; MS: [M+H]⁺ = 492.

### Example 255

### [5-Cyano-4-(1,2,4,5-tetrahydro-benzo[d]azepin-3-yl)-6-(2,2,2-trifluoro-ethoxy)-pyrimidin-2-ylamino]-acetic acid methyl ester

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the glycine-methylester hydrochloride and N-ethyl-N,N-diisopropylamin in dioxan at 50 °C to yield the [5-cyano-4-(1,2,4,5-tetrahydro-benzo[d]azepin-3-yl)-6-(2,2,2-trifluoro-ethoxy)-pyrimidin-2-ylamino]-acetic acid methyl esteras colorless solid; m.p. 136-140 °C; MS: [M+H]⁺ = 436.

### Example 256

### N-[5-Cyano-4-methylsulfanyl-6-(1,2,4,5-tetrahydro-benzo[d]azepin-3-yl)-pyrimidin-2-yl]-acetamide

In analogy to the procedure described in example 250 the 2-amino-4-methylsulfanyl-6-(1,2,4,5-tetrahydro-benzo[d]azepin-3-yl)-pyrimidine-5-carbonitrile (example 249) was treated with the acetylchloride in pyridine at 60 °C to yield the N-[5-cyano-4-methylsulfanyl-6-(1,2,4,5-tetrahydro-benzo[d]azepin-3-yl)-pyrimidin-2-yl]-acetamide as colorless amorphous solid; MS: [M+H]⁺ = 354.

### Example 257

### N-[5-Cyano-4-methylsulfanyl-6-(1,2,4,5-tetrahydro-benzo[d]azepin-3-yl)-pyrimidin-2-yl]-3-methyl-butyramide

In analogy to the procedure described in example 250 the 2-amino-4-methylsulfanyl-6-(1,2,4,5-tetrahydro-benzo[d]azepin-3-yl)-pyrimidine-5-carbonitrile (example 249) was treated with the isovaleric acid chloride in pyridine at 60 °C to yield the N-[5-cyano-4-methylsulfanyl-6-(1,2,4,5-tetrahydro-benzo[d]azepin-3-yl)-pyrimidin-2-yl]-3-methyl-butyramide as colorless amorphous solid; MS: [M+H]⁺ = 396.

### Example 258

### 4-(4-Methoxy-benzyloxy)-3-nitro-2-(1,2,4,5-tetrahydro-benzo[d]azepin-3-yl)-6,7,8,9-tetrahydro-5H-cyclohepta[b]pyridine

In analogy to the procedure described in example 4 the trifluoro-methanesulfonic acid 4-(4-methoxy-benzyloxy)-3-nitro-6,7,8,9-tetrahydro-5H-cyclohepta[b]pyridin-2-yl ester (prepared from the trifluoro-methanesulfonic acid 3-nitro-2-trifluoromethanesulfonyloxy-6,7,8,9-tetrahydro-5H-cyclohepta[b]pyridin-4-yl ester (example 237) and 4-methoxy-benzyl alcohol, sodium hydride in tetrahydrofuran at room temperature) was treated with the 2,3,4,5-tetrahydro-1H-benzo[d]azepine hydrochloride [J. Heterocyd. Chem. (1971), 8(5), 779-83] in N,N-dimethylformamide in the presence of N-ethyl-N,N-diisopropylamine at room temperature to yield the 4-(4-methoxy-benzyloxy)-3-nitro-2-(1,2,4,5-tetrahydro-benzo[d]azepin-3-yl)-6,7,8,9-tetrahydro-5H-cyclohepta[b]pyridine as yellow amorphous solid; MS: [M+H]⁺ = 474.

### Example 259

### [rac]-2-(2,3-Dihydroxy-propylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 70 the [rac]-2-[(2,2-dimethyl-[1,3]dioxolan-4-ylmethyl)-amino]-4-(1,2,4,5-tetrahydro-benzo[d]azepin-3-yl)-6-(2,2,2-trifluoro-ethoxy)-pyrimidine-5-carbonitrile (example 253) was treated with hydrogen chloride in tetrahydrofuran at room temperature to yield the [rac]-2-(2,3-dihydroxy-propylamino)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 438.

### Example 260

### 4-Amino-2-(2-hydroxy-ethylamino)-6-(1,2,4,5-tetrahydro-benzo[d]azepin-3-yl)-pyrimidine-5-carbonitrile

In analogy to the procedure described in example 48 the 4-amino-2-methanesulfonyl-6-(1,2,4,5-tetrahydro-benzo[d]azepin-3-yl)-pyrimidine-5-carbonitrile (example 247) was treated with the ethanolamine in dioxan at 80 °C to yield the 4-amino-2-(2-hydroxy-ethylamino)-6-(1,2,4,5-tetrahydro-benzo[d]azepin-3-yl)-pyrimidine-5-carbonitrile as colorless amorphous solid; MS: [M+H]⁺ = 325.

### Example 261

### 2-(3-Hydroxy-propylamino)-4-(1,2,4,5-tetrahydro-benzo[d]azepin-3-yl)-6-(2,2,2-trifluoro-ethoxy)-pyrimidine-5-carbonitrile

In analogy to the procedure described in example 48 the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 3-aminopropanol in dioxane at 40 °C to yield the 2-(3-hydroxy-propylamino)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 422.

### Example 262

### 2-(2-Methoxy-ethoxy)-4-(1,2,4,5-tetrahydro-benzo[d]azepin-3-yl)-6-(2,2,2-trifluoro-ethoxy)-pyrimidine-5-carbonitrile

In analogy to the procedure described in example 47 a) the 2-methanesulfonyl-4-(1,2,4,5-tetrahydro-benzo[d]azepin-3-yl)-6-(2,2,2-trifluoro-ethoxy)-pyrimidine-5-carbonitrile (example 172) was treated with the 2-methoxy-ethanol in tetrahydrofuran in the presence of sodium hydride at 40 °C to yield the 2-(2-methoxy-ethoxy)-4-(1,2,4,5-tetrahydro-benzo[d]azepin-3-yl)-6-(2,2,2-trifluoro-ethoxy)-pyrimidine-5-carbonitrile as colorless amorphous solid; MS: [M+H]⁺ = 423.

### Example 263

### 1-Isopropoxy-2-methyl-6-oxo-4-(1,2,4,5-tetrahydro-benzo[d]azepin-3-yl)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with isopropyl iodide in N,N-dimethylformamide in the presence of potassium carbonate at room temperature to yield the 1-isopropoxy-2-methyl-6-oxo-4-(1,2,4,5-tetrahydro-benzo[d]azepin-3-yl)-1,6-dihydro-pyrimidine-5-carbonitrile as colorless solid; m.p. 127-128 °C; MS: [M]⁺ = 339.

### Example 264

### 3-[2-Methyl-5-nitro-6-(4-[1,2,4]triazol-1-yl-butoxy)-pyrimidin-4-yl]-2,3,4,5-tetrahydro-1H-benzo[d]azepine

In analogy to the procedure described in example 202 the methanesulfonic acid 4-[2-methyl-5-nitro-6-(1,2,4,5-tetrahydro-benzo[d]azepin-3-yl)-pyrimidin-4-yloxy]-butyl ester (example 235) was treated with the 1,2,4-triazole and sodium hydride in N,N-dimethylformamide at room temperature to yield the 3-[2-methyl-5-nitro-6-(4-[1,2,4]triazol-1-yl-butoxy)-pyrimidin-4-yl]-2,3,4,5-tetrahydro-1H-benzo[d]azepine as light yellow solid; m.p. 88-91 °C; MS: [M+H]⁺ = 424.

### Example 265

### 2-Methyl-5-nitro-6-(1,2,4,5-tetrahydro-benzo[d]azepin-3-yl)-3-(4-[1,2,4]triazol-1-yl-butyl)-3H-pyrimidin-4-one

In analogy to the procedure described in example 202 the methanesulfonic acid 4-[2-methyl-5-nitro-6-oxo-4-(1,2,4,5-tetrahydro-benzo[d]azepin-3-yl)-6H-pyrimidin-1-yl]-butyl ester (example 236) was treated with the 1,2,4-triazole and sodium hydride in N,N-dimethylformamide at room temperature to yield the 2-methyl-5-nitro-6-(1,2,4,5-tetrahydro-benzo[d]azepin-3-yl)-3-(4-[1,2,4]triazol-1-yl-butyl)-3H-pyrimidin-4-one as light yellow amorphous solid; MS: [M+H]⁺ = 424.

### Example 266

### 1-(3,3-Difluoro-allyl)-2-methyl-6-oxo-4-(1,2,4,5-tetrahydro-benzo[d]azepin-3-yl)-1,6-dihydro-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) and 3-bromo-3,3-difluoropropene, potassium carbonate in N,N-dimethylformamide at room temperature as light yellow amorphous solid; m.p. 158-159 °C; MS: [M]⁺ = 356; see example 267.

### Example 267

### 4-(3,3-Difluoro-allyloxy)-2-methyl-6-(1,2,4,5-tetrahydro-benzo[d]azepin-3-yl)-pyrimidine-5-carbonitrile

In analogy to the procedure described in example 3 the 2-methyl-6-oxo-4-( 1,2,4,5-tetrahydro-benzo[d]azepin-3-yl)-1,6-dihydro-pyrimidine-5-carbonitrile (example 17) was treated with the 3-bromo-3,3-difluoropropene in N,N-dimethylformamide in the presence of potassium carbonate at room temperature to yield the 4-(3,3-difluoro-allyloxy)-2-methyl-6-(1,2,4,5-tetrahydro-benzo[d]azepin-3-yl)-pyrimidine-5-carbonitrile as colorless amorphous solid; MS: [M]⁺ = 356; and the 1-(3,3-difluoro-allyl)-2-methyl-6-oxo-4-(1,2,4,5-tetrahydro-benzo[d]azepin-3-yl)-1,6-dihydro-pyrimidine-5-carbonitrile as light yellow amorphous solid; m.p. 158-159 °C; MS: [M]⁺ = 356.

### Example 268

### 4-(1,1-Difluoro-allyloxy)-2-methyl-6-(1,2,4,5-tetrahydro-benzo[d]azepin-3-yl)-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) and 3-bromo-3,3-difluoropropene, silver carbonate in 1,2-dichlorethane at reflux as light yellow amorphous solid; MS: [M]⁺ = 356; see example 269.

### Example 269

### 1-(1,1-Difluoro-allyl)-2-methyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with the 3-bromo-3,3-difluoropropene in 1,2-dichlorethane in the presence of silver carbonate at reflux to yield beside the 4-(3,3-difluoro-allyloxy)-2-methyl-6-(1,2,4,5-tetrahydro-benzo[d]azepin-3-yl)-pyrimidine-5-carbonitrile (example 267) the 1-(1,1-difluoro-allyl)-2-methyl-6-oxo-4-(1,2,4,5-tetrahydro-benzo[d]azepin-3-yl)-1,6-dihydro-pyrimidine-5-carbonitrile as colorless amorphous solid; MS: [M]⁺ = 356; and the 4-(1,1-difluoro-allyloxy)-2-methyl-6-(1,2,4,5-tetrahydro-benzo[d]azepin-3-yl)-pyrimidine-5-carbonitrile as light yellow amorphous solid; MS: [M]⁺ = 356.

### Example 270

### 4-Difluoromethoxy-2-methyl-6-(1,2,4,5-tetrahydro-benzo[d]azepin-3-yl)-pyrimidine-5-carbonitrile

From the 2-methyl-6-oxo-4-(1,2,4,5-tetrahydro-benzo[d]azepin-3-yl)-1,6-dihydro-pyrimidine-5-carbonitrile (example 17) and 1-chloro-1,1-difluoromethane, potassium carbonate in N,N-dimethylformamide at 145 °C in an autoclave as colorless solid; m.p. 143 °C; MS: [M+H]⁺ = 331; see example 271.

### Example 271

### 1-Difluoromethyl-2-methyl-6-oxo-4-(1,2,4,5-tetrahydro-benzo[d]azepin-3-yl)-1,6-dihydro-pyrimidine-5-carbonitrile

In analogy to the procedure described in example 3 the 2-methyl-6-oxo-4-(1,2,4,5-tetrahydro-benzo[d]azepin-3-yl)-1,6-dihydro-pyrimidine-5-carbonitrile (example 17) was treated with the 1-chloro-1,1-difluoromethane in N,N-dimethylformamide in the presence of potassium carbonate at 145 °C in an autoclave for 30 minutes to yield the 1-difluoromethyl-2-methyl-6-oxo-4-(1,2,4,5-tetrahydro-benzo[d]azepin-3-yl)-1,6-dihydro-pyrimidine-5-carbonitrile as yellowish solid; m.p. 217 °C; MS: [M]⁺ = 330; and the 4-difluoromethoxy-2-methyl-6-(1,2,4,5-tetrahydro-benzo[d]azepin-3-yl)-pyrimidine-5-carbonitrile as colorless solid; m.p. 143 °C; MS: [M+H]⁺ = 331.

### Example 272

### 1-Difluoromethoxy-2-methyl-6-oxo-4-(1,2,4,5-tetrahydro-benzo[d]azepin-3-yl)-1,6-dihydro-pyrimidine-5-carbonitrile

In analogy to the procedure described in example 3 the 1-hydroxy-2-methyl-6-oxo-4-(1,2,4,5-tetrahydro-benzo[d]azepin-3-yl)-1,6-dihydro-pyrimidine-5-carbonitrile (example 199) was treated with the 1-chloro-1,1-difluoromethane in N,N-dimethylformamide in the presence of potassium carbonate at 50 °C in an autoclave for 60 hours to yield the 1-difluoromethoxy-2-methyl-6-oxo-4-(1,2,4,5-tetrahydro-benzo[d]azepin-3-yl)-1,6-dihydro-pyrimidine-5-carbonitrile as colorless amorphous solid; MS: [M]⁺ = 346.

### Example 273

### 1-Ethyl-2-methyl-6-oxo-4-(1,1,2-tritritio-1,2,4,5-tetrahydro-benzo[d]azepin-3-yl)-1,6-dihydro-pyrimidine-5-carbonitrile

The 1-ethyl-2-methyl-6-oxo-4-(1,1,2-tritritio-1,2,4,5-tetrahydro-benzo[d]azepin-3-yl)-1,6-dihydro-pyrimidine-5-carbonitrile has been prepared from the ethyl 2-cyano-3,3-bis(methylthio)acrylate and the 1,1,2-tritritio-2,3,4,5-tetrahydro-1H-benzo[d]azepine as described in examples 13 a), 17 and 27/28. The 1,1,2-tritritio-2,3,4,5-tetrahydro-1H-benzo[d]azepine was obtained by the following sequence:
i) reaction of the 1-(1,2,4,5-tetrahydro-benzo[d]azepin-3-yl)-ethanone [J. Heterocycl. Chem. (1971), 8(5), 779-83] with dibenzoylperoxide and N-bromosuccinimide in carbon tetrachloride at reflux yielded the 1-(5-bromo-1,2-dihydro-benzo[d]azepin-3-yl)-ethanone;
ii) hydrogenation of the 1-(5-bromo-1,2-dihydro-benzo[d]azepin-3-yl)-ethanone with tritium using Pd/C in methanol in the presence of triethylamine yielded the 1-(1,1,2-tritritio-1,2,4,5-tetrahydro-benzo[d]azepin-3-yl)-ethanone;
iii) treatment of the 1-(1,1,2-tritritio-1,2,4,5-tetrahydro-benzo[d]azepin-3-yl)-ethanone with conc. aq. hydrochloric acid in methanol gave the 1,1,2-tritritio-2,3,4,5-tetrahydro-1H-benzo[d]azepine.

### Example A

Tablets of the following composition are produced in a conventional manner:

| | mg/Tablet |
|---|---|
| Active ingredient | 100 |
| Powdered lactose | 95 |
| White corn starch | 35 |
| Polyvinylpyrrolidone | 8 |
| Na carboxymethylstarch | 10 |
| Magnesium stearate | 2 |
| | Tablet weight 250 |

### Example B

Tablets of the following composition are produced in a conventional manner:

| | mg/Tablet |
|---|---|
| Active ingredient | 200 |
| Powdered lactose | 100 |
| White corn starch | 64 |
| Polyvinylpyrrolidone | 12 |
| Na carboxymethylstarch | 20 |
| Magnesium stearate | 4 |
| | Tablet weight 400 |

### Example C

Capsules of the following composition are produced:

| | mg/Capsule |
|---|---|
| Active ingredient | 50 |
| Crystalline lactose | 60 |
| Microcrystalline cellulose | 34 |
| Talc | 5 |
| Magnesium stearate | 1 |
| | Capsule fill weight 150 |

The active ingredient having a suitable particle size, the crystalline lactose and the microcrystalline cellulose are homogeneously mixed with one another, sieved and thereafter talc and magnesium stearate are admixed. The final mixture is filled into hard gelatine capsules of suitable size.

## Claims

1. Compounds of the general formula 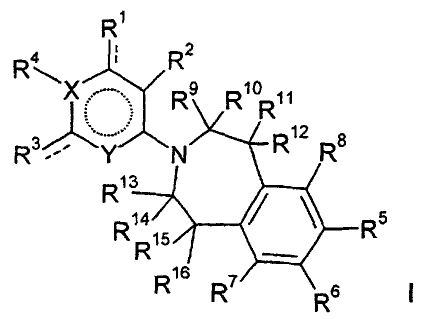 wherein
R¹ signifies hydrogen, C₁₋₇ alkyl, oxygen, halogen, or
-OR, -O(C₃-C₆)cycloalkyl, -O(CHR)ₙ-(C₃-C₆)cycloalkyl, -O(CHR)ₙCN, -O(CHR)ₙCF₃, -O(CHR)(CHR)ₙNR₂, -O(CHR)(CHR)ₙOR, -O(CHR)ₙ-C₂₋₇ alkenyl, -OCF₃, -OCF₂-R, -OCF₂-C₂₋₇, -OCHRF, -OCHF-C₂₋₇ alkenyl, -OCF₂CRF₂, -OCF₂Br, -O(CHR)ₙCF₂Br, -O(CHR)ₙ-phenyl, wherein the phenyl group may be optionally substituted independently from each other by one to three C₁₋₇ alkyl, C₁₋₇ alkoxy, halogen, nitro or cyano groups, -O(CHR)(CHR)ₙ-morpholino, -O(CHR)(CHR)ₙ-pyrrolidino, -O(CHR)(CHR)ₙ-piperidino, -O(CHR)(CHR)ₙ-imidazolo, -O(CHR)(CHR)ₙ-triazolo, -O(CHR)ₙ-pyridino, -O(CHR)(CHR)ₙ-OSi-lower alkyl, -O(CHR)(CHR)ₙOS(O)₂-C₁₋₇ alkyl, -O(CH₂)ₙCH=CF₂, -O(CHR)ₙ-2,2-dimethyl-[1.3]dioxolane, -O(CHR)ₙ-CHOR-CH₂OR, -O(CHR)ₙ-CHOR-(CHR)ₙ-CH₂OR or
- SR or -S(CHR)ₙCOOR, or
-NR², -N(R)(CHR)(CHR)ₙOR, -N(R)(CHR)ₙCF₃, -N(R)(CHR)(CHR)ₙ-morpholino, -N(R)(CHR)(CHR)ₙ-imidazolo, -N(R)(CHR)(CHR)ₙ-pyrrolidino, -N(R)(CHR)(CHR)ₙ-pyrrolidin-2-one, -N(R)(CHR)(CHR)ₙ-piperidino, -N(R)(CHR)(CHR)ₙ-triazolo, -N(R)(CHR)ₙ-pyridino, or
n is 1- 6;
R signifies hydrogen, C₁₋₇ alkyl or C₂₋₇ alkenyl, independently from each other, if more than one R is present;
R² signifies nitro or cyano;
R³ signifies hydrogen, C₁₋₇ alkyl, =O, =S, -SR, -S(O)₂-C₁₋₇ alkyl, -(C₃-C₆)cycloalky or piperazino, optionally substituted by C₁₋₇ alkyl, or
-CONR₂, -(CHR)ₙCONR₂, -(CHR)ₙOR, -(CH₂)ₙ-CF₃, -CF₃, -(CHR)ₙOC(O)CF₃, -(CHR)ₙCOOR, -(CHR)ₙSC₆H₅, wherein the phenyl group may be optionally substituted independently from each other by one to three C₁₋₇ alkyl, C₁₋₇ alkoxy, halogen, nitro or cyano groups, -(CHR)ₙ-1,3-dioxo-1,3-dihydro-isoindol, -(CHR)ₙ-tetrahydro-pyran-2-yloxy or -(CHR)ₙ-S-C₁₋₇ alkyl, or
-NR₂, -NRCO-C₁₋₇ alkyl, -NRCHO, -N(R)(CHR)ₙCN, -N(R)(CHR)ₙCF₃, -N(R)(CHR)(CHR)ₙ-OR, -N(R)C(O)(CHR)ₙO-C₁₋₇ alkyl, -NR(CHR)ₙ C₁₋₇ alkyl, -NR(CHR)(CHR)ₙ-OR, -N(R)(CHR)(CHR)ₙ-O-phenyl, wherein the phenyl group may be optionally substituted independently from each other by one to three C₁₋₇ alkyl, C₂₋₇ alkoxy, halogen, nitro or cyano groups, -N(R)(CHR)ₙ-C₂₋₇ alkenyl, -N(R)(CHR)(CHR)ₙ-O-(CHR)ₙOR, -N(R)(CHR)ₙC(O)O-C₁₋₇ alkyl, -N(R)(CHR)ₙC(O)NR-C₁₋₇ alkyl, -N(R)(CH₂)ₙ-2,2-dimethyl-[1.3]dioxolane, -N(R)(CHR)(CHR)ₙmorpholino, -N(R)(CHR)ₙ-pyridino, -N(R)(CHR)(CHR)ₙ-piperidino, -N(R)(CHR)(CHR)ₙ-pyrrolidino, -N(R)(CHR)(CHR)ₙ-O-pyridino, -N(R)(CHR)(CHR)ₙimidazolo, -N(R)(CHR)ₙ-CR₂-(CHR)ₙ-OR, -N(R)(CHR)ₙ-CR₂-OR, -N(R)(CHR)ₙ-CHOR-CH₂OR, -N(R)(CHR)ₙ-CHOR-(CHR)ₙ-CH₂OR, or
-OR, -O(CHR)ₙCF₃, -OCF₃, -O(CHR)(CHR)ₙ-O-phenyl, wherein the phenyl group may be optionally substituted independently from each other by one to three C₁₋₇ alkyl, C₁₋₇ alkoxy, halogen, nitro or cyano groups, -O(CHR)(CHR)ₙ-O-C₁₋₇ alkyl, -O(CHR)ₙ-pyridino or -O(CHR)(CHR)ₙ-morpholino; and
R⁴ signifies hydrogen, C₁₋₇ alkyl, C₂₋₇ alkenyl or nitro, or -OR, -OCF₃, -OCF₂-R, -OCF₂-C₂₋₇ alkenyl, -OCHRF, -OCHF-C₂₋₇ alkenyl, -O(CHR)ₙCF₃, or
-(CHR)ₙCHRF, -(CHR)ₙCF₂R, -(CHR)ₙCF₃, -(C₃-C₆)cycloalkyl, -(CHR)ₙ(C₃-C₆)cycloalkyl, -(CHR)ₙCN, -(CHR)ₙ-phenyl, wherein the phenyl group may be optionally substituted independently from each other by one to three C₁₋₇ alkyl, C₁₋₇ alkoxy, halogen, nitro or cyano groups, -(CHR)(CHR)ₙOR, -(CHR)ₙCHORCH₂OR, -(CHR)(CHR)ₙNR₂, -(CHR)ₙCOOR, -(CHR)(CHR)ₙOSi-C₁₋₇ alkyl, -(CHR)(CHR)ₙ-OS(O)₂-C₁₋₇ alkyl, -(CH₂)ₙ-CH=CF₂, -CF₃, -CF₂-R, -CF₂-lower alkenyl, -CHRF, -CHF-C₂₋₇ alkenyl, -(CHR)ₙ-2,2-dimethyl-[1.3]dioxolane, -(CH₂)ₙ-2-oxo-azepan-1-yl, -(CHR)(CHR)ₙ-morpholino, -(CHR)ₙ-pyridino, -(CHR)(CHR)ₙ-imidazolo, -(CHR)(CHR)ₙ-triazolo, -(CHR)(CHR)ₙ-pyrrolidino, optionally substituted by -(CH₂)ₙOH, -(CHR)(CHR)ₙ-3-hydroxy-pyrrolidino or -(CHR)(CHR)ₙ-piperidino, or
-NR₂, -N(R)(CHR)ₙ-pyridino, -N(R)C(O)O-C₁₋₇ alkyl, -N(CH₂CF₃)C(O)O-C₁₋₇ alkyl, -N[C(O)O-C₁₋₇ alkyl]₂, -NR-NR-C(O)O-C₁₋₇ alkyl or -N(R)(CHR)ₙCF₃, -NRCF₃, -NRCF₂-R, -NRCF₂-C₂₋₇ alkenyl, -NRCHRF, -NRCHF-C₂₋₇ alkenyl;
or is absent, if X is -N= or =N-;
or R⁴ and R¹ or R³ and R⁴ are interconnected to the groups -(CH₂)₃₋₅-, -(CH₂)₂-N=, -CH=N-N=-, -CH=CH-N=, -NH-CH=CH- or -NR-CH₂-CH₂- and form together with the N and C atoms to which they are attached an additional ring;
R⁵, R⁶ signify hydrogen, C₁₋₇ alkyl, C₁₋₇ alkoxy, amino, nitro, -SO₂NH₂ or halogen; or
R⁵ and R⁶ are interconnected to the group -O-CH₂-O- and form together with the C atoms to which they are attached an additional 5-membered ring;
R⁷, R⁸ signify hydrogen, C₁₋₇ alkyl, C₁₋₇ alkoxy, amino, nitro or halogen;
R⁹, R¹⁰ signify hydrogen or C₁₋₇ alkyl;
R¹¹, R¹² signifies hydrogen, C₁₋₇ alkyl, hydroxy, C₁₋₇ alkoxy, lower alkoxycarbonyloxy or C₁₋₇ alkanoyloxy;
R¹³, R¹⁴ signify hydrogen, tritium or C₁₋₇ alkyl;
R¹⁵, R¹⁶ signifies hydrogen, tritium, C₁₋₇ alkyl, hydroxy, C₁₋₇ alkoxy, C₁₋₇ alkoxycarbonyloxy or C₁₋₇ alkanoyloxy or are together an oxo group; or
X signifies -N=, =N-, -N<, >C= or =C<;
Y signifies -N=, =N-, -NH-, -CH= or =CH-; and
the dotted line may be a bond,
as well as their pharmaceutically acceptable salts in their racemic and optically active form.

2. Compounds in accordance with claim 1, wherein R¹ is =O or hydroxy and R² is NO₂ and pharmaceutically acceptable salts thereof.

3. Compounds in accordance with claims 1 and 2, which are
3-ethyl-2-methyl-5-nitro-6-(1,2,4,5-tetrahydro-benzo[d]azepin-3-yl)-3H-pyrimidin-4-one,
3-(2-fluoro-ethyl)-2-methyl-5-nitro-6-(1,2,4,5-tetrahydro-benzo[d]azepin-3-yl)-3H-pyrimidin-4-one,
2-methyl-5-nitro-6-(1,2,4,5-tetrahydro-benzo[d]azepin-3-yl)-3-(2,2,2-trifluoro-ethyl)-3H-pyrimidin-4-one or
2-methyl-5-nitro-6-(1,2,4,5-tetrahydro-benzo[d]azepin-3-yl)-pyrimidin-4-ol.

4. Compounds in accordance with claim 1, wherein R¹ is =O and R² is -CN and pharmaceutically acceptable salts thereof.

5. Compounds in accordance with claims 1 and 4, which are
2-amino-6-oxo-4-(1,2,4,5-tetrahydro-benzo[d]azepin-3-yl)-1,6-dihydro-pyrimidine-5-carbonitrile,
6-oxo-4-(1,2,4,5-tetrahydro-benzo[d]azepin-3-yl)-1,6-dihydro-pyrimadine-5-carbonitrile,
2-ethylamino-6-oxo-4-(1,2,4,5-tetrahydro-benzo[d]azepin-3-yl)-1,6-dihydro-pyrimidine-5-carbonitrile,
1,2-dimethyl-6-oxo-4-(1,2,4,5-tetrahydro-benzo[d] azepin-3-yl)-1,6-dihydro-pyrimidine-5-carbonitrile,
1-ethyl-2-methyl-6-oxo-4-(1,2,4,5-tetrahydro-benzo[d]azepin-3-yl)-1,6-dihydro-pyrimidine-5-carbonitrile,
2-amino-1-ethyl-6-oxo-4-(1,2,4,5-tetrahydro-benzo[d]azepin-3-yl)-1,6-dihydro-pyrimidine-5-carbonitrile,
1-cyclopropylmethyl-2-methyl-6-oxo-4-(1,2,4,5-tetrahydro-benzo[d]azepin-3-yl)-1,6-dihydro-pyrimidine-5-carbonitrile,
1-allyl-2-methyl-6-oxo-4-(1,2,4,5-tetrahydro-benzo[d]azepin-3-yl)-1,6-dihydro-pyrimidine-5-carbonitrile,
1-cyanomethyl-2-methyl-6-oxo-4-(1,2,4,5-tetrahydro-benzo[d]azepin-3-yl)-1,6-dihydro-pyrimidine-5-carbonitrile,
1-(2-dimethylamino-ethyl)-2-methyl-6-oxo-4-(1,2,4,5-tetrahydro-benzo[d]azepin-3-yl)-1,6-dihydro-pyrimidine-5-carbonitrile,
1-isopropyl-6-oxo-4-(1,2,4,5-tetrahydro-benzo[d]azepin-3-yl)-1,6-dihydro-pyrimidine-5-carbonitrile,
1-(2-hydroxy-ethyl)-6-oxo-4-(1,2,4,5-tetrahydro-benzo[d]azepin-3-yl)-1,6 dihydro-pyrimidine-5-carbonitrile,
2-(2-hydroxy-ethyl)-6-oxo-4-(1,2,4,5-tetrahydro-benzo[d]azepin-3-yl)-1,6 dihydro-pyrimidine-5-carbonitrile,
2-methyl-6-oxo-4-(1,2,4,5-tetrahydro-benzo[d]azepin-3-yl)-1-(2,2,2-trifluoro-ethoxy)-1,6-dihydro-pyrimidine-5-carbonitrile,
2-methyl-1-methylamino-6-oxo-4-(1,2,4,5-tetrahydro-benzo[d]azepin-3-yl)-1,6-dihydro-pyrimidine-5-carbonitrile or
1-amino-2-methyl-6-oxo-4-(1,2,4,5-tetrahydro-benzo[d]azepin-3-yl)-1,6-dihydro-pyrimidine-5-carbonitrile.

6. Compounds in accordance with claim 1, wherein R¹ is 2,2,2-trifluoroethoxy and R² is -CN.

7. Compounds in accordance with claims 1 and 6, which are
2-(2-morpholin-4-yl-ethylamino)-4-(1,2,4,5-tetrahydro-benzo[d]azepin-3-yl)-6-(2,2,2-trifluoro-ethoxy)-pyrimidine-5-carbonitrile,
2-(3-morpholin-4-yl-propylamino)-4-(1,2,4,5-tetrahydro-benzo[d]azepin-3-yl)-6-(2,2,2-trifluoro-ethoxy)-pyrimidine-5-carbonitrile,
2-(2-hydroxy-ethylamino)-4-(1,2,4,5-tetrahydro-benzo[d]azepin-3-yl)-6-(2,2,2-trifluoro-ethoxy)-pyrimidine-5-carbonitrile or
(3-imidazol-1-yl-propylamino)-4-(1,2,4,5-tetrahydro-benzo[d]azepin-3-yl)-6-(2,2,2-trifluoro-ethoxy)-pyrimidine-5-carbonitrile.

8. Compounds in accordance with claim 1, wherein R¹ is 3-[1,2,4]triazol-1-yl-propoxy and R² is -NO₂ or -CN.

9. A compound in accordance with claims 1 and 8, which is
3-[2-methyl-5-nitro-6-(3-[1,2,4]triazol-1-yl-propoxy)-pyrimidin-4-yl]-2,3,4,5-tetrahydro-1H-benzo[d]azepine.

10. A compound in accordance with claim 1, wherein R³ and R⁴ are interconnected to the groups -(CH₂)₃₋₅- to form together with the N and C atoms to which they are attached an additional ring and R² is -NO₂ or -CN.

11. A compound in accordance with claims 1 and 10, which is
4-oxo-2-(1,2,4,5-tetrahydro-benzo[d]azepin-3-yl)-4,6,7,8,9,10-hexahydro-pyrimido[1,2-a] azepine-3-carbonitrile.

12. A medicament comprising a compound according to any one of claims 1-11 as well as pharmaceutically acceptable salts thereof in their racemic and optically active form and pharmaceutically acceptable excipients.

13. A medicament in accordance with claim 12 for the control or prevention of acute and/or chronic neurological disorders such as epilepsy, stroke, chronic and acute pain, psychosis, schizophrenia, Alzheimer's disease cognitive disorders, memory deficits, restricted brain function caused by bypass operations or transplants, poor blood supply to the brain, spinal cord injuries, head injuries, hypoxia caused by pregnancy, cardiac arrest, hypoglycaemia, Huntington's chorea, ALS, dementia caused by AIDS, eye injuries, retinopathy, idiopathic parkinsonism or parkinsonism caused by medicaments as well as conditions which lead to glutamate deficiency functions, such as e.g. muscle spasms, convulsions, migraine, urinary incontinence, nicotine addiction, psychoses, opiate addiction, anxiety, vomiting, dyskinesia and depression.

14. Compounds in accordance with any one of claims 1-11 as well as pharmaceutically acceptable salts thereof in their racemic and optically active form for use in the control or prevention of illness.

15. The use of compounds of formula I in accordance with any one of claims 1-11 as well as pharmaceutically acceptable salts thereof in their racemic and optically active form for the manufacture of medicaments for the control or prevention of acute and/or chronic neurological disorders such as epilepsy, stroke, chronic and acute pain, psychosis, schizophrenia, Alzheimer's disease cognitive disorders, memory deficits, restricted brain function caused by bypass operations or transplants, poor blood supply to the brain, spinal cord injuries, head injuries, hypoxia caused by pregnancy, cardiac arrest, hypoglycaemia, Huntington's chorea, ALS, dementia caused by AIDS, eye injuries, retinopathy, idiopathic parkinsonism or parkinsonism caused by medicaments as well as conditions which lead to glutamate deficiency functions, such as e.g. muscle spasms, convulsions, migraine, urinary incontinence, nicotine addiction, psychoses, opiate addiction, anxiety, vomiting, dyskinesia and depression.

16. A compound of formula I in accordance with claims 1 - 11 as well as pharmaceutically acceptable salts thereof for the control or prevention of acute and/or chronic neurological disorders.

17. A process for the manufacture of compounds of formula I according to any one of claim 1-11 as well as of pharmaceutically acceptable salts thereof, which process comprises
a) reacting a compound of the formula 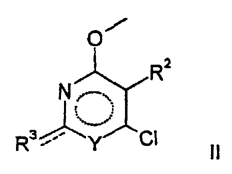 with a compound of formula 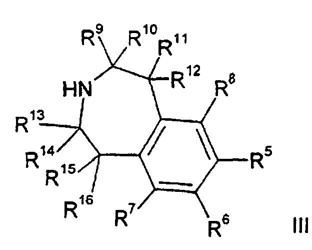 to a compound of formula 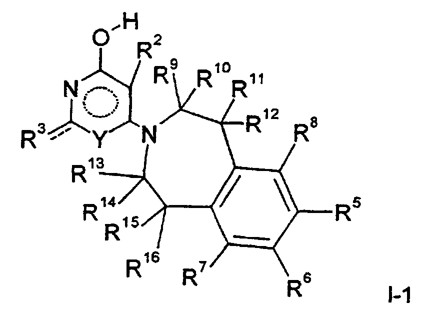 wherein the substituents are described above, or
b) reacting a compound of the formula 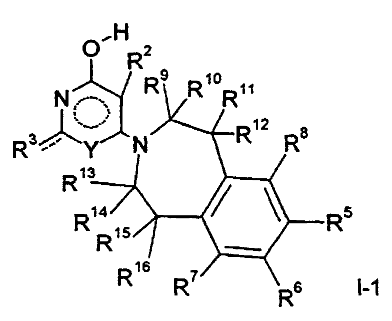 to a compound of formula 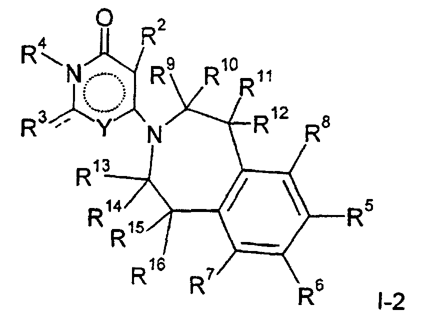 or to a compound of formula 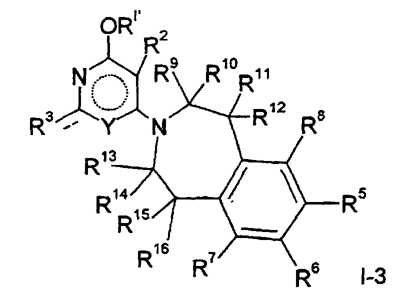 wherein R² to R¹⁶ have the significances given above and R^{I'} is lower alkyl, -(C₃-C₆)cycloalkyl, -(CHR)ₙ-(C₃-C₆)cycloalkyl, -(CHR)ₙCN, -(CHR)ₙCF₃, -(CHR)(CHR)ₙNR₂, -(CHR)(CHR)ₙOR, -(CHR)ₙ-lower alkenyl, -CF₃, -CF₂-R, -CF₂-lower alkenyl, -CHRF, -CHF-lower alkenyl, -CF₂CRF₂, -CF₂Br, -(CHR)ₙCF₂Br, -(CHR)ₙ-phenyl, wherein the phenyl group may be optionally substituted independently from each other by one to three lower alkyl, lower alkoxy, halogen, nitro or cyano groups, -(CHR)(CHR)ₙ-morpholino, -(CHR)(CHR)ₙ-pyrrolidino, -(CHR)(CHR)ₙ-piperidino, -(CHR)(CHR)ₙ-imidazolo, -(CHR)(CHR)ₙ-triazolo, -(CHR)ₙ-pyridino, -(CHR)(CHR)ₙ-OSi-lower alkyl, -(CHR)(CHR)ₙOS(O)₂-lower alkyl, -(CH₂)ₙCH=CF₂, -(CHR)ₙ-2,2-dimethyl-[1.3]dioxolane, -(CHR)ₙ-CHOR-CH₂OR or -(CHR)ₙ-CHOR-(CHR)ₙ-CH₂OR.
c) reacting a compound of formula 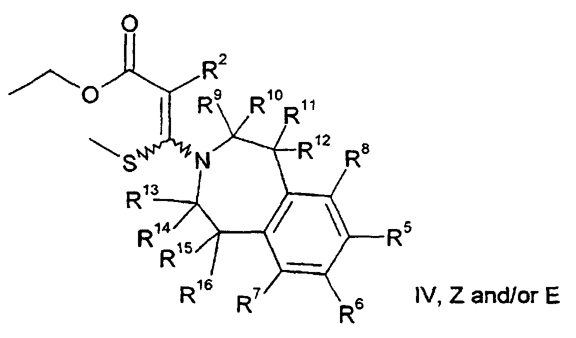 with a compound of formula 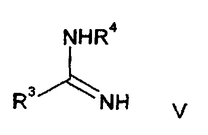 to a compound of formula 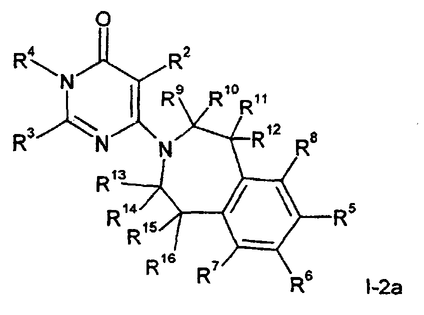 wherein the substituents are described above,
or
d) reacting a compound of formula 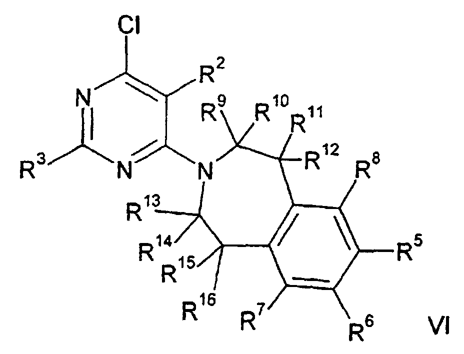 with a an alcohol, thiol, a primary or secondary amine to a compound of formula 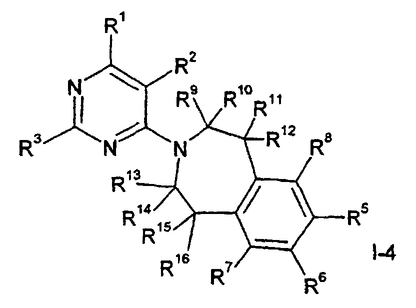 wherein the substituents are given above, or
e) reacting a compound of formula 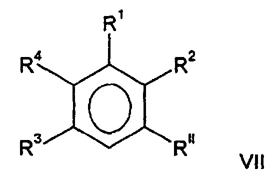 wherein R^{II} in formula VII is fluoro, chloro, bromo or a trifluoro-methansulfonyloxy group,
with a compound of formula 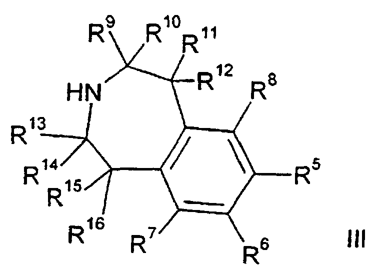 to a compound of formula 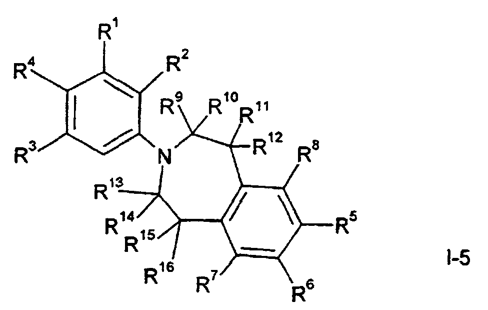 wherein the substituents are described above,
f) reacting a compound of formula 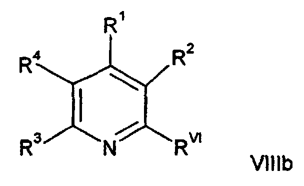 wherein, R^{VI} is a fluoro, chloro, bromo or a trifluoro-methansulfonyloxy group, with a compound of formula 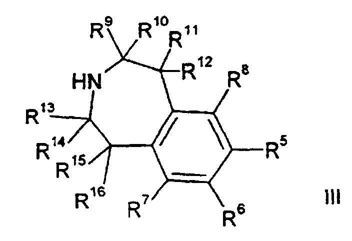 to a compound of formula I-5, 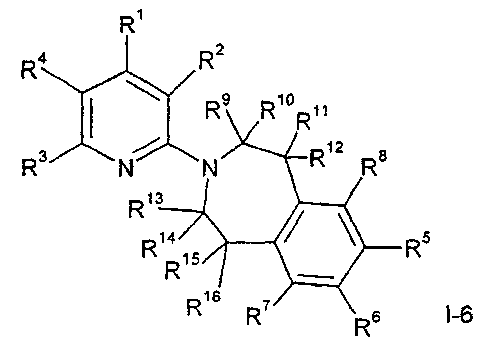 wherein the substituents are described above,
and, if desired,
introducing and removing protective groups in compounds of formula I, alkylating OH or NH functions in compounds of formula I, cleaving ether functions, converting a functional group in a compound of formula I into another functional group directly or via a suitable activating group and, if desired,
converting a compound of formula I into a pharmaceutically acceptable salt or into its optically active form.

18. Compounds of formula I in accordance with claims 1 - 11, when manufactured according to a process in accordance with claim 17.

19. The use of a radiolabeled mGluR1 receptor antagonist of formula I in accordance with claim 1 in an in vitro binding assay.

20. The use of a radiolabeled mGluR1 receptor antagonist of formula I in an in vitro binding assay in accordance with claim 19, which is
1-ethyl-2-methyl-6-oxo-4-(1,1,2-tritritio-1,2,4,5-tetrahydro-benzo[d]azepin-3-yl)-1,6-dihydro-pyrimidine-5-carbonitrile

## Patentansprüche

1. Verbindungen der allgemeinen Formel 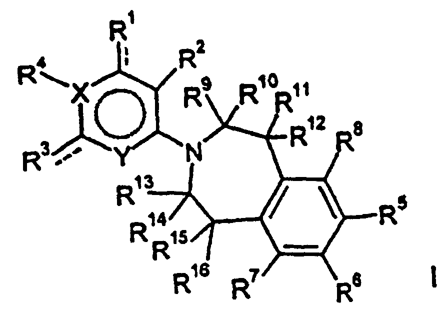 wobei
R¹ Wasserstoff, C₁₋₇ Alkyl, Sauerstoff, Halogen oder -OR, -O(C₃-C₆)cycloalkyl, -O(CHR)ₙ-(C₃-C₆)cycloalkyl, -O(CHR)ₙCN, -O(CHR)ₙCF₃, -O(CHR)(CHR)ₙNR₂, -O(CHR)(CHR)ₙOR, -O(CHR)ₙ-C₂₋₇ alkenyl, -OCF₃, -OCF₂-R, -OCF₂-C₂₋₇ alkenyl, -OCHRF, -OCHF- C₂₋₇ alkenyl, -OCF₂CRF₂, -OCF₂Br, -O(CHR)ₙCF₂Br, -O(CHR)ₙ-phenyl, wobei die Phenylgruppe wahlweise substituiert sein kann unabhängig voneinander mit 1 bis 3 C₁₋₇ Alkyl-, C₁₋₇ Alkoxy-, Halogen-, Nitro- oder Cyanogruppen,
-O(CHR)(CHR)ₙ-morpholino, -O(CHR)(CHR)ₙ-pyrrolidino, -O(CHR)(CHR)ₙ-piperidino, -O(CHR)(CHR)ₙ-imidazolo, -O(CHR)(CHR)ₙ-triazolo, -O(CHR)ₙ-pyridino, -O(CHR)(CHR)ₙ-OSi-nieder Alkyl, -O(CHR)(CHR)ₙ-OS(O)₂-C₁₋₇ alkyl, -O(CH₂)ₙCH=CF₂, -O(CHR)ₙ-2,2-dimethyl-[1,3]dioxolan, -O(CHR)ₙ-CHOR-CH₂OR, -O(CHR)ₙ-CHOR-(CHR)ₙ-CH₂OR oder
- SR oder -S(CHR)ₙCOOR, oder
-NR², -N(R)(CHR)(CHR)ₙOR, -N(R)(CHR)ₙCF₃, -N(R)(CHR)(CHR)ₙ-morpholino, -N(R)(CHR)(CHR)ₙ-imidazolo, -N(R)(CHR)(CHR)ₙ-pyrrolidino, -N(R)(CHR)(CHR)ₙ-pyrrolidin-2-on, -N(R)(CHR)(CHR)ₙ-piperidino, -N(R)(CHR)(CHR)ₙ-triazolo, -N(R)(CHR)ₙ-pyridino bedeutet,
n 1 - 6 ist;
R Wasserstoff, C₁₋₇ Alkyl, oder C₂₋₇ Alkenyl bedeutet, unabhängig voneinander, wenn mehr als ein R anwesend ist;
R² Nitro oder Cyano bedeutet;
R³ Wasserstoff, C₁₋₇ Alkyl, =O, =S, -SR, -S(O)₂-C₁₋₇ alkyl, -(C₃-C₆)cycloalkyl oder piperazino, wahlweise substituiert mit C₁₋₇ Alkyl, oder
-CONR₂, -(CHR)ₙCONR₂, -(CHR)ₙOR, -(CH₂)ₙ-CF₃, -CF₃, -(CHR)ₙOC(O)CF₃, -(CHR)ₙCOOR, -(CHR)ₙSC₆H₅, wobei die Phenylgruppe wahlweise substituiert sein kann unabhängig voneinander mit 1 bis 3 C₁₋₇ Alkyl-, C₁₋₇ Alkoxy-, Halogen-, Nitro- oder Cyanogruppen, -(CHR)ₙ-1,3-dioxo-1,3-dihydro-isoindol, -(CHR)ₙ-tetrahydro-pyran-2-yloxy, oder -(CHR)ₙ-S-C₁₋₇ alkyl, oder
-NR₂, -NRCO-C₁₋₇ alkyl, NRCHO, -N(R)(CHR)ₙCN, -N(R)(CHR)ₙCF₃, -N(R)(CHR)(CHR)ₙ-OR, -N(R)C(O)(CHR)ₙO-C₁₋₇ alkyl, -N(R)(CHR)ₙ-C₁₋₇ alkyl, -NR(CHR)(CHR)ₙ-OR, -N(R)(CHR)(CHR)ₙ-O-phenyl, wobei die Phenylgruppe wahlweise substituiert sein kann unabhängig voneinander mit 1 bis 3 C₁₋₇ Alkyl-, C₁₋₇ Alkoxy-, Halogen-, Nitro- oder Cyanogruppen, -N(R)(CHR)ₙ-C₂-₇ alkenyl, -N(R)(CHR)(CHR)ₙ-O-(CHR)ₙOR, -N(R)(CHR)ₙC(O)O-C₁₋₇ alkyl, -N(R)(CHR)ₙC(O)NR-C₁₋₇ alkyl, -N(R)(CH₂)ₙ-2,2-dimethyl-[1,3]dioxolan, -N(R)(CHR)(CHR)ₙ-morpholino, -N(R)(CHR)ₙ-pyridino, -N(R)(CHR)(CHR)ₙ-piperidino, -N(R)(CHR)(CHR)ₙ-pyrrolidino, -N(R)(CHR)(CHR)ₙ-O-pyridino, -N(R)(CHR)(CHR)ₙ-imidazolo, -N(R)(CHR)ₙ-CR₂-(CHR)ₙ-OR, -N(R)(CHR)ₙ-CR₂-OR, -N(R)(CHR)ₙ-CHOR-CH₂OR, -N(R)(CHR)ₙ-CHOR-(CHR)ₙ-CH₂OR, oder
-OR, -O(CHR)ₙCF₃, -OCF₃, -O(CHR)(CHR)ₙ-O-phenyl, wobei die Phenylgruppe wahlweise substituiert sein kann unabhängig voneinander mit 1 bis 3 C₁₋₇ Alkyl-, C₁₋₇ Alkoxy-, Halogen-, Nitro- oder Cyanogruppen, -O(CHR)(CHR)ₙ-O-C₁₋₇ alkyl, -O(CHR)ₙ-pyridino, oder -O(CHR)(CHR)ₙ-morpholino bedeutet; und
R⁴ Wasserstoff, C₁₋₇ Alkyl, C₂₋₇ Alkenyl, oder Nitro, oder -OR, -OCF₃, -OCF₂-R, -OCF₂-C₂₋₇ alkenyl, -OCHRF, -OCHF- C₂₋₇ alkenyl, -O(CHR)ₙCF₃, oder
-(CHR)ₙCHRF, -(CHR)ₙCF₂R, -(CHR)ₙCF₃, -(C₃-C₆)cycloalkyl, -(CHR)ₙ(C₃-C₆)cycloalkyl, -(CHR)ₙCN, -(CHR)ₙ-phenyl, wobei die Phenylgruppe wahlweise substituiert sein kann unabhängig voneinander mit 1 bis 3 C₁₋₇ Alkyl-, C₁₋₇ Alkoxy-, Halogen-, Nitro- oder Cyanogruppen, -(CHR)(CHR)ₙOR, -(CHR)ₙCHORCH₂OR, -(CHR)(CHR)ₙNR₂, -(CHR)ₙCOOR, -(CHR)(CHR)ₙ-OSi-C₁₋₇ alkyl, -(CHR)(CHR)ₙ-OS(O)₂-C₁₋₇ alkyl, -(CH₂)ₙCH=CF₂, -CF₃, CF₂-R, -CF₂-nieder alkenyl, -CHRF, -CHF-C₂₋₇ alkenyl, -(CHR)ₙ-2,2-dimethyl-[1,3]dioxolan, -(CH₂)ₙ-2-oxo azepan-1-yl, -(CHR)(CHR)ₙ-morpholino, -(CHR)ₙ-pyridino, -(CHR)(CHR)ₙ-imidazolo, -(CHR)(CHR)ₙ-triazolo, -(CHR)(CHR)ₙ-pyrrolidino, wahlweise substituiert mit -(CH₂)ₙOH, -(CHR)(CHR)ₙ-3-hydroxy-pyrrolidino oder -(CHR)(CHR)ₙ-piperidino, oder
-NR₂, -N(R)(CHR)ₙ-pyridino, -N(R)C(O)O-C₁₋₇ alkyl, -N(CH₂CF₃)C(O)O-C₁₋₇ alkyl, -N[C(O)O-C₁₋₇ alkyl]₂, -NR-NR-C(O)O-C₁₋₇ alkyl oder -N(R)(CHR)ₙCF₃, -NRCF₃, -NRCF₂-R, -NRCF₂-C₂₋₇ alkenyl, -NRCHRF, -NRCHF-C₂₋₇ alkenyl bedeutet;
oder abwesend ist, wenn X -N= oder =N- ist;
oder R⁴ und R¹ oder R³ und R⁴ miteinander zu den Gruppen -(CH₂)₃₋₅-, -(CH₂)₂- N=, -CH=N-N=-, -CH=CH-N=, -NH-CH=CH- oder -NR-CH₂-CH₂- verbunden sind und zusammen mit den N und C-Atomen an die sie gebunden sind, einen zusätzlichen Ring formen;
R⁵, R⁶ Wasserstoff, C₁₋₇ Alkyl, C₁₋₇ Alkoxy, Amino, Nitro, -SO₂NH₂ oder Halogen bedeuten; oder
R⁵ und R⁶ miteinander zu der Gruppe -O-CH₂-O- verbunden sind und zusammen mit den C-Atomen an die sie gebunden sind einen zusätzlichen 5-gliedrigen Ring formen;
R⁷, R⁸ Wasserstoff, C₁₋₇ Alkyl, C₁₋₇ Alkoxy, Amino, Nitro oder Halogen bedeuten;
R⁹, R¹⁰ Wasserstoff oder C₁₋₇ Alkyl bedeuten;
R¹¹, R¹² Wasserstoff, C₁₋₇ Alkyl, Hydroxy, C₁₋₇ Alkoxy, nieder Alkoxycarbonyloxy oder C₁₋₇ Alkanoyloxy bedeuten;
R¹³, R¹⁴ Wasserstoff, Tritium oder C₁₋₇ Alkyl bedeuten;
R¹⁵, R¹⁶ Wasserstoff, Tritium, C₁₋₇ Alkyl, Hydroxy, C₁₋₇ Alkoxy, C₁₋₇ Alkoxycarbonyloxy oder C₁₋₇ Alkanoyloxy bedeuten oder zusammen eine Oxogruppe sind;
X -N=, =N-, -N<, >C=, oder =C< bedeutet;
Y -N=, =N-, -NH-, -CH= oder =CH- bedeutet; und
die gestrichelte Linie eine Bindung sein kann,
sowie wie ihre pharmazeutisch annehmbaren Salze in ihrer racemischen und optisch aktiven Form.

2. Verbindungen nach Anspruch 1, wobei R¹ =O oder Hydroxy ist und R² NO₂ ist und pharmazeutisch annehmbare Salze davon.

3. Verbindungen nach den Ansprüchen 1 und 2, welche
3-Ethyl-2-methyl-5-nitro-6-(1,2,4,5-tetrahydrobenzo[d]azepin-3-yl)-3H-pyrimidin-4-on,
3-(2-Fluorethyl)-2-methyl-5-nitro-6-(1,2,4,5-tetrahydrobenzo[d]azepin-3-yl)-3H-pyrimidin-4-on,
2-Methyl-5-nitro-6-(1,2,4,5-tetrahydrobenzo[d]azepin-3-yl)-3-(2,2,2-trifluorethyl)-3H-pyrimidin-4-on oder
2-Methyl-5-nitro-6-(1,2,4,5-tetrahydrobenzo[d]azepin-3-yl)-pyrimidin-4-ol sind.

4. Verbindungen nach Anspruch 1, wobei R¹ =O ist und R² -CN ist und pharmazeutisch annehmbare Salze davon.

5. Verbindungen nach den Ansprüchen 1 und 4, welche
2-Amino-6-oxo-4-(1,2,4,5-tetrahydrobenzo[d]azepin-3-yl)-1,6-dihydropyrimidin-5-carbonitril,
6-Oxo-4-(1,2,4,5-tetrahydrobenzo[d]azepin-3-yl)-1,6-dihydropyrimidin-5-carbonitril,
2-Ethylamino-6-oxo-4-(1,2,4,5-tetrahydrobenzo[d]azepin-3-yl)-1,6-dihydropyrimidin-5-carbonitril,
1,2-Dimethyl-6-oxo-4-(1,2,4,5-tetrahydrobenzo[d]azepin-3-yl)-1,6-dihydropyrimidin-5-carbonitril,
1-Ethyl-2-methyl-6-oxo-4-(1,2,4,5-tetrahydrobenzo[d]azepin-3-yl)-1,6-dihydropyrimidin-5-carbonitril,
2-Amino-1-ethyl-6-oxo-4-(1,2,4,5-tetrahydrobenzo[d]azepin-3-yl)-1,6-dihydropyrimidin-5-carbonitril,
1-Cyclopropylmethyl-2-methyl-6-oxo-4-(1,2,4,5-tetrahydrobenzo[d]azepin-3-yl)-1,6-dihydropyrimidin-5-carbonitril,
1-Allyl-2-methyl-6-oxo-4-(1,2,4,5-tetrahydrobenzo[d]azepin-3-yl)-1,6-dihydropyrimidin-5-carbonitril,
1-Cyanomethyl-2-methyl-6-oxo-4-(1,2,4,5-tetrahydrobenzo[d]azepin-3-yl)-1,6-dihydropyrimidin-5-carbonitril,
1-(2-Dimethylaminoethyl)-2-methyl-6-oxo-4-(1,2,4,5-tetrahydrobenzo[d]azepin-3-yl)-1,6-dihydropyrimidin-5-carbonitril,
1-Isopropyl-6-oxo-4-(1,2,4,5-tetrahydrobenzo[d]azepin-3-yl)-1,6-dihydropyrimidin-5-carbonitril,
1-(2-Hydroxyethyl)-6-oxo-4-(1,2,4,5-tetrahydrobenzo[d]azepin-3-yl)-1,6-dihydropyrimidin-5-carbonitril,
2-(2-Hydroxyethyl)-6-oxo-4-(1,2,4,5-tetrahydrobenzo[d]azepin-3-yl)-1,6-dihydropyrimidin-5-carbonitril,
2-Methyl-6-oxo-4-(1,2,4,5-tetrahydrobenzo[d]azepin-3-yl)-1-(2,2,2-Trifluorethoxy)-1,6-dihydropyrimidin-5-carbonitril,
2-Methyl-1-methylamino-6-oxo-4-(1,2,4,5-tetrahydrobenzo[d]azepin-3-yl)-1,6-dihydropyrimidin-5-carbonitril oder
1-Amino-2-methyl-6-oxo-4-(1,2,4,5-tetrahydrobenzo[d]azepin-3-yl)-1,6-dihydropyrimidin-5-carbonitril sind.

6. Verbindungen nach Anspruch 1, wobei R¹ 2,2,2-Trifluorethoxy ist und R² -CN ist.

7. Verbindungen nach den Ansprüchen 1 und 6, welche
2-(2-Morpholin-4-yl-ethylamino)-4-(1,2,4,5-tetrahydrobenzo[d]azepin-3-yl)-6-(2,2,2-trifluorethoxy)-pyrimidin-5-carbonitril,
2-(3-Morpholin-4-yl-propylamino)-4-(1,2,4,5-tetrahydrobenzo[d]azepin-3-yl)-6-(2,2,2-trifluorethoxy)-pyrimidin-5-carbonitril,
2-(2-Hydroxyethylamino)-4-(1,2,4,5-tetrahydrobenzo[d]azepin-3-yl)-6-(2,2,2-trifluorethoxy)-pyrimidin-5-carbonitril oder
(3-Imidazol-1-yl-propylamino)-4-(1,2,4,5-tetrahydrobenzo[d]azepin-3-yl)-6-(2,2,2-trifluorethoxy)-pyrimidin-5-carbonitril sind.

8. Verbindungen nach Anspruch 1, wobei R¹ 3-[1,2,4]triazol-1-yl-propoxy ist und R² -NO₂ oder -CN ist.

9. Verbindung nach den Ansprüchen 1 und 8, welche
3-[2-Methyl-5-nitro-6-(3-[1,2,4]triazol-1-yl-propoxy)-pyrimidin-4-yl]-2,3,4,5-tetrahydro-1H-benzo[d]azepin ist.

10. Verbindung nach Anspruch 1, wobei R³ und R⁴ miteinander zu den Gruppen -(CH₂)₃₋₅- verbunden sind, um zusammen mit den N und C-Atomen an die sie gebunden sind einen zusätzlichen Ring zu formen und R² -NO₂ oder -CN ist.

11. Verbindung nach den Ansprüchen 1 und 10, welche
4-Oxo-2-(1,2,4,5-tetrahydrobenzo[d]azepin-3-yl)-4,6,7,8,9,10-hexahydro-pyrimido[1,2 a]azepin-3-carbonitril ist.

12. Medikament umfassend eine Verbindung nach einem der Ansprüche 1-11 oder pharmazeutisch annehmbare Salze davon in ihrer racemischen oder optisch aktiven Form und pharmazeutisch annehmbare Zusatzstoffe.

13. Medikament nach Anspruch 12 zur Behandlung oder Prävention von akuten und/oder chronischen neurologischen Störungen, wie Epilepsie, Schlaganfall, chronische und akute Schmerzen, Psychose, Schizophrenie, kognitive Störungen bei der Alzheimer-Krankheit, Merkfähigkeitsstörungen, durch Bypass-Operationen oder Transplantationen verursachte eingeschränkte Gehirnfunktion, geringe Blutversorgung zum Gehirn, Rückenmarksverletzungen, Kopfverletzungen, durch Schwangerschaft verursachte Hypoxie, Herzstillstand, Hypoglykämie, Huntingtonsche Chorea, ALS, durch AIDS verursachte Demenz, Augenverletzungen, Retinopathie, idiopathischer Parkinsonismus oder durch Medikamente verursachter Parkinsonismus sowie Zustände, welche zu Glutamatmangelfunktionen führen, wie z.B. Muskelspasmen, Konvulsionen, Migräne, urinäre Inkontinenz, Nikotinabhängigkeit, Psychose, Opiatabhängigkeit, Angst, Erbrechen, Dyskinesie und Depression.

14. Verbindungen nach einem der Ansprüche 1-11 sowie pharmazeutisch annehmbare Salze davon in ihrer racemischen und optisch aktiven Form für die Verwendung zur Behandlung oder Prävention von Krankheit.

15. Verwendung von Verbindungen der Formel I nach einem der Ansprüche 1 bis 11 sowie pharmazeutisch annehmbarer Salze davon in ihrer racemischen und optisch aktiven Form zur Herstellung von Medikamenten zur Behandlung oder Prävention von akuten und/oder chronischen neurologischen Störungen, wie Epilepsie, Schlaganfall, chronische und akute Schmerzen, Psychose, Schizophrenie, kognitive Störungen bei der Alzheimer-Krankheit, Merkfähigkeitsstörungen, durch Bypass-Operationen oder Transplantationen verursachte eingeschränkte Gehirnfunktion, geringe Blutversorgung zum Gehirn, Rückenmarksverletzungen, Kopfverletzungen, durch Schwangerschaft verursachte Hypoxie, Herzstillstand, Hypoglykämie, Huntingtonsche Chorea, ALS, durch AIDS verursachte Demenz, Augenverletzungen, Retinopathie, idiopathischer Parkinsonismus oder Parkinsonismus verursacht durch Medikamente sowie Zuständen, welche zu Glutamatmangelfunktionen führen, wie z.B. Muskelspasmen, Konvulsionen, Migräne, urinäre Inkontinenz, Nikotinabhängigkeit, Psychose, Opiatabhängigkeit, Angst, Erbrechen, Dyskinesie und Depression.

16. Verbindung der Formel I nach den Ansprüchen 1 bis 11 sowie pharmazeutisch annehmbare Salze davon zur Behandlung oder Prävention von akuten und/oder chronischen neurologischen Störungen.

17. Verfahren zur Herstellung von Verbindungen der Formel I nach einem der Ansprüche 1-11 sowie pharmazeutisch annehmbarer Salze davon, wobei das Verfahren umfasst
a) Umsetzung einer Verbindung der Formel 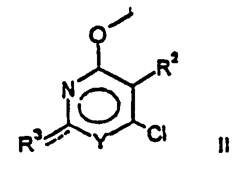 mit einer Verbindung der Formel 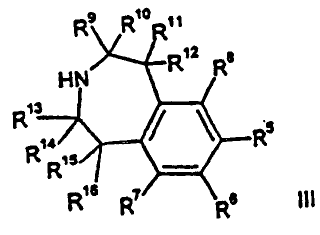 zu einer Verbindung der Formel 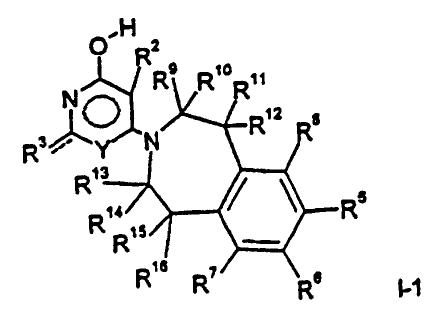 wobei die Substituenten oben beschrieben sind, oder
b) Umsetzung einer Verbindung der Formel 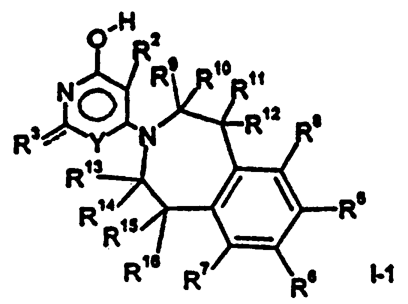 zu einer Verbindung der Formel 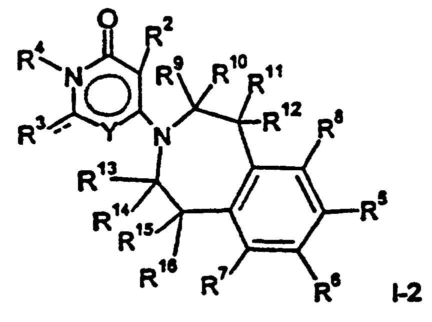 oder zu einer Verbindung der Formel 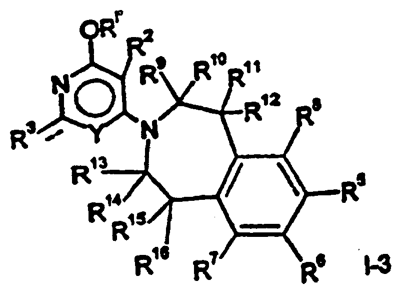 wobei R² bis R¹⁶ die oben angeführten Bedeutungen haben, und R^{1'} nieder Alkyl, -(C₃-C₆)cycloalkyl, -(CHR)ₙ-(C₃-C₆)cycloalkyl, -(CHR)ₙCN, -(CHR)ₙCF₃, -(CHR)(CHR)ₙNR₂, -(CHR)(CHR)ₙOR, -(CHR)ₙ-nieder alkenyl, -CF₃, -CF₂-R, - CF₂-nieder alkenyl, -CHRF, -CHF-nieder alkenyl, -CF₂CRF₂, -CF₂Br, -(CHR)ₙCF₂Br, -(CHR)ₙ-phenyl, wobei die Phenylgruppe wahlweise substituiert sein kann unabhängig voneinander mit 1 bis 3 nieder Alkyl-, nieder Alkoxy-, Halogen-, Nitro- oder Cyanogruppen, -(CHR)(CHR)ₙ-morpholino, -(CHR)(CHR)ₙ-pyrrolidino, -(CHR)(CHR)ₙ-piperidino, -(CHR)(CHR)ₙ-imidazolo, -(CHR)(CHR)ₙ-triazolo, -(CHR)ₙ-pyridino, -(CHR)(CHR)ₙ-OSi-nieder alkyl, -(CHR)(CHR)ₙOS(O)₂-nieder alkyl, -(CH₂)ₙCH=CF₂, -(CHR)ₙ-2,2-dimethyl-[1,3]dioxolan, -(CHR)ₙ-CHOR-CH₂OR oder -(CHR)ₙ-CHOR-(CHR)ₙ-CH₂OR ist;
c) Umsetzung einer Verbindung der Formel 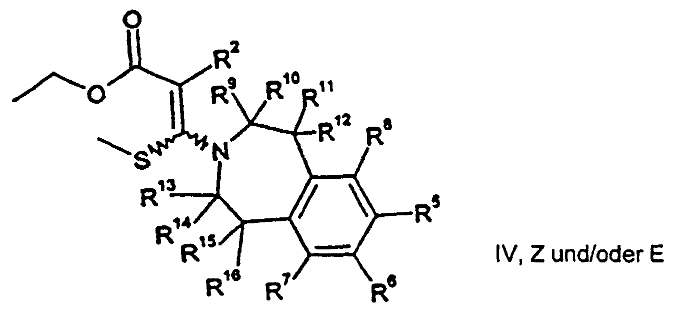 mit einer Verbindung der Formel 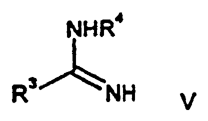 zu einer Verbindung der Formel 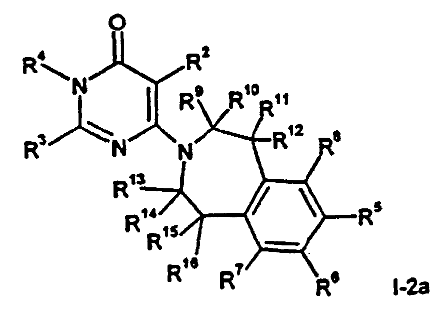 wobei die Substituenten oben beschrieben sind,
oder
d) Umsetzung einer Verbindung der Formel 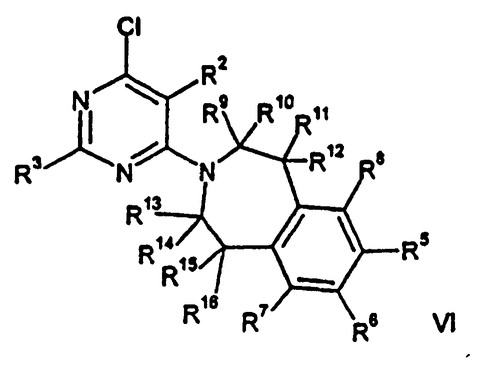 mit einem Alkohol, Thiol, einem primären oder sekundären Amin zu einer Verbindung der Formel 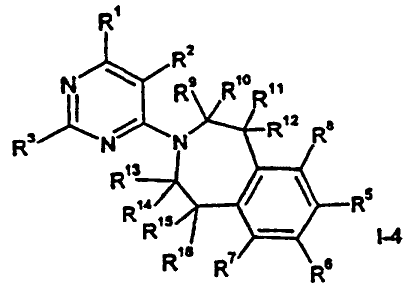 wobei die Substituenten oben beschrieben sind, oder
e) Umsetzung einer Verbindung der Formel 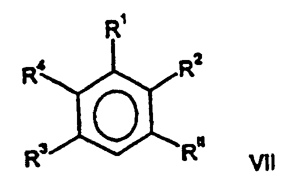 wobei R^{II} in Formel VII Fluor, Chlor, Brom oder eine Trifluormethansulfonyloxygruppe ist,
mit einer Verbindung der Formel 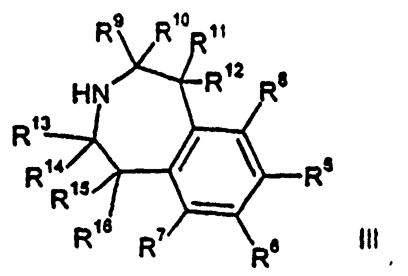 zu einer Verbindung der Formel 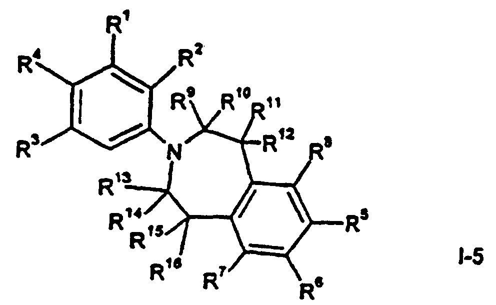 wobei die Substituenten oben beschrieben sind,
f) Umsetzung einer Verbindung der Formel 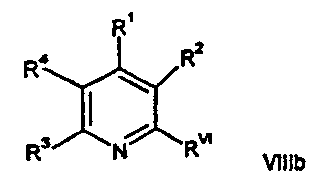 wobei R^{VI} Fluor, Chlor, Brom oder eine Trifluormethansutfonyloxygruppe ist,
mit einer Verbindung der Formel 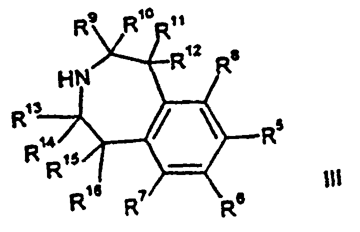 zu einer Verbindung der Formel I-6, 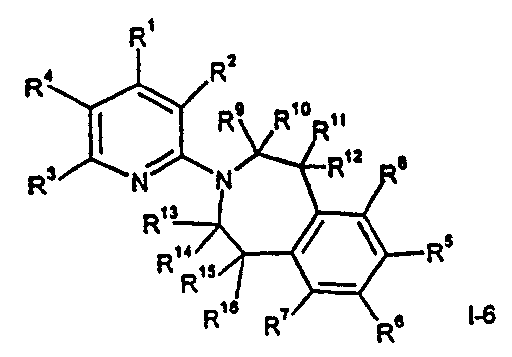 wobei die Substituenten oben beschrieben sind,
und, falls gewünscht,
Einführung und Entfernung von Schutzgruppen in Verbindungen der Formel I, Alkylierung von OH- oder NH- Funktionen in Verbindungen der Formel I, Abspaltung von Ether-Funktionen, Umwandlung einer funktionellen Gruppe in einer Verbindung der Formel I in eine andere funktionelle Gruppe direkt oder durch eine geeignete Aktivierungsgruppe und, falls gewünscht,
Umwandlung einer Verbindung der Formel in ein pharmazeutisch, annehmbares Salz oder in ihre optisch aktive Form.

18. Verbindungen der Formel I nach den Ansprüchen 1 bis 11, hergestellt nach einem Verfahren nach Anspruch 17.

19. Verwendung eines radiomarkierten mGluR1 Rezeptor Antagonisten der Formel I nach Anspruch 1 in einem in vitro Bindungsassay.

20. Verwendung eines radiomarkierten mGluR1 Rezeptor Antagonisten der Formel I in einem in vitro Bindungsassay nach Anspruch 19, welcher
1-Ethyl-2-methyl-6-oxo-4-(1,1,2-tritritio-1,2,4,5-tetrahydrobenzo[d]azepin-3-yl)-1,6-dihydropyrimidin-5-carbonitril ist.

## Revendications

1. Composés de formule générale 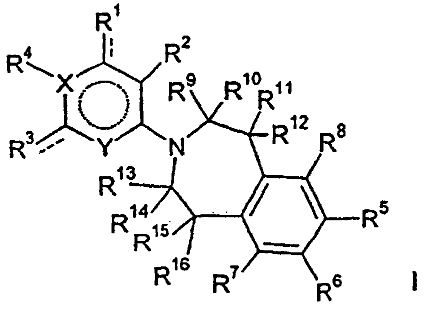 dans laquelle
R¹ représente un hydrogène, un groupe alkyle en C₁-C₇, un oxygène, un halogène, ou -OR, -O(cycloalkyle en C₃-C₆), -O(CHR)ₙ-(cycloalkyle en C₃-C₆), -O(CHR)ₙCN, -O(CHR)ₙCF₃, -O(CHR)(CHR)ₙNR₂, -O(CHR)(CHR)ₙOR, -O(CHR)ₙ-alcényle en C₂-C₇, -OCF₃, -OCF₂-R, -OCF₂-alcényle en C₂-C₇, -OCHRF, -OCHF-alcényle en C₂-C₇, -OCF₂CRF₂, -OCF₂Br, -O(CHR)ₙCF₂Br, -O(CHR)ₙ-phényle, où le groupe phényle peut éventuellement être substitué indépendamment par un à trois groupes alkyle en C₁-C₇, alcoxy en C₁-C₇, halogènes, nitro ou cyano,
-O(CHR)(CHR)ₙ-morpholino, -O(CHR)(CHR)ₙ-pyrrolidino, -O(CHR)(CHR)ₙ-pipéridino, -O(CHR)(CHR)ₙ-imidazolo, -O(CHR)(CHR)ₙ-triazolo, -O(CHR)ₙ-pyridino, -O(CHR)(CHR)ₙ-OSi-alkyle inférieur, -O(CHR)(CHR)ₙOS(O)₂-alkyle en C₁-C₇, -O(CH₂)ₙCH=CF₂, -O(CHR)ₙ-2,2-diméthyl-[1.3]dioxolane, -O(CHR)ₙ-CHOR-CH₂OR, -O(CHR)ₙ-CHOR-(CHR)ₙ-CH₂OR ou
- SR ou -S(CHR)ₙCOOR, ou
-NR², -N(R) (CHR) (CHR)ₙOR, -N(R)(CHR)ₙCF₃, -N(R)(CHR)(CHR)ₙ-morpholino, -N(R)(CHR)(CHR)ₙ-imidazolo, -N(R)(CHR)(CHR)ₙ-pyrrolidino, -N(R)(CHR)(CHR)ₙ-pyrrolidin-2-one, -N(R)(CHR)(CHR)ₙ-pipéridino, -N(R)(CHR)(CHR)ₙ-triazolo, -N(R)(CHR)ₙ-pyridino, ou
n vaut 1-6 ;
R représente un hydrogène, un groupe alkyle en C₁-C₇ ou alcényle en C₂-C₇, indépendamment les uns des autres, si plus,d'un R est présent ;
R² représente un groupe nitro ou cyano ;
R³ représente un hydrogène, un groupe alkyle en C₁-C₇, =O, =S, -SR, -S(O)₂-alkyle en C₁-C₇, cycloalkyle en C₃-C₆ ou pipérazino, éventuellement substitué par un groupe alkyle en C₁-C₇, ou
-CONR₂, -(CHR)ₙCONR₂, -(CHR)ₙOR, -(CH₂)ₙ-CF₃, -CF₃, -(CHR)ₙOC(O)CF₃, -(CHR)ₙCOOR, -(CHR)ₙSC₆H₅, où le groupe phényle peut être éventuellement substitué indépendamment par un à trois groupes alkyle en C₁-C₇, alcoxy en C₁-C₇, halogènes, nitro ou cyano,
-(CHR)ₙ-1,3-dioxo-1,3-dihydro-isoindol, -(CHR)ₙ-tétrahydro-pyran-2-yloxy ou -(CHR)ₙ-S-alkyle en C₁-C₇, ou
-NR₂, -NRCO-alkyle en C₁-C₇, -NRCHO, -N(R)(CHR)ₙCN, -N (R) (CHR)ₙCF₃, -N (R) (CHR) (CHR)ₙ-OR, -N(R)C(O)(CHR)ₙO-alkyle en C₁-C₇, -NR(CHR)ₙ-alkyle en C₁-C₇, -NR(CHR)(CHR)ₙ-OR, -N(R)(CHR)(CHR)ₙ-O-phényle, où le groupe phényle peut être éventuellement substitué indépendamment par un à trois groupes alkyle en C₁-C₇, alcoxy en C₂-C₇, halogènes, nitro ou cyano,
-N(R)(CHR)ₙ-alcényle en C₂-C₇, -N(R)(CHR)(CHR)ₙ-O-(CHR)ₙOR, -N(R)(CHR)ₙC(O)O-alkyle en C₁-C₇, -N(R)(CHR)ₙC(O)NR-alkyle en C₁-C₇, -N(R)(CH₂)ₙ2,2-diméthyl-[1.3]dioxolane,
-N(R)(CHR)(CHR)ₙmorpholino, -N (R) (CHR)ₙ-pyridino, -N(R)(CHR)(CHR)ₙ-pipéridino, -N(R)(CHR)(CHR)ₙ-pyrrolidino, -N(R)(CHR)(CHR)ₙ-O-pyridino, -N(R)(CHR)(CHR)ₙimidazolo, -N(R)(CHR)ₙ-CR₂-(CHR)ₙ-OR, -N(R) (CHR)ₙ-CR₂-OR, -N(R) (CHR)ₙ-CHOR-CH₂OR, -N(R)(CHR)ₙ-CHOR-(CHR)ₙ-CH₂OR, ou
-OR, -O(CHR)ₙCF₃, -OCF₃, -O(CHR)(CHR)ₙ-O-phényle, où le groupe phényle peut être éventuellement substitué indépendamment par un à trois groupes alkyle en C₁-C₇, alcoxy en C₁-C₇, halogènes, nitro ou cyano,
-O(CHR)(CHR)ₙ-O-alkyle en C₁-C₇, -O(CHR)ₙ-pyridino ou -O(CHR)(CHR)ₙ-morpholino ; et
R⁴ représente un hydrogène, un groupe alkyle en C₁-C₇, alcényle en C₂-C₇ ou nitro, ou
-OR, -OCF₃, -OCF₂-R, -OCF₂-alcényle en C₂-C₇, -OCHRF, -OCHF-alcényle en C₂-C₇, -O(CHR)ₙCF₃, ou (CHR)ₙCHRF, -(CHR)ₙCF₂R, -(CHR)ₙCF₃, -cycloalkyle en C₃-C₆, -(CHR)ₙ-cycloalkyle en C₃-C₆, -(CHR)ₙCN, -(CHR)ₙ-phényle, où le groupe phényle peut éventuellement être substitué indépendamment par un à trois groupes alkyle en C₁-C₇, alcoxy en C₁-C₇, halogènes, nitro ou cyano,
-(CHR)(CHR)ₙOR, -(CHR)ₙCHORCH₂OR, -(CHR)(CHR)ₙNR₂, -(CHR)ₙCOOR, -(CHR)(CHR)ₙOSi-alkyle en C₁-C₇, -(CHR)(CHR)ₙ-OS(O)₂-alkyle en C₁-C₇, -(CH₂)ₙ-CH=CF₂, -CF₃, -CF₂-R, -CF₂-alcényle inférieur, -CHRF, -CHF-alcényle en C₂-C₇, -(CHR)ₙ-2,2-diméthyl-[1.3]dioxolane, -(CH₂)ₙ-2-oxo-azépan-1-yle, -(CHR)(CHR)ₙ-morpholino, -(CHR)ₙ-pyridino, -(CHR)(CHR)ₙ-imidazolo, -(CHR)(CHR)ₙ-triazolo, -(CHR)(CHR)ₙ-pyrrolidino, éventuellement substitué par -(CH₂)ₙOH, -(CHR)(CHR)ₙ-3-hydroxy-pyrrolidino ou -(CHR)(CHR)ₙ-pipéridino, ou
-NR₂, -N (R) (CHR)ₙ-pyridino, -N(R)C(O)O-alkyle en C₁-C₇, -N(CH₂CF₃)C(O)O-alkyle en C₁-C₇, -N[C(O)O-alkyle en C₁-C₇]₂, -NR-NR-C(O)O-alkyle en C₁-C₇, ou -N(R) (CHR)ₙCF₃, -NRCF₃, -NRCF₂-R, -NRCF₂-alcényle en C₂-C₇, -NRCHRF, -NRCHF-alcényle en C₂-C₇ ;
ou est absent, si X est -N= ou =N- ;
ou R⁴ et R¹ ou R³ et R⁴ sont interconnectés aux groupes -(CH₂)₃₋₅-, -(CH₂)₂-N=, -CH=N-N=-, -CH=CH-N=, -NH-CH=CH- ou -NR-CH₂-CH₂- et forment ensemble avec les atomes N et C auxquels ils sont attachés un cycle supplémentaire ;
R⁵,R⁶ représentent un hydrogène, un groupe alkyle en C₁-C₇, alcoxy en C₁-C₇, amino, nitro, -SO₂NH₂ ou un halogène ; ou
R⁵ et R⁶ sont interconnectés au groupe -O-CH₂-O- et forment ensemble avec les atomes C auxquels ils sont attachés un cycle à 5 chaînons supplémentaire ;
R⁷, R⁸ représentent un hydrogène, un groupe alkyle en C₁-C₇, alcoxy en C₁-C₇, amino, nitro ou un halogène ;
R⁹, R¹⁰ représentent un hydrogène ou un groupe alkyle en C₁-C₇ ;
R¹¹, R¹² représentent un hydrogène, un groupe alkyle en C₁-C₇, hydroxy, alcoxy en C₁-C₇, (alcoxy inférieur)carbonyloxy ou (alcanoyle en C₁-C₇)oxy ;
R¹³, R¹⁴ représentent un hydrogène, un tritium ou un groupe alkyle en C₁-C₇ ;
R¹⁵, R¹⁶ représentent un hydrogène, un tritium, un groupe alkyle en C₁-C₇, hydroxy, alcoxy en C₁-C₇, (alcoxy en C₁-C₇)carbonyloxy ou (alcanoyle en C₁-C₇)oxy ou sont ensemble un groupe oxo ; ou
X représente -N=, =N-, -N<, >C= ou =C< ;
Y représente -N=, =N-, -NH-, -CH= ou =CH- ; et
la ligne pointillée peut être une liaison,
ainsi que leurs sels pharmaceutiquement acceptables sous leur forme racémique et optiquement active.

2. Composés selon la revendication 1, dans lesquels R¹ est =O ou un groupe hydroxy et R² est NO₂ et leurs sels pharmaceutiquement acceptables.

3. Composés selon les revendications 1 et 2 , qui sont
la 3-éthyl-2-méthyl-5-nitro-6-(1,2,4,5-tétrahydro-benzo[d]azépin-3-yl)-3H-pyrimidin-4-one,
la 3-(2-fluoro-éthyl)-2-méthyl-5-nitro-6-(1,2,4,5-tétrahydro-benzo[d]azépin-3-yl)-3H-pyrimidin-4-one,
la 2-méthyl-5-nitro-6-(1,2,4,5-tétrahydro-benzo[d]azépin-3-yl)-3-(2,2,2-trifluoroéthyl)-3H-pyrimidin-4-one ou
le 2-méthyl-5-nitro-6-(1,2,4,5-tétrahydro-benzo[d]azépin-3-yl)-pyrimidin-4-ol.

4. Composés selon la revendication 1, dans lesquels R¹ est =O et R² est -CN et leurs sels pharmaceutiquement acceptables.

5. Composés selon les revendications 1 et 4, qui sont
le 2-amino-6-oxo-4-(1,2,4,5-tétrahydro-benzo[d]azépin-3-yl)-1,6-dihydro-pyrimidine-5-carbonitrile,
le 6-oxo-4-(1,2,4,5-tétrahydro-benzo[d]azépin-3-yl)-1,6-dihydro-pyrimidine-5-carbonitrile,
le 2-éthylamino-6-oxo-4-(1,2,4,5-tétrahydro-benzo[d]-azépin-3-yl)-1,6-dihydro-pyrimidine-5-carbonitrile,
le 1,2-diméthyl-6-oxo-4-(1,2,4,5-tétrahydro-benzo[d]-azépin-3-yl)-1,6-dihydro-pyrimidine-5-carbonitrile,
le 1-éthyl-2-méthyl-6-oxo-4-(1,2,4,5-tétrahydro-benzo[d]azépin-3-yl)-1,6-dihydro-pyrimidine-5-carbonitrile,
le 2-amino-1-éthyl-6-oxo-4-(1,2,4,5-tétrahydro-benzo[d]-azépin-3-yl)-1,6-dihydro-pyrimidine-5-carbonitrile,
le 1-cyclopropylméthyl-2-méthyl-6-oxo-4-(1,2,4,5-tétrahydro-benzo[d]azépin-3-yl)-1,6-dihydro-pyrimidine-5-carbonitrile,
le 1-allyl-2-méthyl-6-oxo-4-(1,2,4,5-tétrahydro-benzo[d]azépin-3-yl)-1,6-dihydro-pyrimidine-5-carbonitrile,
le 1-cyanométhyl-2-méthyl-6-oxo-4-(1,2,4,5-tétrahydro-benzo[d]azépin-3-yl)-1,6-dihydro-pyrimidine-5-carbonitrile,
le 1-(2-diméthylamino-éthyl)-2-méthyl-6-oxo-4-(1,2,4,5-tétrahydro-benzo[d]azépin-3-yl)-1,6-dihydro-pyrimidine-5-carbonitrile,
le 1-isopropyl-6-oxo-4-(1,2,4,5-tétrahydro-benzo[d]-azépin-3-yl)-1,6-dihydro-pyrimidine-5-carbonitrile,
le 1-(2-hydroxy-éthyl)-6-oxo-4-(1,2,4,5-tétrahydro-benzo[d]azépin-3-yl)-1,6-dihydro-pyrimidine-5-carbonitrile,
le 2-(2-hydroxy-éthyl)-6-oxo-4-(1,2,4,5-tétrahydro-benzo[d]azépin-3-yl)-1,6-dihydro-pyrimidine-5-carbonitrile,
le 2-méthyl-6-oxo-4-(1,2,4,5-tétrahydro-benzo[d]azépin-3-yl)-1-(2,2,2-trifluoro-éthoxy)-1,6-dihydro-pyrimidine-5-carbonitrile,
le 2-méthyl-1-méthylamino-6-oxo-4-(1,2,4,5-tétrahydro-benzo[d]azépin-3-yl)-1,6-dihydro-pyrimidine-5-carbonitrile ou
le 1-amino-2-méthyl-6-oxo-4-(1,2,4,5-tétrahydro-benzo[d]azépin-3-yl)-1,6-dihydro-pyrimidine-5-carbonitrile.

6. Composés selon la revendication 1, dans lesquels R¹ est un groupe 2,2,2-trifluoroéthoxy et R² est -CN.

7. Composés selon les revendications 1 et 6, qui sont
le 2-(2-morpholin-4-yl-éthylamino)-4-(1,2,4,5-tétrahydro-benzo[d]azépin-3-yl)-6-(2,2,2-trifluoro-éthoxy)-pyrimidine-5-carbonitrile,
le 2-(3-morpholin-4-yl-propylamino)-4-(1,2,4,5-tétrahydro-benzo[d]azépin-3-yl)-6-(2,2,2-trifluoro-éthoxy)-pyrimidine-5-carbonitrile,
le 2-(2-hydroxy-éthylamino)-4-(1,2,4,5-tétrahydro-benzo[d]azépin-3-yl)-6-(2,2,2-trifluoro-éthoxy)-pyrimidine-5-carbonitrile ou
le (3-imidazol-1-yl-propylamino)-4-(1,2,4,5-tétrahydro-benzo[d]azépin-3-yl)-6-(2,2,2-trifluoro-éthoxy)-pyrimidine-5-carbonitrile.

8. Composés selon la revendication 1, dans lesquels R¹ est un groupe 3-[1,2,4]triazol-1-yl-propoxy et R² est -NO₂ ou -CN.

9. Composé selon les revendications 1 et 8, qui est
la 3-[2-méthyl-5-nitro-6-(3-[1,2,4]triazol-1-yl-propoxy)-pyrimidin-4-yl]-2,3,4,5-tétrahydro-1H-benzo[d]azépine.

10. Composé selon la revendication 1, dans lequel R³ et R⁴ sont interconnectés aux groupes -(CH₂)₃₋₅-pour former ensemble avec les atomes N et C auxquels ils sont attachés un cycle supplémentaire et R² est -NO₂ ou -CN.

11. Composé selon les revendications 1 et 10, qui est
le 4-oxo-2-(1,2,4,5-tétrahydro-benzo[d]azépin-3-yl)-4,6,7,8,9,10-hexahydro-pyrimido[1,2-a]azépine-3-carbonitrile.

12. Médicament comprenant un composé selon l'une quelconque des revendications 1-11 ainsi que des sels pharmaceutiquement acceptables de ceux-ci sous leur forme racémique et optiquement active et des excipients pharmaceutiquement acceptables.

13. Médicament selon la revendication 12 pour le contrôle ou la prévention des troubles neurologiques aigus et/ou chroniques tels que l'épilepsie, l'accident vasculaire cérébral, la douleur chronique et aiguë, la psychose, la schizophrénie, les troubles cognitifs de la maladie d'Alzheimer, les défauts de mémoire, la fonction cérébrale restreinte causée par les opérations de pontage ou les greffes, un mauvais apport sanguin au cerveau, les lésions de la moelle épinière, les blessures à la tête, l'hypoxie causée par la grossesse, l'arrêt cardiaque, l'hypoglycémie, la chorée de Huntington, la maladie de Charcot, la démence causée par le SIDA, les blessures à l'oeil, la rétinopathie, le parkinsonisme idiopathique ou le parkinsonisme causé par des médicaments ainsi que les états qui conduisent à des fonctions de déficience en glutamate, comme par exemple les spasmes musculaires, les convulsions, la migraine, l'incontinence urinaire, la dépendance à la nicotine, les psychoses, la dépendance aux opiacés, l'anxiété, le vomissement, la dyskinésie et la dépression.

14. Composés selon l'une quelconque des revendications 1-11 ainsi que des sels pharmaceutiquement acceptables de ceux-ci sous leur forme racémique et optiquement active pour une utilisation dans le contrôle ou la prévention de la maladie.

15. Utilisation des composés de formule I selon l'une quelconque des revendications 1-11 ainsi que des sels pharmaceutiquement acceptables de ceux-ci sous leur forme racémique et optiquement active pour la fabrication de médicaments pour le contrôle ou la prévention des troubles neurologiques aigus et/ou chroniques tels que l'épilepsie, l'accident vasculaire cérébral, la douleur chronique et aiguë, la psychose, la schizophrénie, les troubles cognitifs de la maladie d'Alzheimer, Les défauts de mémoire, la fonction cérébrale restreinte causée par les opérations de pontage ou les greffes, un mauvais apport sanguin au cerveau, les lésions de la moelle épinière, les blessures à la tête, l'hypoxie causée par la grossesse, l'arrêt cardiaque, l'hypoglycémie, la chorée de Huntington, la maladie de Charcot, la démence causée par le SIDA, les blessures à l'oeil, la rétinopathie, le parkinsonisme idiopathique ou le parkinsonisme causé par des médicaments ainsi que les états qui conduisent à des fonctions de déficience en glutamate, comme par exemple les spasmes musculaires, les convulsions, la migraine, l'incontinence urinaire, la dépendance à la nicotine, les psychoses, la dépendance aux opiacés, l'anxiété, le vomissement, la dyskinésie et la dépression.

16. Composé de formule I selon les revendications 1-11 ainsi que les sels pharmaceutiquement acceptables de ceux-ci pour le contrôle ou la prévention des troubles neurologiques aigus et/ou chroniques.

17. Procédé de fabrication des composés de formule I selon l'une quelconque des revendications 1-11 ainsi que des sels pharmaceutiquement acceptables de ceux-ci, lequel procédé comprend
a) la réaction d'un composé de formule 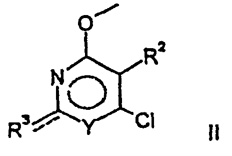 avec un composé de formule 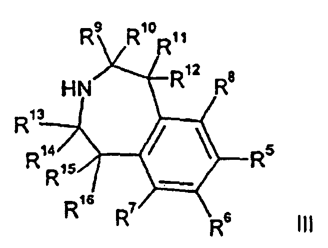 pour donner un composé de formule 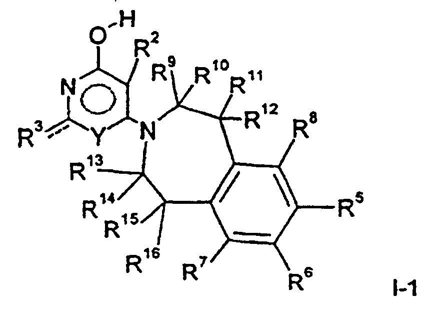 dans laquelle les substituants sont décrits ci-dessus, ou
b) la réaction d'un composé de formule 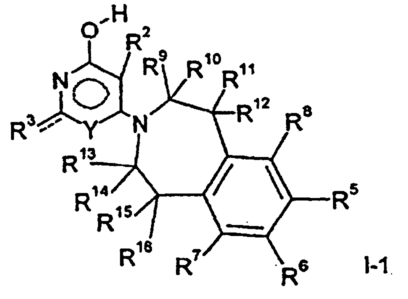 pour donner un composé de formule 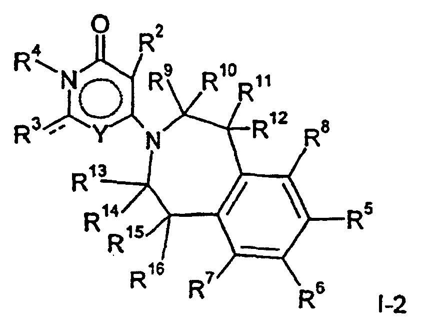 ou un composé de formule 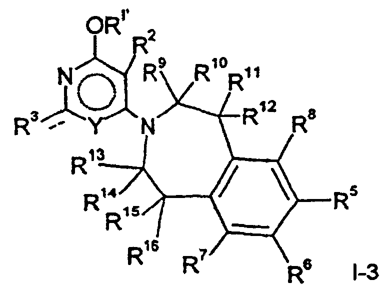 dans laquelle R² à R¹⁶ ont les significations données ci-dessus et R^{I'} est un groupe alkyle inférieur, cycloalkyle en C₃-C₆, -(CHR)ₙ-cycloalkyle en C₃-C₆, -(CHR)ₙCN, -(CHR)ₙCF₃, -(CHR)(CHR) ₙNR₂, -(CHR) (CHR)ₙOR, -(CHR)ₙ-alcényle inférieur, -CF₃, -CF₂-R, -CF₂-alcényle inférieur, -CHRF, -CHF-alcényle inférieur, -CF₂CRF₂, -CF₂Br, -(CHR)ₙCF₂Br, -(CHR)ₙ-phényle, où le groupe phényle peut éventuellement être substitué indépendamment par un à trois groupes alkyle inférieur, alcoxy inférieur, halogènes, nitro ou cyano, -(CHR)(CHR)ₙ-morpholino, -(CHR)(CHR)ₙ-pyrrolidino, -(CHR)(CHR)ₙ-pipéridino, -(CHR)(CHR)ₙ-imidazolo, -(CHR)(CHR)ₙ-triazolo, -(CHR)ₙ-pyridino, -(CHR) (CHR) ₙ-OSi-alkyle inférieur, -(CHR) (CHR) ₙOS(O)₂-alkyle inférieur, -(CH₂)ₙCH=CF₂, -(CHR)ₙ-2,2-diméthyl-[1.3]dioxolane, -(CHR)ₙ-CHOR-CH₂OR ou -(CHR)ₙ-CHOR-(CHR)ₙ-CH₂OR.
c) la réaction d'un composé de formule 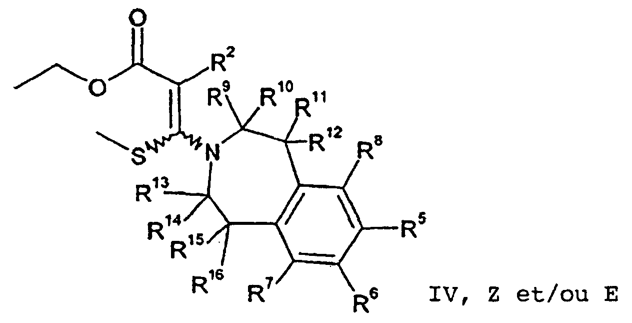 avec un composé de formule 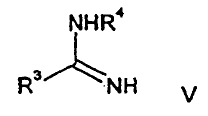 pour donner un composé de formule 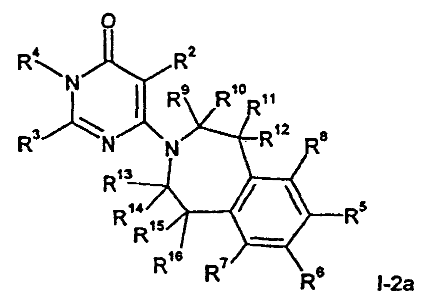 dans laquelle les substituants sont décrits ci-dessus, ou
d) la réaction d'un composé de formule 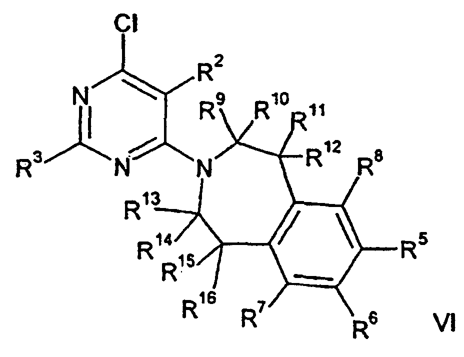 avec un alcool, un thiol, une amine primaire ou secondaire pour donner un composé de formule 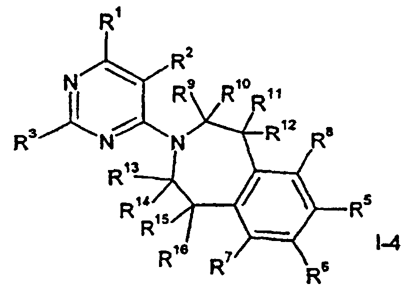 dans laquelle les substituants sont décrits ci-dessus, ou
e) la réaction d'un composé de formule 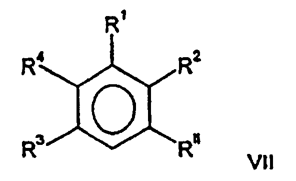 dans laquelle R^{II} dans la formule VII est un fluor, un chlore, un brome ou un groupe trifluorométhanesulfonyloxy,
avec un composé de formule 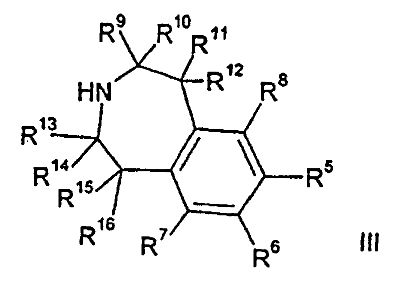 pour donner un composé de formule 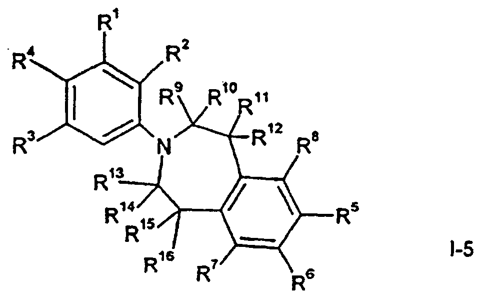 dans laquelle les substituants sont décrits ci-dessus, ou
f) la réaction d'un composé de formule 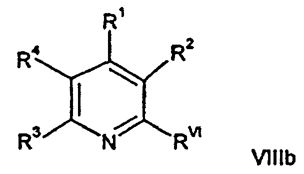 dans laquelle R^{VI} est un fluor, un chlore, un brome ou un groupe trifluorométhanesulfonyloxy,
avec un composé de formule 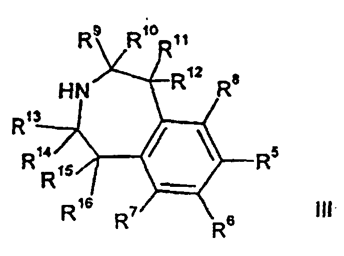 pour donner un composé de formule I-5, 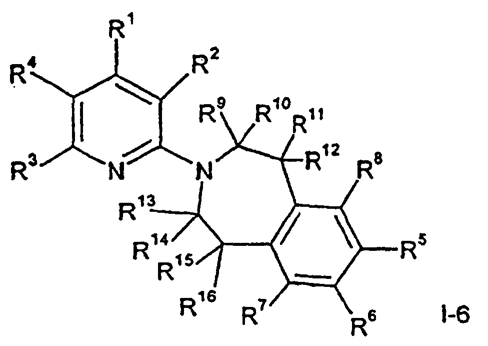 dans laquelle les substituants sont décrits ci-dessus, et, si on le souhaite,
l'introduction et l'élimination des groupes protecteurs dans les composés de formule I, l'alkylation des fonctions OH ou NH dans les composés de formule I, la coupure des fonctions éthers, la conversion d'un groupe fonctionnel dans un composé de formule I en un autre groupe fonctionnel directement ou via un groupe activateur approprié et, si on le souhaite,
la conversion d'un composé de formule I en un sel pharmaceutiquement acceptable ou en sa forme optiquement active.

18. Composés de formule I selon les revendications 1-11, lorsqu'ils sont fabriqués conformément à un procédé selon la revendication 17.

19. Utilisation d'un antagoniste de récepteur mGluR1 radiomarqué de formule I selon la revendication 1 dans un test de liaison in vitro.

20. Utilisation d'un antagoniste de récepteur mGluR1 radiomarqué de formule I dans un test de liaison in vitro selon la revendication 19, qui est
le 1-éthyl-2-méthyl-6-oxo-4-(1,1,2-tritritio-1,2,4,5-tétrahydro-benzo[d]azépin-3-yl)-1,6-dihydro-pyrimidine-5-carbonitrile.
